# EUROPEAN PATENT SPECIFICATION

(11) **EP 1 749 822 B1**
(45) Date of publication and mention of the grant of the patent: **15.10.2008**
(21) Application number: 05291683.0
(22) Date of filing: 05.08.2005
(51) Int. Cl.: C07D 241/38, A61K 31/495, A61P 35/00, A61P 25/28, A61P 9/00, A61P 31/00

(54) **Novel cysteine protease inhibitors and their therapeutic applications**
Neue Cysteine Protease Hemmers und ihre therapeutische Anwendungen
Nouvelles inhibiteurs de cysteine protease et leur utilisation therapeutique

(43) Date of publication of application: 07.02.2007
(73) Proprietor: Hybrigenics S.A., 75014 Paris (FR)
(72) Inventor: Guedat, Philippe, 25260 Montenois (FR); Boissy, Guillaume, 94300 Vincennes (FR); Borg-Capra, Catherine, 92150 Suresnes (FR); Colland, Frédéric, 95380 Puiseux en France (FR); Daviet, Laurent, 92160 Antony (FR); Formstecher, Etienne, 75015 Paris (FR); Jacq, Xavier, 75012 Paris (FR); Rain, Jean-Christophe, 95120 Ermont (FR); Delansorne, Rémi, 75006 Paris (FR); Vallese, Stefania, 21013 Gallarate (VA) (IT); Colombo, Matteo, 20030 Camnago (MI) (IT)
(74) Representative: Colombet, Alain André

(56) References cited:
- WO-A-03/048123
- WO-A-20/04022526
- US-A- 6 103 720
- US-A1- 2004 198 716
- LANG M ET AL: "aza-2-diènes; nouvelles voies d'accès à des amino-2-pyrazines substituées" HELVETICA CHIMICA ACTA, vol. 69, 1986, pages 793-802, XP009060166
- LANG M ET AL: "nouvelle synthese univoque d'aminopyrazines" TETRAHEDRON LETTERS, vol. 45, 1974, pages 3967-3970, XP002363833

## Description

The present invention concerns new inhibitors of cysteine proteases, their process of preparation and their therapeutic use.

Protease can be categorized based on their substrate specificities or mechanisms of catalysis. Upon the basis of the mechanism of peptide hydrolysis, five major protease classes are known: serine, cysteine, aspartic, threonine and metallo-proteases. Cysteine proteases comprise, *inter allia,* de-ubiquitination enzymes, caspases, cathepsins, calpains as well as viral, bacterial or parasitic cysteine proteases.

De-ubiquitination enzymes include Ubiquitin Specific Proteases (USPs) and Ubiquitin Carboxy Hydrolases (UCHs). Broadly speaking, the ubiquitin pathway regulates protein degradation and is more particularly involved in cancer, in neurodegenerative diseases such as Alzheimer's disease, Parkinson's disease, in inflammation, in viral infectivity and latency (in particular for Herpes simplex virus-1, Epstein-Barr virus, SARS coronavirus), or in cardiovascular diseases (Chem. Rev. 1997, 97, p. 133-171; Chem. Rev. 2002, 102, p. 4459-4488; J. Biochem. 2003, 134, p. 9-18 ; J. Virology, 2005, 79(7), p. 4550-4551; Cardiovasc. Res. 2004, 61, p. 11-21).

Caspases have been shown to be involved in apoptosis and hence are targets in hepatitis, liver failure, inflammation, cardiac ischemia and failure, renal failure, neurodegeneration, deafness, diabetes, or stroke (J. Pharmacol Exp. Ther., 2004, 308(3), p. 1191-1196, J. Cell. Physiol., 2004, 200(2), p. 177-200; Kidney Int, 2004, 66(2), p. 500-506; Am. J. Pathol., 2004, 165(2), p. 353-355; Mini Rev. Chem., 2004, 4(2), p. 153-165; Otol. Neurotol., 2004, 25(4), p. 627-632; Ref. 7, 21, 22, 23, 24, 25.

Cathepsins generally have been shown to be involved in cancer and metastasis, inflammation, immunology/immunoregulation (Eur. Respir. J., 2004 23(4), p. 620-628) and atherosclerosis (Ageing Res. Rev.. 2003, 2(4), p. 407-418). More particularly, cathepsins include cathepsin B and B-like which are implicated in cancer and metastasis, and arthritis (Cancer Metastasis Rev., 2003, 22(2-3), p. 271-286; Biol. Chem., 2003, 384(6), p. 845-854 and Biochem. Soc. Symp., 2003, 70, p. 263-276), cathepsin D, involved in particular in cancer and metastasis (Clin. Exp. Metastasis, 2004, 21(2), p. 91-106), cathepsin K acting in osteoporosis and arthritis (Int. J. Pharm., 2004, 277(1-2), p. 73-79), cathepsin S which has been shown to play a role in antigen presentation in immunology (Drug News Perspective, 2004, 17(6), p. 357-363).

Calpains play a role in aging in general (Ageing Res. Rev. 2003, 2(4), p. 407-418), as well as diabetes (Mol. Cell. Biochem., 2004, 261(1). p.161-167) and cataract (Trends Mol. Med., 2004, 10(2), p. 78-84) more particularly.

Viral cysteine proteases have been identified in rhinoviruses, poliomyelitis virus, hepatitis A virus, hepatitis C virus, adenovirus, or SARS coronavirus (Chem. Rev. 1997, 97, p. 133-171; Chem. Rev. 2002, 102, p. 4459-4488 ; J. Virology, 2005, 79(7), p. 4550-4551 and Acta Microbiol. Immunol. Hung., 2003, 50(1), p. 95-101).

Bacterial cysteine proteases include streptopain, staphylococcal cysteine protease, clostripain or gingipains; yeasts such as *Aspergillus flavus* have also been shown to express cysteine proteases which may constitute a virulence factor (Chem. Rev. 1997, 97, p. 133-171).

Parasitic cysteine proteases have been reviewed in Molecular & Biochemical Parasitology (2002, 120, p. 1-21) and Chem. Rev. (2002, 102, p. 4459-4488) for example. It is worth noting that the parasitic agents responsible for most major parasitic diseases are making use of their own cysteine proteases at some point or another of their infective, nutritive or reproductive cycles; such diseases include malaria, Chagas' disease, African trypanosomiasis, leishmaniasis, giardiasis, trichomoniasis, amoebiasis, cryptosporidiasis, toxoplamiasis, schistosomiasis, fasciolasis, onchocercosis, and other infections by some other flat or round worms.

Therefore, identifying a novel class of inhibitors of cysteine proteases is of significant importance in a wide range of diseases and pathological conditions.

Cyano-pyrazine derivatives have been disclosed, mainly as charge-transporting agents for electrophotographic photoreceptors (WO03/055853, JP200128885, JP200122316, JP07281460, JP07179440, JP07098508, JP06345742, JP07175235, JP07199487, JP07199486, JP07281460 and Helvetica Chemica Acta, 1986, 69(4), 793-802, Tetrahedron Letters 1974, 45, 3967-70, J. Heteterocyclic Chemistry, 1972, 9(6), 1399-401, Tetrahedron Letters, 1990, 31(49), 7215-18). However, it has never been disclosed nor suggested that cyano-pyrazine derivatives can inhibit cysteine proteases.

According to a first object, the present invention concerns a compound of formula (I): 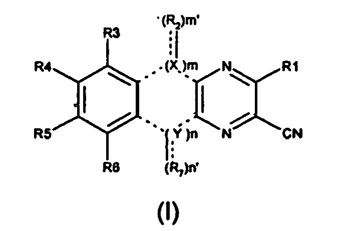 wherein :
m is 0 ; 1 or 2 , wherein when m=0, ----- (X(R2)_{m'})ₘ ----- is none so as to form an open ring or a single bond;
n is 0, 1 or 2, , wherein when n=0, ----- (Y(R7)_{n'})ₙ ----- is none so as to form an open ring or a single bond;
m' and n' are independently 0, 1 or 2;
X is a carbon atom or S or N;
Y is a carbon atom, or S or N;
Provided m and n are not simultaneously 0;
- - - -is either a single or double bond, as appropriate ;
- - - - - - is either none or a single bond, as appropriate;
R1 is chosen from the group consisting in H, CN, Hal, OAlk, OH, NRCN, C(CN)=C(OH)(OAlk), SR, NRR', C(O)NRR', Heterocycle, Aryle, Heteroaryle, where Alk, Aryle, Heteroaryle, heterocycle are optionally substituted by Hal, NRR', CN, OH, CF₃, Aryle, Heteroaryle, OAlk;
R3, R4, R5, R6 are each identical or different and are independently chosen from the group consisting in H, OAlk, Alk, Hal, NRR', CN, OH, CF₃, Aryle, Heteroaryle;
R2 is chosen from the group consisting in H, O, OH, N-OH, N-Aryle, N-OAlk, NO-Aryle, N-O-Alk-Aryle or 2 R2 bound at the same X form together with that X an heterocycle; wherein said Alk, Aryle or heterocycle are optionally substituted by OAlk, Alk, Hal, NRR', CN, OH, CF₃;
R7 is chosen from the group consisting in H, O, OH, N-OH, N-Aryle, N-OAlk, NO-Aryle or N-O-Alk-Aryle or 2 R7 bound at the same Y form together with that Y an heterocycle; wherein said Alk, Aryle or heterocycle are optionally substituted by OAlk, Alk, Hal, NRR', CN, OH, CF₃;
R and R' are each identical or different and are independently chosen from the group consisting in H, Alk, wherein Alk is optionally substituted by Hal, NRR', CN, OH, CF₃, Aryle, Heteroaryle;
or their pharmaceutically acceptable salts, hydrates, or hydrated salts, or the polymorphic crystalline structures of these compounds or their optical isomers, racemates, diastereomers or enantiomers,
with the exception of compounds where:
R3, R4, R5, R6=H, R1=CN, ---(X(R2)_{m'})ₘ--- represents a single bond, and
---(Y(R7)_{n'})ₙ--- represents -C(=N-OH)- or -C(=N-(2-,4-,6-trimethylphenyl))-,
-C(=N-(2-,6-dimethylphenyl))-, -C(=N-(2-,6-diethylphenyl))-, -C(=N-(2-methylphenyl))-, -C(=N-(2-ethylphenyl))-, -C(=N-(2-trifluoromethylphenyl))-, -C(=N-(2-isopropylphenyl))-, -C(=N-phenyl)-, -C(=N-(naphtyl)- or -C(=O)-, -CH2-, or
R3, R5, R6=H, R4=OMe, R1=CN, ---(X(R2)_{m'})ₘ--- represents a single bond, and
--(Y(R7)ₙ)ₙ-- represents -C(=O)-, or
R3, R4, R6=H, R5=OMe, R1=CN, ---(X(R2)_{m'})ₘ--- represents a single bond, and
--(Y(R7)_{n'})ₙ--- represents -C(=O)-, or
R3, R4, R5, R6=H, R1=NH2, ---(X(R2)_{m'})ₘ--- represents a single bond, and
---(Y(R7)_{n'})ₙ--- represents -CH2- or -CH2-CH2-; or
R3, R4, R5, R6=H, R1=NH2, ---(X(R2)_{m'})ₘ--- represents -CH2- or -CH2-CH2- and ---(Y(R7)_{n'})ₙ--- represents a single bond.

Preferably, the compounds of the invention are defined as above, preferably with the exception of compounds where:
R3, R4, R5, R6=H, R1=CN, ---(X(R2)_{m'})ₘ --- represents a single bond, and
--- (Y(R7)_{n'})ₙ--- represents -C(=N-(2-,4-,6-trimethylphenyl))-, -C(=N-(2-,6-dimethylphenyl))-, -C(=N-(2-,6-diethylphenyl))-, -C(=N-(2-methylphenyl))-,
-C(=N-(2-ethyl-phenyl))-, -C(=N-(2-trifluoromethylphenyl))-, -C(=N-(2-isopropylphenyl))-, -C(=N-phenyl)-, -C(=N-(naphtyl)- or -C(=O)-, -CH2-, or
R3, R5, R6=H, R4=OMe, R1=CN, ---(X(R2)_{m'})ₘ--- represents a single bond, and
---(Y(R7)_{n'})ₙ--- represents -C(=O)-, or
R3, R4, R6=H, R5=OMe, R1=CN, ---(X(R2)_{m'})ₘ--- represents a single bond, and
---(Y(R7)_{n'})ₙ--- represents -C(=O)-, or
R3, R4, R5, R6=H, R1=NH2, ---(X(R2)_{m'})ₘ--- represents a single bond, and
---(Y(R7)_{n'})ₙ--- represents -CH2- or -CH2-CH2-; or
R3, R4, R5, R6=H, R1=NH2, ---(X(R2)_{m'})ₘ--- represents -CH2- and
---(Y(R7)_{n'})ₙ--- represents a single bond.

Preferably, R1 is chosen from the group consisting in H, CN, Hal, OAlk, OH, NRCN, C(CN)=C(OH)(OAlk), SR, NRR', C(O)NRR', Heterocycle, where Alk is optionally substituted by OAlk and where Heterocycle is optionally substituted by Hal.

Preferably, R3, R4, R5, R6 are each identical or different and are independently chosen from the group consisting in H, OAlk, OH, Alk, Hal.

---(Y(R7)_{n'})ₙ--- is a single bond or Y represents a carbon atom or a S atom;

Preferably, R2 is chosen from the group consisting in H, O.

Preferably, R7 is chosen from the group consisting in H, O, OH, N-OH, N-OAlk, N-Aryle, N-O-Aryle or N-O-Alk-Aryle or 2 R7 bound at the same Y form together with that Y an heterocycle.

Preferably, R and R' are each identical or different and are independently chosen from the group consisting in H, Alk.

More preferably, in formula (I), ---(X(R2)_{m'})ₘ--- represents a single bond, n is 1, n' is 1, Y is a carbon atom;
R1 is chosen from the group consisting in H, CN, Hal, OAlk, OH, NRCN, C(CN)=C(OH)(OAlk), SR, NRR', C(O)NRR', Heterocycle, where Alk is optionally substituted by OAlk and where Heterocycle is optionally substituted by Hal;
R3, R4, R5, R6 are each identical or different and are independently chosen from the group consisting in H, OAlk, OH, Alk, Hal;
R7 is chosen from the group consisting in O, N-OH, N-OAlk, N-Aryle, N-O-Aryle or N-O-Alk-Aryle;
R and R' are each identical or different and are independently chosen from the group consisting in H, Alk;
or their pharmaceutically acceptable salts, hydrates, or hydrated salts, or the polymorphic crystalline structures of these compounds or their optical isomers, racemates, diastereomers or enantiomers.

Preferred compounds of the invention are chosen from the group consisting in:
9-hydroxy-3-methoxy-9H-indeno[1,2-b]pyrazine-2-carbonitrile
3-methoxy-9-oxo-9H-indeno[1,2-b]pyrazine-2-carbonitrile
3-dimethylamino-9-oxo-9H-indeno[1,2-b]pyrazine-2-carbonitrile
3-(2-methoxy-ethoxy)-9-oxo-9H-indeno[1,2-b]pyrazine-2-carbonitrile
3-hydroxy-9-oxo-9H-indeno[1,2-b]pyrazine-2-carbonitrile
3-amino-9-oxo-9H-indeno[1,2-b]pyrazine-2-carbonitrile
3-(4,4-difluoro-piperidin-1-yl)-9-oxo-9H-indeno[1,2-b]pyrazine-2-carbonitrile
3-chloro-9-oxo-9H-indeno[1,2-b]pyrazine-2-carbonitrile
9-(1',3'-dioxolan-2'-yl)-9H-indeno[1,2-b]pyrazine-2,3-dicarbonitrile
2-cyano-9-[hydroxyimino]-9H-indeno[1,2-b]pyrazine-3-carboxylic acid amide
9-(methoxyimino)-9H-indeno[1,2-b]pyrazine-2,3-dicarbonitrile
9-(Allyloxyimino)-9H-indeno[1,2-b]pyrazine-2,3-dicarbonitrile
9-Benzyloxyimino-9H-indeno[1,2-b]pyrazine-2,3-dicarbonitrile
9-Ethoxyimino-9H-indeno[1,2-b]pyrazine-2,3-dicarbonitrile
9-Phenoxyimino-9H-indeno[1,2-b]pyrazine-2,3-dicarbonitrile
6-Methoxy-9H-indeno[1,2-b]pyrazine-2,3-dicarbonitrile
6,7-Dimethoxy-9H-indeno[1,2-b]pyrazine-2,3-dicarbonitrile
8-Methyl-9H-indeno[1,2-b]pyrazine-2,3-dicarbonitrile
7,8-Dimethoxy-9H-indeno[1,2-b]pyrazine-2,3-dicarbonitrile
6-Methyl-9H-indeno[1,2-b]pyrazine-2,3-dicarbonitrile
5,8-Dimethoxy-9H-indeno[1,2-b]pyrazine-2,3-dicarbonitrile
6-Methoxy-9-oxo-9H-indeno[1,2-b]pyrazine-2,3-dicarbonitrile
6,7-Dimethoxy-9-oxo-9H-indeno[1,2-b]pyrazine-2,3-dicarbonitrile
8-Methyl-9-oxo-9H-indeno[1,2-b]pyrazine-2,3-dicarbonitrile
7,8-Dimethoxy-9-oxo-9H-indeno[1,2-b]pyrazine-2,3-dicarbonitrile
6-Methyl-9-oxo-9H-indeno[1,2-b]pyrazine-2,3-dicarbonitrile
5,8-Dimethoxy-9-oxo-9H-indeno[1,2-b]pyrazine-2,3-dicarbonitrile
7-Chloro-9-oxo-9H-indeno[1,2-b]pyrazine-2,3-dicarbonitrile
7-Fluoro-9-oxo-9H-indeno[1,2-b]pyrazine-2,3-dicarbonitrile
7-Hydroxy-9-oxo-9H-indeno[1,2-b]pyrazine-2,3-dicarbonitrile
benzo[4,5]thieno[2,3-b]pyrazine-2,3-dicarbonitrile
5,10-dioxo-5,10-dihydro-benzo[g]quinoxaline-2,3-dicarbonitrile
9-[hydroxyimino]-9H-indeno[1,2-b]pyrazine-2,3-dicarbonitrile
2-cyano-9-oxo-9H-indeno[1,2-b]pyrazin-3-yl-cyanamide
3-(1-cyano-2-ethoxy-2-hydroxy-vinyl)-9-oxo-9H-indeno[1,2-b]pyrazine-2-carbonitrile
3-ethylsulfanyl-9-oxo-9H-indeno[1,2-b]pyrazine-2-carbonitrile
7-Chloro-9-methoxyimino-9H-indeno[1,2-b]pyrazine-2,3-dicarbonitrile
9-Allyloxyimino-7-chloro-9H-indeno[1,2-b]pyrazine-2,3-dicarbonitrile
6-Chloro-9-oxo-9H-indeno[1,2-b]pyrazine-2,3-dicarbonitrile
or their pharmaceutically acceptable salts, hydrates, or hydrated salts, or
the polymorphic crystalline structures of these compounds or their optical isomers, racemates, diastereomers or enantiomers.

According to another aspect, preferred compounds of the invention are chosen from the group consisting in:
9-hydroxy-3-methoxy-9H-indeno[1,2-b]pyrazine-2-carbonitrile
3-methoxy-9-oxo-9H-indeno[1,2-b]pyrazine-2-carbonitrile
3-dimethylamino-9-oxo-9H-indeno[1,2-b]pyrazine-2-carbonitrile
3-(2-methoxy-ethoxy)-9-oxo-9H-indeno[1,2-b]pyrazine-2-carbonitrile
3-hydroxy-9-oxo-9H-indeno[1,2-b]pyrazine-2-carbonitrile
3-amino-9-oxo-9H-indeno[1,2-b]pyrazine-2-carbonitrile
3-(4,4-difluoro-piperidin-1-yl)-9-oxo-9H-indeno[1,2-b]pyrazine-2-carbonitrile
3-chloro-9-oxo-9H-indeno[1,2-b]pyrazine-2-carbonitrile
9-(1',3'-dioxolan-2'-yl)-9H-indeno[1,2-b]pyrazine-2,3-dicarbonitrile
2-cyano-9-[hydroxyimino]-9H-indeno[1,2-b]pyrazine-3-carboxylic acid amide
9-(methoxyimino)-9H-indeno[1,2-b]pyrazine-2,3-dicarbonitrile
9-(Allyloxyimino)-9H-indeno[1,2-b]pyrazine-2,3-dicarbonitrile
9-Benzyloxyimino-9H-indeno[1,2-b]pyrazine-2,3-dicarbonitrile
9-Ethoxyimino-9H-indeno[1,2-b]pyrazine-2,3-dicarbonitrile
9-Phenoxyimino-9H-indeno[1,2-b]pyrazine-2,3-dicarbonitrile
6-Methoxy-9H-indeno[1,2-b]pyrazine-2,3-dicarbonitrile
6,7-Dimethoxy-9H-indeno[1,2-b]pyrazine-2,3-dicarbonitrile
8-Methyl-9H-indeno[1,2-b]pyrazine-2,3-dicarbonitrile
7,8-Dimethoxy-9H-indeno[1,2-b]pyrazine-2,3-dicarbonitrile
6-Methyl-9H-indeno[1,2-b]pyrazine-2,3-dicarbonitrile
5,8-Dimethoxy-9H-indeno[1,2-b]pyrazine-2,3-dicarbonitrile
6-Methoxy-9-oxo-9H-indeno[1,2-b]pyrazine-2,3-dicarbonitrile
6,7-Dimethoxy-9-oxo-9H-indeno[1,2-b]pyrazine-2,3-dicarbonitrile
8-Methyl-9-oxo-9H-indeno[1,2-b]pyrazine-2,3-dicarbonitrile
7,8-Dimethoxy-9-oxo-9H-indeno[1,2-b]pyrazine-2,3-dicarbonitrile
6-Methyl-9-oxo-9H-indeno[1,2-b]pyrazine-2,3-dicarbonitrile
5,8-Dimethoxy-9-oxo-9H-indeno[1,2-b]pyrazine-2,3-dicarbonitrile
7-Chloro-9-oxo-9H-indeno[1,2-b]pyrazine-2,3-dicarbonitrile
7-Fluoro-9-oxo-9H-indeno[1,2-b]pyrazine-2,3-dicarbonitrile
7-Hydroxy-9-oxo-9H-indeno[1,2-b]pyrazine-2,3-dicarbonitrile
benzo[4,5]thieno[2,3-b]pyrazine-2,3-dicarbonitrile
5,10-dioxo-5,10-dihydro-benzo[g]quinoxaline-2,3-dicarbonitrile
2-cyano-9-oxo-9H-indeno[1,2-b]pyrazin-3-yl-cyanamide
3-(1-cyano-2-ethoxy-2-hydroxy-vinyl)-9-oxo-9H-indeno[1,2-b]pyrazine-2-carbonitrile
3-ethylsulfanyl-9-oxo-9H-indeno[1,2-b]pyrazine-2-carbonitrile
7-Chloro-9-methoxyimino-9H-indeno[1,2-b]pyrazine-2,3-dicarbonitrile
9-Allyloxyimino-7-chloro-9H-indeno[1,2-b]pyrazine-2,3-dicarbonitrile
6-Chloro-9-oxo-9H-indeno[1,2-b]pyrazine-2,3-dicarbonitrile
or their pharmaceutically acceptable salts, hydrates, or hydrated salts, or the polymorphic crystalline structures of these compounds or their optical isomers, racemates, diastereomers or enantiomers.

More preferred compounds of the invention are chosen from the group consisting in:
2-cyano-9-[hydroxyimino]-9H-indeno[1,2-b]pyrazine-3-carboxylic acid amide
9-(methoxyimino)-9H-indeno[1,2-b]pyrazine-2,3-dicarbonitrile
9-Benzyloxyimino-9H-indeno[1,2-b]pyrazine-2,3-dicarbonitrile (13c).
9-Ethoxyimino-9H-indeno[1,2-b]pyrazine-2,3-dicarbonitrile (13d).
9-Phenoxyimino-9H-indeno[1,2-b]pyrazine-2,3-dicarbonitrile (13e).
8-Methyl-9-oxo-9H-indeno[1,2-b]pyrazine-2,3-dicarbonitrile
6-Methyl-9-oxo-9H-indeno[1,2-b]pyrazine-2,3-dicarbonitrile
5,8-Dimethoxy-9-oxo-9H-indeno[1,2-b]pyrazine-2,3-dicarbonitrile
7-Chloro-9-oxo-9H-indeno[1,2-b]pyrazine-2,3-dicarbonitrile
7-Fluoro-9-oxo-9H-indeno[1,2-b]pyrazine-2,3-dicarbonitrile
or their pharmaceutically acceptable salts, hydrates, or hydrated salts, or the polymorphic crystalline structures of these compounds or their optical isomers, racemates, diastereomers or enantiomers.

According to a further object, the present invention concerns also the pharmaceutical compositions comprising a compound of formula (I) 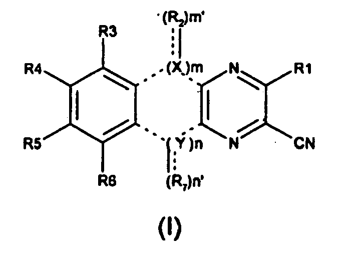 wherein :
m is 0 ; 1 or 2 , wherein when m=0, ----- (X(R2)_{m'})ₘ ----- is none so as to form an open ring or a single bond;
n is 0, 1 or 2, , wherein when n=0, ----- (Y(R7)_{n'})ₙ ----- is none so as to form an open ring or a single bond;
m' and n' are independently 0, 1 or 2;
X is a carbon atom or S or N;
Y is a carbon atom, or S or N;

Provided m and n are not simultaneously 0;
- - - - is either a single or double bond, as appropriate ;
- - - - - - is either none or a single bond, as appropriate;
R1 is chosen from the group consisting in H, CN, Hal, OAlk, OH, NRCN, C(CN)=C(OH)(OAlk), SR, NRR', C(O)NRR', Heterocycle, Aryle, Heteroaryle, where Alk, Aryle, Heteroaryle, Heterocycle are optionally substituted by Hal, NRR', CN, OH, CF₃, Aryle, Heteroaryle , OAlk;
R3, R4, R5, R6 are each identical or different and are independently chosen from the group consisting in H, OAlk, Alk, Hal, NRR', CN, OH, CF₃, Aryle, Heteroaryle;
R2 is chosen from the group consisting in H, O, OH, N-OH, N-Aryle, N-OAlk, NO-Aryle, N-O-Alk-Aryle or 2 R2 bound at the same X form together with that X an heterocycle; wherein said Alk, Aryle or heterocycle are optionally substituted by OAlk, Alk, Hal, NRR', CN, OH, CF₃;
R7 is chosen from the group consisting in H, O, OH, N-OH, N-Aryle, N-OAlk, NO-Aryle, N-O-Alk-Aryle or 2 R7 bound at the same Y form together with that Y an heterocycle; wherein said Alk, Aryle or heterocycle are optionally substituted by OAlk, Alk, Hal, NRR', CN, OH, CF₃;
R and R' are each identical or different and are independently chosen from the group consisting in H, Alk, wherein Alk is optionally substituted by Hal, NRR', CN, OH, CF₃, Aryle, Heteroaryle;
or their pharmaceutically acceptable salts, hydrates, or hydrated salts, or the polymorphic crystalline structures of these compounds or their optical isomers, racemates, diastereomers or enantiomers.

According to a still further object, the present invention concerns the use of a compound of formula (I) as defined in respect of the pharmaceutical compositions of the invention, for the preparation of a medicament for inhibiting cysteine protease.

Preferred embodiments of formula (I) for the pharmaceutical compositions and use of the invention are defined as above.

Preferred compounds for the pharmaceutical compositions and use of the invention are chosen from the group consisting in:
9-oxo-9H-indeno[1,2-b]pyrazine-2,3-dicarbonitrile
9-hydroxy-3-methoxy-9H-indeno[1,2-b]pyrazine-2-carbonitrile
3-methoxy-9-oxo-9H-indeno[1,2-b]pyrazine-2-carbonitrile
3-dimethylamino-9-oxo-9H-indeno[1,2-b]pyrazine-2-carbonitrile
3-(2-methoxy-ethoxy)-9-oxo-9H-indeno[1,2-b]pyrazine-2-carbonitrile
3-hydroxy-9-oxo-9H-indeno[1,2-b]pyrazine-2-carbonitrile
3-amino-9-oxo-9H-indeno[1,2-b]pyrazine-2-carbonitrile
3-(4,4-difluoro-piperidin-1-yl)-9-oxo-9H-indeno[1,2-b]pyrazine-2-carbonitrile
3-chloro-9-oxo-9H-indeno[1,2-b]pyrazine-2-carbonitrile
9-(1',3'-dioxolan-2'-yl)-9H-indeno[1,2-b]pyrazine-2,3-dicarbonitrile
2-cyano-9-[hydroxyimino]-9H-indeno[1,2-b]pyrazine-3-carboxylic acid amide
9-[hydroxyimino]-9H-indeno[1,2-b]pyrazine-2,3-dicarbonitrile
9-(methoxyimino)-9H-indeno[1,2-b]pyrazine-2,3-dicarbonitrile
9-(Allyloxyimino)-9H-indeno[1,2-b]pyrazine-2,3-dicarbonitrile
9-Benzyloxyimino-9H-indeno[1,2-b]pyrazine-2,3-dicarbonitrile
9-Ethoxyimino-9H-indeno[1,2-b]pyrazine-2,3-dicarbonitrile
9-Phenoxyimino-9H-indeno[1,2-b]pyrazine-2,3-dicarbonitrile
9-[phenylimino]-9H-indeno[1,2-b]pyrazine-2,3-dicarbonitrile
6-Methoxy-9H-indeno[1,2-b]pyrazine-2,3-dicarbonitrile
6,7-Dimethoxy-9H-indeno[1,2-b]pyrazine-2,3-dicarbonitrile
8-Methyl-9H-indeno[1,2-b]pyrazine-2,3-dicarbonitrile
7,8-Dimethoxy-9H-indeno[1,2-b]pyrazine-2,3-dicarbonitrile
6-Methyl-9H-indeno[1,2-b]pyrazine-2,3-dicarbonitrile
5,8-Dimethoxy-9H-indeno[1,2-b]pyrazine-2,3-dicarbonitrile
6-Methoxy-9-oxo-9H-indeno[1,2-b]pyrazine-2,3-dicarbonitrile
6,7-Dimethoxy-9-oxo-9H-indeno[1,2-b]pyrazine-2,3-dicarbonitrile
8-Methyl-9-oxo-9H-indeno[1,2-b]pyrazine-2,3-dicarbonitrile
7,8-Dimethoxy-9-oxo-9H-indeno[1,2-b]pyrazine-2,3-dicarbonitrile
6-Methyl-9-oxo-9H-indeno[1,2-b]pyrazine-2,3-dicarbonitrile
5,8-Dimethoxy-9-oxo-9H-indeno[1,2-b]pyrazine-2,3-dicarbonitrile
7-Chloro-9-oxo-9H-indeno[1,2-b]pyrazine-2,3-dicarbonitrile
7-Fluoro-9-oxo-9H-indeno[1,2-b]pyrazine-2,3-dicarbonitrile
7-Methoxy-9-oxo-9H-indeno[1,2-b]pyrazine-2,3-dicarbonitrile
7-Hydroxy-9-oxo-9H-indeno[1,2-b]pyrazine-2,3-dicarbonitrile benzo[4,5]thieno[2,3-b]pyrazine-2,3-dicarbonitrile
5,10-dioxo-5,10-dihydro-benzo[g]quinoxaline-2,3-dicarbonitrile
2-cyano-9-oxo-9H-indeno[1,2-b]pyrazin-3-yl-cyanamide
3-(1-cyano-2-ethoxy-2-hydroxy-vinyl)-9-oxo-9H-indeno[1,2-b]pyrazine-2-carbonitrile
3-ethylsulfanyl-9-oxo-9H-indeno[1,2-b]pyrazine-2-carbonitrile
7-Chloro-9-methoxyimino-9H-indeno[1,2-b]pyrazine-2,3-dicarbonitrile
9-Allyloxyimino-7-chloro-9H-indeno[1,2-b]pyrazine-2,3-dicarbonitrile
6-Chloro-9-oxo-9H-indeno[1,2-b]pyrazine-2,3-dicarbonitrile
or their pharmaceutically acceptable salts, hydrates, or hydrated salts, or the polymorphic crystalline structures of these compounds or their optical isomers, racemates, diastereomers or enantiomers.

More preferred compounds for the pharmaceutical compositions and use of the invention are chosen from the group consisting in:
9-oxo-9H-indeno[1,2-b]pyrazine-2,3-dicarbonitrile
2-cyano-9-[hydroxyimino]-9H-indeno[1,2-b]pyrazine-3-carboxylic acid amide
9-(methoxyimino)-9H-indeno[1,2-b]pyrazine-2,3-dicarbonitrile
9-Benzyloxyimino-9H-indeno[1,2-b]pyrazine-2,3-dicarbonitrile (13c).
9-Ethoxyimino-9H-indeno[1,2-b]pyrazine-2,3-dicarbonitrile (13d).
9-Phenoxyimino-9H-indeno[1,2-b]pyrazine-2,3-dicarbonitrile (13e).
9-[phenylimino]-9H-indeno[1,2-b]pyrazine-2,3-dicarbonitrile
8-Methyl-9-oxo-9H-indeno[1,2-b]pyrazine-2,3-dicarbonitrile
6-Methyl-9-oxo-9H-indeno[1,2-b]pyrazine-2,3-dicarbonitrile
5,8-Dimethoxy-9-oxo-9H-indeno[1,2-b]pyrazine-2,3-dicarbonitrile
7-Chloro-9-oxo-9H-indeno[1,2-b]pyrazine-2,3-dicarbonitrile
7-Fluoro-9-oxo-9H-indeno[1,2-b]pyrazine-2,3-dicarbonitrile
or their pharmaceutically acceptable salts, hydrates, or hydrated salts, or the polymorphic crystalline structures of these compounds or their optical isomers, racemates, diastereomers or enantiomers.

### As used hereabove or hereafter:

Alk represents alkyl, alken or alkyn.

"Alkyl" means an aliphatic hydrocarbon group which may be straight or branched having 1 to 20 carbon atoms in the chain. Preferred alkyl groups have 1 to 12 carbon atoms in the chain. "Branched" means that one or more lower alkyl groups such as methyl, ethyl or propyl are attached to a linear alkyl chain. Exemplary alkyl groups include methyl, ethyl, *n*-propyl, *i*-propyl, *n*-butyl, *t*-butyl, *n*-pentyl, 3-pentyl, octyl, nonyl, decyl.

"Alken" means an aliphatic hydrocarbon group containing a carbon-carbon double bond and which may be straight or branched having 2 to 15 carbon atoms in the chain. Preferred alkenyl groups have 2 to 12 carbon atoms in the chain; and more preferably about 2 to 4 carbon atoms in the chain. Exemplary alkenyl groups include ethenyl, propenyl, *n*-butenyl, *i*-butenyl, 3-methylbut-2-enyl, *n*-pentenyl, heptenyl, octenyl, nonenyl, decenyl.

"Alkyn" means an aliphatic hydrocarbon group containing a carbon-carbon triple bond and which may be straight or branched having 2 to 15 carbon atoms in the chain. Preferred alkynyl groups have 2 to12 carbon atoms in the chain; and more preferably 2 to 4 carbon atoms in the chain. Exemplary alkynyl groups include ethynyl, propynyl, *n*-butynyl, 2-butynyl, 3-methylbutynyl, *n*-pentynyl, heptynyl, octynyl and decynyl.

"Halogen atom" refers to fluorine, chlorine, bromine or iodine atom; preferably fluorine and chlorine atom.

"Aryl" means an aromatic monocyclic or multicyclic hydrocarbon ring system of 6 to 14 carbon atoms, preferably of 6 to 10 carbon atoms. Exemplary aryl groups include phenyl or naphthyl.

As used herein, the terms "heterocycle" or "heterocyclic" refer to a saturated, partially unsaturated or unsaturated, non aromatic stable 3 to 14, preferably 5 to 10 membered mono, bi or multicyclic rings wherein at least one member of the ring is a hetero atom. Typically, heteroatoms include, but are not limited to, oxygen, nitrogen, sulfur, selenium, and phosphorus atoms. Preferable heteroatoms are oxygen, nitrogen and sulfur.

Suitable heterocycles are also disclosed in The Handbook of Chemistry and Physics, 76th Edition, CRC Press, Inc., 1995-1996, p. 2-25 to 2-26, the disclosure of which is hereby incorporated by reference.

Preferred non aromatic heterocyclic include, but are not limited to pyrrolidinyl, pyrazolidinyl, imidazolidinyl, oxiranyl, tetrahydrofuranyl, dioxolanyl, tetrahydropyranyl, dioxanyl, dioxolanyl, piperidyl, piperazinyl, morpholinyl, pyranyl, imidazolinyl, pyrrolinyl, pyrazolinyl, thiazolidinyl, tetrahydrothiopyranyl, dithianyl, thiomorpholinyl, dihydro-pyranyl, tetrahydropyranyl, dihydropyranyl, tetrahydropyridyl, dihydropyridyl, tetrahydropyrinidinyl, dihydrothiopyranyl, azepanyl, as well as the fused systems resulting from the condensation with a phenyl group.

As used herein, the term "heteroaryl" or aromatic heterocycles refers to a 5 to 14, preferably 5 to 10 membered aromatic hetero, mono-, bi- or multicyclic ring. Examples include pyrrolyl, pyridyl, pyrazolyl, thienyl, pyrimidinyl, pyrazinyl, tetrazolyl, indolyl, quinolinyl, purinyl, imidazolyl, thienyl, thiazolyl, benzothiazolyl, furanyl, benzofuranyl, 1,2,4-thiadiazolyl, isothiazolyl, triazoyl, tetrazolyl, isoquinolyl, benzothienyl, isobenzofuryl, pyrazolyl, carbazolyl, benzimidazolyl, isoxazolyl, pyridyl-N-oxide , as well as the fused systems resulting from the condensation with a phenyl group.

"Alkyl", "cycloalkyl", "alkenyl", "alkynyl", "aryl", "heteroaryl", "heterocycle" and the likes refers also to the corresponding "alkylene", "cycloalkylene", "alkenylene", "alkynylene", "arylene", "heteroarylene", "heterocyclene" and the likes which are formed by the removal of two hydrogen atoms.

As used herein, the term "patient" refers to either an animal, such as a valuable animal for breeding, company or preservation purposes, or preferably a human or a human child, which is afflicted with, or has the potential to be afflicted with one or more diseases and conditions described herein.

As used herein, a "therapeutically effective amount" refers to an amount of a compound of the present invention which is effective in preventing, reducing, eliminating, treating or controlling the symptoms of the herein-described diseases and conditions. The term "controlling" is intended to refer to all processes wherein there may be a slowing, interrupting, arresting, or stopping of the progression of the diseases and conditions described herein, but does not necessarily indicate a total elimination of all disease and condition symptoms, and is intended to include prophylactic treatment.

As used herein, the term "pharmaceutically acceptable" refers to those compounds, materials, excipients, compositions or dosage forms which are, within the scope of sound medical judgment, suitable for contact with the tissues of human beings and animals without excessive toxicity, irritation, allergic response or other problem complications commensurate with a reasonable benefit/risk ratio.

As used herein, "pharmaceutically acceptable salts" refer to derivatives of the disclosed compounds wherein the parent compound is modified by making acid or base salts thereof. The pharmaceutically acceptable salts include the conventional non-toxic salts or the quaternary ammonium salts of the parent compound formed, for example, from non-toxic inorganic or organic acids. For example, such conventional non-toxic salts include those derived from inorganic acids such as hydrochloric, hydrobromic, sulfuric, sulfamic, phosphoric, nitric and the like; and the salts prepared from organic acids such as acetic, propionic, succinic, tartaric, citric, methanesulfonic, benzenesulfonic, glucoronic, glutamic, benzoic, salicylic, toluenesulfonic, oxalic, fumaric, maleic, lactic and the like. Further addition salts include ammonium salts such as tromethamine, meglumine, epolamine, etc., metal salts such as sodium, potassium, calcium, zinc or magnesium.

The pharmaceutically acceptable salts of the present invention can be synthesized from the parent compound which contains a basic or acidic moiety by conventional chemical methods. Generally, such salts can be prepared by reacting the free acid or base forms of these compounds with a stoichiometric amount of the appropriate base or acid in water or in an organic solvent, or in a mixture of the two. Generally, non-aqueous media like ether, ethyl acetate, ethanol, isopropanol, or acetonitrile are preferred. Lists of suitable salts are found in Remington's Pharmaceutical Sciences, 17th ed., Mack Publishing Company, Easton, PA, 1985, p. 1418, the disclosure of which is hereby incorporated by reference. The compounds of the general formula (I) having geometrical and stereoisomers are also a part of the invention.

According to a further object, the present invention is also concerned with the process of preparation of the compounds of formula (I).

The compounds and process of the present invention may be prepared in a number of ways well known to those skilled in the art. The compounds can be synthesized, for example, by application or adaptation of the methods described below, or variations thereon as appreciated by the skilled artisan. The appropriate modifications and substitutions will be readily apparent and well known or readily obtainable from the scientific literature to those skilled in the art.

In particular, such methods can be found in R.C. Larock, Comprehensive Organic Transformations, Wiley-VCH Publishers, 1999.

It will be appreciated that the compounds of the present invention may contain one or more asymmetrically substituted carbon atoms, and may be isolated in optically active or racemic forms. Thus, all chiral, diastereomeric, racemic forms and all geometric isomeric forms of a structure are intended, unless the specific stereochemistry or isomeric form is specifically indicated. It is well known in the art how to prepare and isolate such optically active forms. For example, mixtures of stereoisomers may be separated by standard techniques including, but not limited to, resolution of racemic forms, normal, reverse-phase, and chiral chromatography, preferential salt formation, recrystallization, and the like, or by chiral synthesis either from chiral starting materials or by deliberate synthesis of target chiral centers.

Compounds of the present invention may be prepared by a variety of synthetic routes. The reagents and starting materials are commercially available, or readily synthesized by well-known techniques by one of ordinary skill in the arts. All substituents, unless otherwise indicated, are as previously defined.

In the reactions described hereinafter, it may be necessary to protect reactive functional groups, for example hydroxy, amino, imino, thio or carboxy groups, where these are desired in the final product, to avoid their unwanted participation in the reactions. Conventional protecting groups may be used in accordance with standard practice, for examples see T.W. Greene and P. G. M. Wuts in Protective Groups in Organic Chemistry, 3rd ed., John Wiley and Sons, 1999; J. F. W. McOmie in Protective Groups in Organic Chemistry, Plenum Press, 1973.

Some reactions may be carried out in the presence of a base. There is no particular restriction on the nature of the base to be used in this reaction, and any base conventionally used in reactions of this type may equally be used here, provided that it has no adverse effect on other parts of the molecule. Examples of suitable bases include: sodium hydroxide, potassium carbonate, triethylamine, alkali metal hydrides, such as sodium hydride and potassium hydride; alkyllithium compounds, such as methyllithium and butyllithium; and alkali metal alkoxides, such as sodium methoxide and sodium ethoxide.

Usually, reactions are carried out in a suitable solvent. A variety of solvents may be used, provided that it has no adverse effect on the reaction or on the reagents involved. Examples of suitable solvents include: hydrocarbons, which may be aromatic, aliphatic or cycloaliphatic hydrocarbons, such as hexane, cyclohexane, benzene, toluene and xylene; amides, such as dimethylformamide; alcohols such as ethanol and methanol and ethers, such as diethyl ether and tetrahydrofuran.

The reactions can take place over a wide range of temperatures. In general, we find it convenient to carry out the reaction at a temperature of from 0°C to 150°C (more preferably from about room temperature to 100°C). The time required for the reaction may also vary widely, depending on many factors, notably the reaction temperature and the nature of the reagents. However, provided that the reaction is effected under the preferred conditions outlined above, a period of from 3 hours to 20 hours will usually suffice.

The compound thus prepared may be recovered from the reaction mixture by conventional means. For example, the compounds may be recovered by distilling off the solvent from the reaction mixture or, if necessary after distilling off the solvent from the reaction mixture, pouring the residue into water followed by extraction with a water-immiscible organic solvent and distilling off the solvent from the extract. Additionally, the product can, if desired, be further purified by various well known techniques, such as recrystallization, reprecipitation or the various chromatography techniques, notably column chromatography or preparative thin layer chromatography.

The process of preparation of a compound of formula (I) of the invention is a further object of the present invention.

According to a first aspect, compounds of the invention of formula (I) can be obtained from corresponding compounds of formula (II) 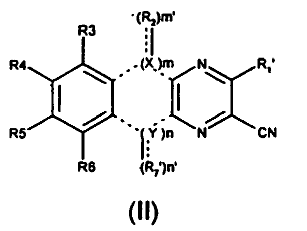 wherein R3, R4, R5, R6, X, Y, m, m', n, n' are defined as in formula (I) and R7' is R7 as defined in formula (I) or a precursor thereof and R1' is R1 as defined in formula (I) or a precursor thereof.

According to the present invention, the expression "precursor group" of a functional group refers to any group which can, by one or more reactions, lead to the desired function, by means of one or more suitable reagents. Those reactions include de-protection, as well as usual addition, substitution or functionalization reactions.

Preferably, in formula (II), R1' represents a CN group.

Generally, the compound of formula (I) is obtained from compound of formula (II) by one or more step allowing a precursor function to be transformed into the desired -R1 group. Simultaneously, the R7' group can be transformed to the desired R7, if appropriate.

The compounds of formula (II) can be obtained from corresponding compounds of formula (III): 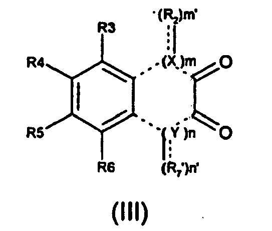 wherein R3, R4, R5, R6, X, Y, m, m', n, n' are defined as in formula (I) and R7' is defined as in formula (II). Generally, when R1'=CN, this reaction is usually carried out in the presence of diaminomaleodinitrile.

According to an alternative embodiment, the compounds of formula (II) can be obtained from corresponding compounds of formula (III'): 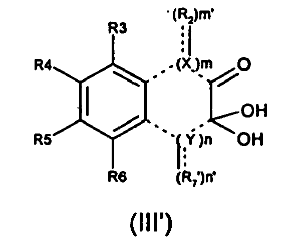 wherein R3, R4, R5, R6, X, Y, m, m', n, n' are defined as in formula (I) and R7' is defined as in formula (III').

Generally, when R1'=CN, this reaction is usually carried out in the presence of diaminomaleodinitrile.

According to an alternative embodiment, the compound of formula (II) can be obtained from corresponding compounds of formula (IV): 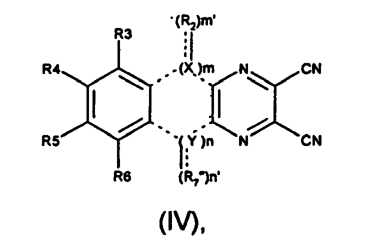 wherein R3, R4, R5, R6, X, Y, m, m', n, n' are defined as in formula (I) and R7" represents R7' or a precursor thereof, if appropriate.

The compound of formula (III) can be obtained from a corresponding compound of formula (V): 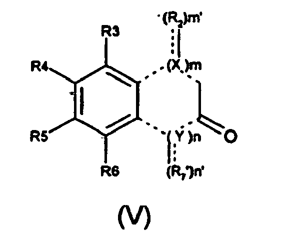 wherein R3, R4, R5, R6, X, Y, m, m', n, n' are defined as in formula (I) and R7' is defined as in formula (II).

The compound of formula (IV) can be obtained from a corresponding compound of formula (III). Generally, this reaction is carried in the presence of diaminomaleodinitrile.

The above reactions can be carried out by the skilled person by applying or adapting the methods illustrated in the examples hereinafter.

Further, the process of the invention may also comprise the additional step of isolating the compound of formula (I). This can be done by the skilled person by any of the known conventional means, such as the recovery methods described above.

The starting products are commercially available or may be obtained by applying or adapting any known methods or those described in the examples.

The synthesis may also be carried out in one pot as a multicomponent reaction.

According to a further object, the present invention is also concerned with pharmaceutical compositions comprising a compound of formula (I) together with pharmaceutically acceptable excipients.

The compounds of the invention are useful for inhibiting cysteine proteases, in particular de-ubiquitination enzymes (such as USPs and UCHs), caspases, cathepsins (in particular cathepsin B, D, K, S and the likes), calpains as well as viral, bacterial or parasitic cysteine proteases in patients in the need thereof.

The compounds of the invention are particularly useful for treating and/or preventing cancer and metastasis, neurodegenerative diseases such as Alzheimer's disease and Parkinson's disease, deafness, disorders associated with ageing, inflammatory disorders, arthritis, osteoporosis, hepatitis, liver failure, cardiac ischemia and failure, stroke, atherosclerosis, renal failure, diabetes, cataract; viral acute or latent infections by Herpes simplex virus-1, Epstein-Barr virus, SARS coronavirus, rhinoviruses, poliomyelitis virus, hepatitis A virus, hepatitis C virus, adenoviruses, and the like; bacterial or fungal infections by pathogenic agents belonging to the *Streptococcus sp., Staphylococcus sp., Clostidium sp., Aspergillus sp.,* genera and the like; protozoal infections by species members of the *Trypanosoma sp., Plasmodium sp., Leishmania sp., Trichomonas sp., Entamoeba sp., Giardia sp., Toxoplasma sp., Cryptosporidium sp.,* genera and the like; flat or round worm infections by species members of the *Fasciola sp., Schistosoma sp., Onchocerca sp., Ascaris sp., Taenia sp., Caenorhabitis sp., Toxocara sp., Haemonchus sp., Ancylostoma sp., Trichuris sp., Trichinella sp., Strongyloides sp., Brugia sp.,* genera and the like; as well as immunological, immunoregulatory or antigen presentation disorders.

The present invention also concerns the corresponding methods of treatment comprising the administration of a compound of the invention together with a pharmaceutically acceptable carrier or excipient to a patient in the need thereof.

The identification of those subjects who are in need of treatment of herein-described diseases and conditions is well within the ability and knowledge of one skilled in the art. A veterinarian or a physician skilled in the art can readily identify, by the use of clinical tests, physical examination, medical/family history or biological and diagnostic tests, those subjects who are in need of such treatment.

A therapeutically effective amount can be readily determined by the attending diagnostician, as one skilled in the art, by the use of conventional techniques and by observing results obtained under analogous circumstances. In determining the therapeutically effective amount, a number of factors are considered by the attending diagnostician, including, but not limited to: the species of subject; its size, age, and general health; the specific disease involved; the degree of involvement or the severity of the disease; the response of the individual subject; the particular compound administered; the mode of administration; the bioavailability characteristic of the preparation administered; the dose regimen selected; the use of concomitant medication; and other relevant circumstances.

The amount of a compound of formula (I), which is required to achieve the desired biological effect, will vary depending upon a number of factors, including the chemical characteristics (e.g. hydrophobicity) of the compounds employed, the potency of the compounds, the type of disease, the species to which the patient belongs, the diseased state of the patient, the route of administration, the bioavailability of the compound by the chosen route, all factors which dictate the required dose amounts, delivery and regimen to be administered.

"Pharmaceutically" or "pharmaceutically acceptable" refer to molecular entities and compositions that do not produce an adverse, allergic or other untoward reaction when administered to an animal, or a human, as appropriate.

As used herein, "pharmaceutically acceptable excipient" includes any carriers, diluents, adjuvants, or vehicles, such as preserving or antioxidant agents, fillers, disintegrating agents, wetting agents, emulsifying agents, suspending agents, solvents, dispersion media, coatings, antibacterial and antifungal agents, isotonic and absorption delaying agents and the like. The use of such media and agents for pharmaceutical active substances is well known in the art. Except insofar as any conventional media or agent is incompatible with the active ingredient, its use in the therapeutic compositions is contemplated. Supplementary active ingredients can also be incorporated into the compositions as suitable therapeutic combinations.

In the context of the invention, the term "treating" or "treatment", as used herein, means reversing, alleviating, inhibiting the progress of, or preventing the disorder or condition to which such term applies, or one or more symptoms of such disorder or condition.

"Therapeutically effective amount" means an amount of a compound/medicament according to the present invention effective in preventing or treating a pathological condition requiring the inhibition of an active cysteine protease involved in its pathogenesis.

According to the invention, the term "patient", or "patient in need thereof", is intended for an animal or a human being affected or likely to be affected with a pathological condition involving an active cysteine protease in its pathogenesis. Preferably, the patient is human.

In general terms, the compounds of this invention may be provided in an aqueous physiological buffer solution containing 0.1 to 10 % w/v compound for parenteral administration. Typical dose ranges are from 1 µg/kg to 0.1 g/kg of body weight per day; a preferred dose range is from 0.01 mg/kg to 10 mg/kg of body weight per day or an equivalent dose in a human child. The preferred dosage of drug to be administered is likely to depend on such variables as the type and extent of progression of the disease or disorder, the overall health status of the particular patient, the relative biological efficacy of the compound selected, the formulation of the compound, the route of administration (intravenous, intramuscular, or other), the pharmacokinetic properties of the compound by the chosen delivery route, and the speed (bolus or continuous infusion) and schedule of administrations (number of repetitions in a given period of time).

The compounds of the present invention are also capable of being administered in unit dose forms, wherein the term "unit dose" means a single dose which is capable of being administered to a patient, and which can be readily handled and packaged, remaining as a physically and chemically stable unit dose comprising either the active compound itself, or as a pharmaceutically acceptable composition, as described hereinafter. As such, typical total daily dose ranges are from 0.01 to 100 mg/kg of body weight. By way of general guidance, unit doses for humans range from 1 mg to 3000 mg per day. Preferably the unit dose range is from 1 to 500 mg administered one to six times a day, and even more preferably from 10 mg to 500 mg, once a day. Compounds provided herein can be formulated into pharmaceutical compositions by admixture with one or more pharmaceutically acceptable excipients. Such unit dose compositions may be prepared for use by oral administration, particularly in the form of tablets, simple capsules or soft gel capsules; or intranasally, particularly in the form of powders, nasal drops, or aerosols; or dermally, for example, topically in ointments, creams, lotions, gels or sprays, or via trans-dermal patches.

The compositions may conveniently be administered in unit dosage form and may be prepared by any of the methods well known in the pharmaceutical art, for example, as described in Remington: The Science and Practice of Pharmacy, 20th ed.; Gennaro, A. R., Ed.; Lippincott Williams & Wilkins: Philadelphia, PA, 2000.

Preferred formulations include pharmaceutical compositions in which a compound of the present invention is formulated for oral or parenteral administration.

For oral administration, tablets, pills, powders, capsules, troches and the like can contain one or more of any of the following ingredients, or compounds of a similar nature: a binder such as microcrystalline cellulose, or gum tragacanth; a diluent such as starch or lactose; a disintegrant such as starch and cellulose derivatives; a lubricant such as magnesium stearate; a glidant such as colloidal silicon dioxide; a sweetening agent such as sucrose or saccharin; or a flavoring agent such as peppermint, or methyl salicylate. Capsules can be in the form of a hard capsule or soft capsule, which are generally made from gelatin blends optionally blended with plasticizers, as well as a starch capsule. In addition, dosage unit forms can contain various other materials that modify the physical form of the dosage unit, for example, coatings of sugar, shellac, or enteric agents. Other oral dosage forms syrup or elixir may contain sweetening agents, preservatives, dyes, colorings, and flavorings. In addition, the active compounds may be incorporated into fast dissolve, modified-release or sustained-release preparations and formulations, and wherein such sustained-release formulations are preferably bimodal. Preferred tablets contain lactose, cornstarch, magnesium silicate, croscarmellose sodium, povidone, magnesium stearate, or talc in any combination.

Liquid preparations for parenteral administration include sterile aqueous or non-aqueous solutions, suspensions, and emulsions. The liquid compositions may also include binders, buffers, preservatives, chelating agents, sweetening, flavoring and coloring agents, and the like. Non-aqueous solvents include alcohols, propylene glycol, polyethylene glycol, vegetable oils such as olive oil, and organic esters such as ethyl oleate. Aqueous carriers include mixtures of alcohols and water, buffered media, and saline. In particular, biocompatible, biodegradable lactide polymer, lactide/glycolide copolymer, or polyoxyethylene-polyoxypropylene copolymers may be useful excipients to control the release of the active compounds. Intravenous vehicles can include fluid and nutrient replenishers, electrolyte replenishers, such as those based on Ringer's dextrose, and the like. Other potentially useful parenteral delivery systems for these active compounds include ethylene-vinyl acetate copolymer particles, osmotic pumps, implantable infusion systems, and liposomes.

Alternative modes of administration include formulations for inhalation, which include such means as dry powder, aerosol, or drops. They may be aqueous solutions containing, for example, polyoxyethylene-9-lauryl ether, glycocholate and deoxycholate, or oily solutions for administration in the form of nasal drops, or as a gel to be applied intranasally. Formulations for buccal administration include, for example, lozenges or pastilles and may also include a flavored base, such as sucrose or acacia, and other excipients such as glycocholate. Formulations suitable for rectal administration are preferably presented as unit-dose suppositories, with a solid based carrier, such as cocoa butter, and may include a salicylate. Formulations for topical application to the skin preferably take the form of an ointment, cream, lotion, paste, gel, spray, aerosol, or oil. Carriers which can be used include petroleum jelly, lanolin, polyethylene glycols, alcohols, or their combinations. Formulations suitable for transdermal administration can be presented as discrete patches and can be lipophilic emulsions or buffered, aqueous solutions, dissolved and/or dispersed in a polymer or an adhesive.

The invention is further illustrated but not restricted by the description in the following examples.

Representative compounds of the invention are summarized in the table below:

| **Formula** | **Experimental N°** |
|---|---|
| 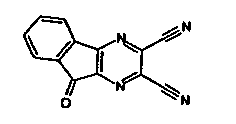 | **Example 1** |
| 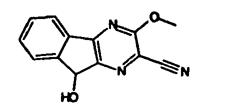 | **Example 2** |
| 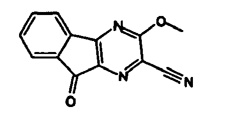 | **Example 3** |
| 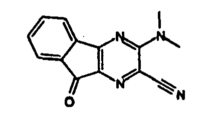 | **Example 4** |
| 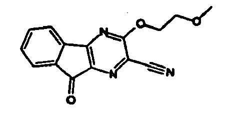 | **Example 5** |
| 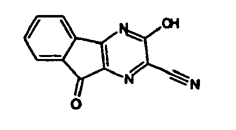 | **Example 6** |
| 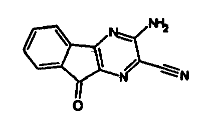 | **Example 7** |
| 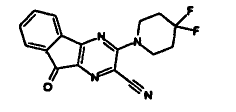 | **Example 8** |
| 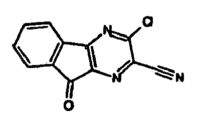 | **Example 9** |
| 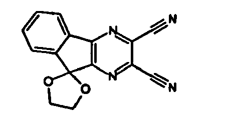 | **Example 10** |
| 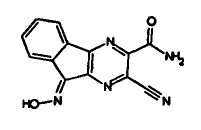 | **Example 11** |
| 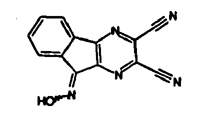 | **Example 12** |
| 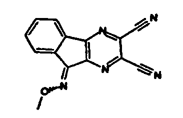 | **Example 13** |
| 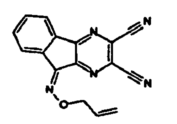 | **Example 13b** |
| 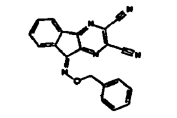 | **Example 13C** |
| 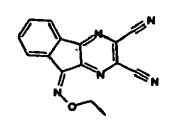 | **Example 13d** |
| 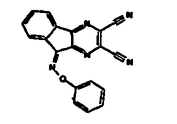 | **Example 13e** |
| 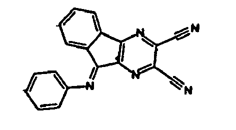 | **Example 14** |
| 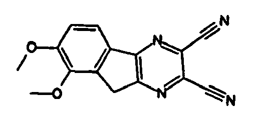 | **Example 16b** |
| 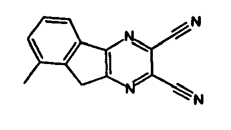 | **Example 16c** |
| 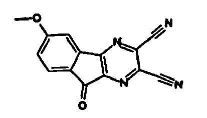 | **Example 17a** |
| 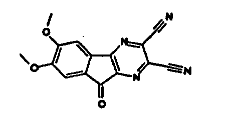 | **Example 17b** |
| 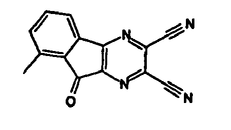 | **Example 17c** |
| 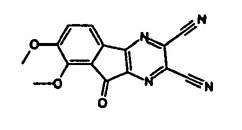 | **Example 17d** |
| 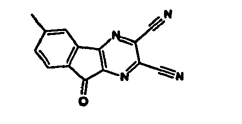 | **Example 17e** |
| 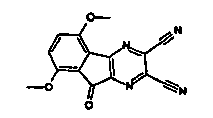 | **Example 17f** |
| 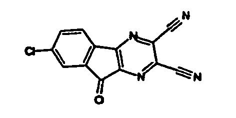 | **Example 17g** |
| 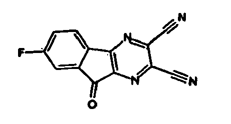 | **Example 17h** |
| 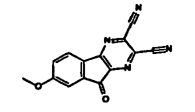 | **Example 17i** |
| 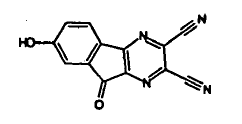 | **Example 17j** |
| 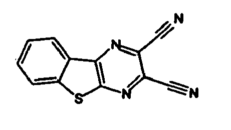 | **Example 19** |
| 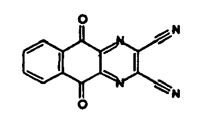 | **Example 20** |
| 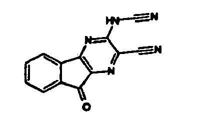 | **Example 21** |
| 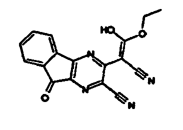 | **Example 22** |
| 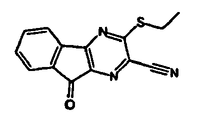 | **Example 23** |
| 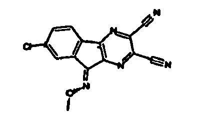 | **Example 24a** |
| 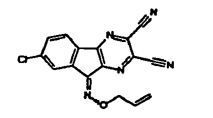 | **Example 24b** |
| 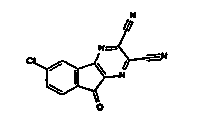 | **Example 25** |

### Experimental

Representative compounds of the invention can be synthesized according to the following procedures:

### Synthesis of 9-oxo-9H-indeno[1,2-b]pyrazine-2,3-dicarbonitrile (1):

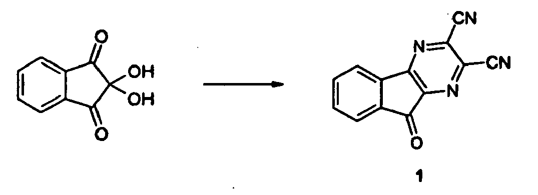

To a solution of ninhydrin (18,58 g, 104.3 mmol) in H₂O/EtOH/AcOH (130:195:9.1; 167 ml) a solution of diaminomaleodinitrile (11.27 g, 104.3 mmol) in H₂O/EtOH/AcOH (130:195:9.1; 167 ml) was added and the mixture was stirred at 60°C. After 3 hours, the precipitate was collected by filtration, washed with EtOH (100 ml) and dried under vacuum, affording **1** (23.64 g, 98%) as yellow-brown solid.
¹H NMR (300 MHz, CDCl₃): δ 8.07 (d, 1H), 7.98 (d, 1H), 7.87 (dd, 1H), 7.76 (dd, 1 H). ESI⁺MS: calcd for C₁₃H₄N₄O: 232.20; found: 233.0 (MH⁺).

### Synthesis of 9-hydroxy-3-methoxy-9H-indeno[1,2-b]pyrazine-2-carbonitrile (2):

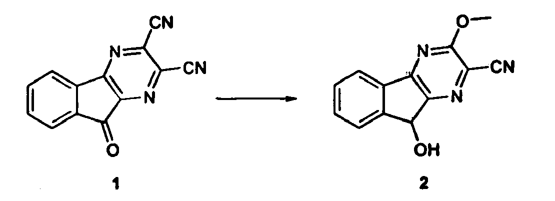

To a suspension of **1** (150 mg, 0.646 mmol) in MeOH (6.5 ml), cooled at 0°C, NaBH₄ (24 mg, 0.646 mmol) was added. After 30 min, water (5 ml) was added, MeOH was evaporated and the residue was extracted with CH₂Cl₂ (3x5 ml). The organic layers were dried over Na₂SO₄, filtered and evaporated. EtOH was added and the precipitate was collected by filtration affording **2** (82 mg, 53%) as white solid.
¹H NMR (300 MHz, DMSO d₆): δ 7.93 (d, 1 H), 7.76 (d, 1H), 7.65 (dd, 1 H), 7.58 (dd, 1H), 6.27 (d, 1H), 5.50 (d, 1H), 4.17 (s, 3H). ESI⁺MS: calcd for C₁₃H₉N₃O₂: 239.**24**; found: 240.1 (MH⁺).

### Synthesis of 3-methoxy-9-oxo-9H-indeno[1,2-b]pyrazine-2-carbonitrile (3):

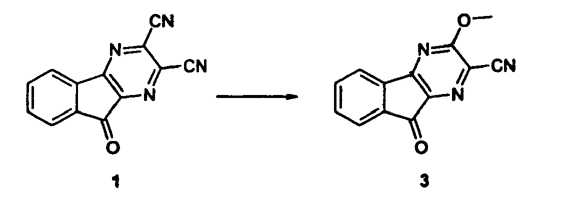

To a suspension of **1** (1.10 g, 4.7 mmol) in MeOH (47 ml) sodium (110 mg) was added and the mixture was stirred at room temperature for 16 hours. The precipitate was filtered, washed with EtOH and dried under vacuum, yielding **3** (1.03 g, 93%) as yellow-green solid.
¹H NMR (300 MHz, DMSO d₆): δ 7.92 (d, 1H), 7.83 (m, 2H), 7.71 (dd,1H), 4.25 (s, 3H). ESI⁺MS: calcd for C₁₃H₇N₃O₂: 237.22; found: 238.0 (MH⁺).

### Synthesis of 3-dimethylamino-9-oxo-9H-indeno[1,2-b]pyrazine-2-carbonitrile (4):

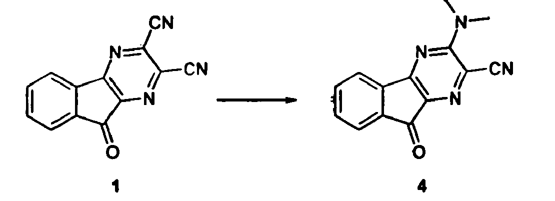

To a solution of **1** (53 mg, 0.228 mmol) in THF (2 ml) dimethylamine (2M in THF, 1.1 ml, 2.28 mmol) was added. The mixture was stirred at room temperature for 16 hours, then the solvent was evaporated affording **4** (56 mg, 98%) as yellow solid.
¹H NMR (300 MHz, DMSO d₆): δ 7.85 (d, 1 H), 7.80-7.73 (m, 2H), 7.67 (dd, 1 H), 3.47 (s, 6H). ESI⁺MS: calcd for C₁₄H₁₀N₄O: 250.26; found: 251.1 (MH⁺).

### Synthesis of 3-(2-methoxy-ethoxy)-9-oxo-9H-indeno[1,2-b]pyrazine-2-carbonitrile (5):

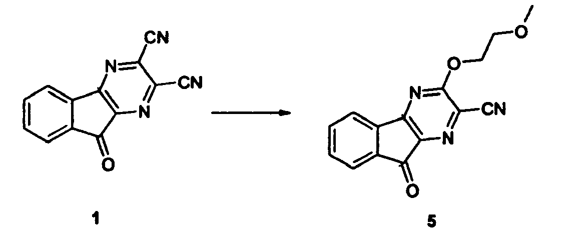

A suspension of **1** (59 mg, 0,254 mmol) in methoxyethanol (2,5 ml) was heated by MW (150°C, 30 min) in a sealed tube. The resulting suspension was filtered and the solid collected, washed with EtOH and dried under vacuum, yielding **5** (50 mg, 70%) as green solid.
¹H NMR (300 MHz, DMSO d₆): δ 7.90 (d, 1 H), 7.83 (dd, 1 H), 7.82 (d, 1 H), 7.71 (dd, 1 H), 4.79 (m, 2H), 3.80 (m, 2H), 3.36 (s, 3H). ESI⁺MS: calcd for C₁₅H₁₁N₃O₃: 281.27; found: 282.0 (MH⁺).

### Synthesis of 3-hydroxy-9-oxo-9H-indeno[1,2-b]pyrazine-2-carbonitrile (6):

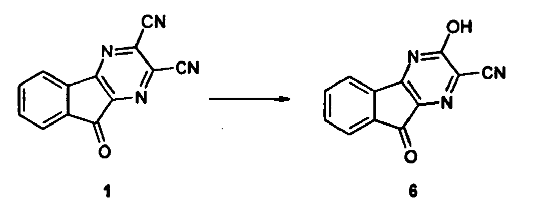

A suspension of **1** (5.66 g, 24.3 mmol) in aqueous NaOH (2% w/v, 81 ml) was stirred at room temperature for 16 hours. The mixture was acidified with 3N HCl to pH 1, the precipitate was collected by filtration, washed with water and dried under vacuum, affording **6** (4.88 g, 90%) as light brown solid.
¹H NMR (300 MHz, DMSO d₆): δ 7.89 (d, 1 H), 7.79-7.62 (m, 3H). ESI⁺MS: calcd for C₁₂H₅N₃O₂: 223.19; found: 224.0 (MH⁺).

### Synthesis of 3-amino-9-oxo-9H-indeno[1,2-b]pyrazine-2-carbonitrile (7):

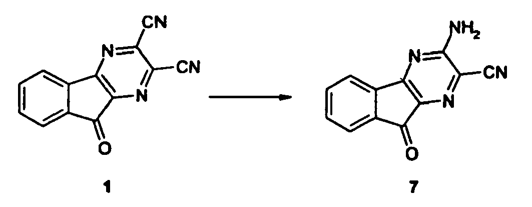

A mixture of **1** (201 mg, 0.86 mmol), ammonium acetate (331 mg, 4.3 mmol) and Na₂SO₄ (200 mg) in THF (2.9 ml) was stirred at 70°C in sealed tube for 18 hours. The solvent was evaporated, water (5 ml) was added and the precipitate filtered, washed with water and dried under vacuum, affording **7** (171 mg, 90%) as green solid.
¹H NMR (300 MHz, DMSO d₆): δ 8.45 (bs, 2H), 7.78-7.63 (m, 4H). ESI⁺MS: calcd for C₁₂H₆N₄O: 222.21; found: 223.1 (MH⁺).

### Synthesis of 3-(4,4-difluoro-piperidin-1-yl)-9-oxo-9H-indeno[1,2-b] pyrazine-2-carbonitrile (8):

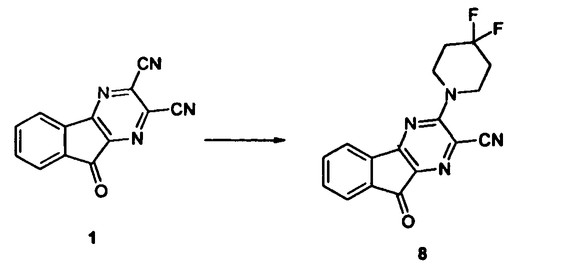

4,4-Difluoropiperidine hydrochloride (249 mg, 1.58 mmol) was dissolved in 1 N NaOH (5 ml) and extracted with CH₂Cl₂ (2x5 ml). Organic phase was dried over Na₂SO₄, filtered and evaporated. The residue was dissolved in THF (2 ml) and this solution was added to a solution of **1** (185 mg, 0.79 mmol) in THF (2 ml); the mixture was stirred at room temperature for 48 hours. The solvent was evaporated, the crude solid washed with EtOH and dried under vacuum, affording **8** (245 mg, 95%) as yellow-brown solid.
¹H NMR (300 MHz, DMSO d₆): δ 7.89 (d, 1 H), 7.79 (dd, 1 H), 7.78 (d, 1 H), 7.69 (dd, 1H), 4.13 (m, 4H), 2.22 (m, 4H). ESI⁺MS: calcd for C₁₇H₁₂F₂N₄O: 326.31; found: 327.1 (MH⁺).

### Synthesis of 3-chloro-9-oxo-9H-indeno[1,2-b]pyrazine-2-carbonitrile (9):

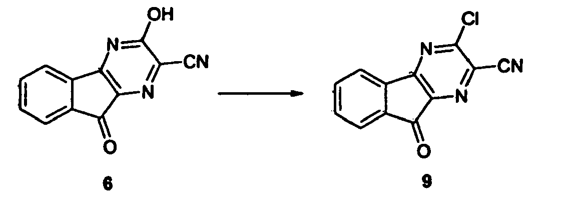

A suspension of **6** (671 mg, 3.0 mmol) in POCl₃ (8.4 ml) was heated under stirring to 100°C for 17h. Excess of POCl₃ was evaporated under reduced pressure and the crude was purified by flash chromatography on silica (CH₂Cl₂), affording **9** (320 mg, 44%) as yellow solid.
¹H NMR (300 MHz, DMSO d₆): δ 8.02 (d, 1H), 7.90 (m, 2H), 7.78(dd, 1H). ESI⁺MS: calcd for C₁₂H₄ClN₃O: 241.64; found: 241.9 (MH⁺).

### Synthesis of 9-(1',3'-dioxolan-2'-yl)-9H-indeno[1,2-b]pyrazine-2,3-dicarbonitrile (10):

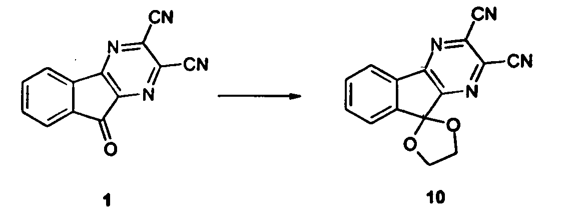

To a suspension of **1** (5.09 g, 21.9 mmol) in toluene (146 ml) ethylene glycol (2.4 ml, 43.8 mmol) and PTSA (6.25 g, 32.8 mmol) were added. The mixture was refluxed in a Dean-Stark apparatus for 28 hours, then the solvent was evaporated. The crude was purified by flash chromatography on silica (CH₂Cl₂), affording **10** (3.87 g, 64%) as light yellow solid.
¹H NMR (300 MHz, DMSO d₆): δ 8.03 (m, 1H), 7.83-7.70 (m, 3H), 4.47 (s, 4H). ESI⁺MS: calcd for C₁₅H₈N₄O₂: 276.26; found: 277.3 (MH⁺).

### Synthesis of 2-cyano-9-[hydroxyimino]-9H-indeno[1,2-b]pyrazine-3-carboxylic acid amide (11):

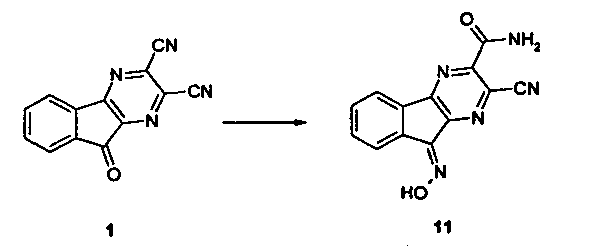

To a solution of **1** (500 mg, 2.1 mmol) in CH₃CN (20 ml) hydroxylamine (50% wt. in water, 0.25 ml, 4.2 mmol) was added at 0°C. The mixture was stirred at this temperature for 2.5 hours, then the formed precipitate was collected by filtration and dried under vacuum, affording **11** (355 mg, 62%) as red-brown solid.
¹H NMR (300 MHz, DMSO d₆): δ 10.96 (s, 1H), 8.11 (d, 1 H), 7.89 (dd, 1 H), 7.88 (d, 1H), 7.74 (dd, 1H), 6.32 (bs, 2H). ESI⁺MS: calcd for C₁₃H₇N₅O₂: 265.23; found: 265.9 (MH⁺).

### Synthesis of 9-[hydroxyimino]-9H-indeno[1,2-b]pyrazine-2,3-dicarbonitrile (12):

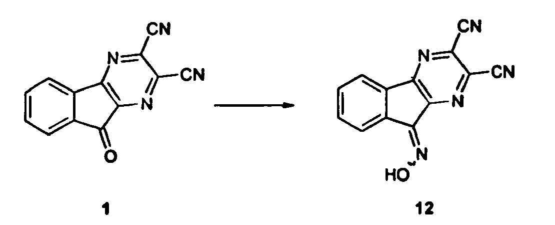

To a suspension of **1** (150 mg, 0.646 mmol) in pyridine (10 ml) hydroxylamine hydrochloride (134 mg, 1.94 mmol) was added at 0°C. Molecular sieves were added and the mixture was stirred at room temperature for 16 hours. The insoluble residue was filtered, the solvent evaporated and the crude purified by flash chromatography on silica (petroleum spirit/EtOAc 9:1), affording **12** (55 mg, 35%) as yellow solid in diastereoisomeric ratio 9:1.
¹H NMR for the main product (300 MHz, DMSO d₆): main product: δ 14.28 (bs, 1 H), 8.54 (d, 1 H), 8.22 (d, 1H), 7.84 (dd, 1 H), 7.78 (dd, 1 H). ESI⁺MS: calcd for C₁₃H₅N₅O: 247.22; found: 247.9 (MH⁺).

### General procedure A: synthesis of alkyloxyimines

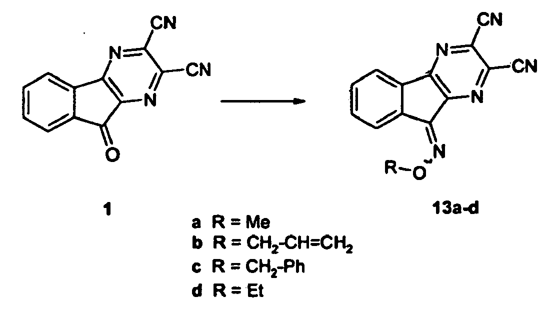

To a suspension of **1** (620 mg, 2.67 mmol) in pyridine (15 ml) a solution of O-alkyl-hydroxylamine hydrochloride (8.31 mmol) in pyridine (15 ml) was added dropwise at 0°C. Molecular sieves were added and the mixture was stirred at room temperature for 16 hours. The insoluble residue was filtered, the solvent evaporated and the crude purified by flash chromatography on silica petroleum spirit/EtOAc 9:1).

### Synthesis of 9-(methoxyimino)-9H-indeno[1,2-b]pyrazine-2,3-dicarbonitrile (13a):

Prepared according to the general procedure A in 35% yield as yellow solid in diastereoisomeric ratio 3:1. ¹H NMR (300 MHz, CDCl₃)(mixture of syn-anti diastereoisomers): main product: δ 8.09 (dd, 1 H), 7.94 (dd, 1 H), 7.79-7.68 (m, 2H); 4.34 (s, 3H). Minority product: δ 8.38 (m, 1 H), 8.18 (m, 1 H), 7.86-7.78 (m, 2H); 4.39 (s, 3H). ESI⁺MS: calcd for C₁₄H₇N₅O: 261.24; found: 262.1 (MH⁺).

### 9-(Allyloxyimino)-9H-indeno[1,2-b]pyrazine-2,3-dicarbonitrile (13b).

Prepared according to the general procedure A in 15% yield as yellow solid in diastereoisomeric ratio 1:1.¹H NMR (300 MHz, DMSO d₆) (mixture of syn-anti diastereoisomers): δ 8.43 (m, 1 H), 8.22 (m, 1 H), 7.90-7.80 (m, 2H), 6.20 (m, 1 H), 5.47 (m, 1 H), 5.35 (m, 1 H), 5.13 (ddd, 2H), and 8.12 (m, 1 H), 7.96 (m, 1 H), 7.80-7.69 (m, 2H), 6.14 (m, 1 H), 5.53 (m, 1 H), 5.38 (m, 1 H), 5.08 (ddd, 2H). ESI⁺MS: calcd for C₁₆H₉N₅O: 287.28; found: 288.2 (MH⁺).

### 9-Benzyloxyimino-9H-indeno[1,2-b]pyrazine-2,3-dicarbonitrile (13c).

Prepared according to the general procedure A in 32% yield as yellow solid in diastereoisomeric ratio 2:1.¹H NMR (300 MHz, DMSO d₆) (mixture of syn-anti diastereoisomers): δ 8.42 (m, 1 H), 8.21 (m, 1 H), 7.88-7.78 (m, 2H), 7.56-7.49 (m, 2H), 7.47-7.33 (m, 3H), 5.67 (s, 2H) and 8.11 (m, 1 H), 7.97 (m, 1 H), 7.79-7.69 (m, 2H), 7.56-7.49 (m, 2H), 7.47-7.33 (m, 3H), 5.63 (s, 2H). ESI⁺MS: calcd for C₂₀H₁₁N₅O: 337.34; found: 338.2 (MH⁺).

### 9-Ethoxyimino-9H-indeno[1,2-b]pyrazine-2,3-dicarbonitrile (13d).

Prepared according to the general procedure A in 28% yield as yellow solid in diastereoisomeric ratio 7:3. ¹H NMR (300 MHz, DMSO d₆) (mixture of syn-anti diastereoisomers): δ 8.44 (m, 1H), 8.22 (m, 1 H), 7.84 (m, 2H), 4.65 (q, 2H), 1.48(t, 3H) and 8.12 (m, 1H), 7.98 (m, 1H), 7.75 (m, 2H), 4.61 (q, 2H), 1.44 (t, 3H). ESI⁺MS: calcd for C₁₅H₉N₅O: 275.27; found: 276.2 (MH⁺).

### Synthesis of 9-[phenylimino]-9H-indeno[1,2-b]pyrazine-2,3-dicarbonitrile (14):

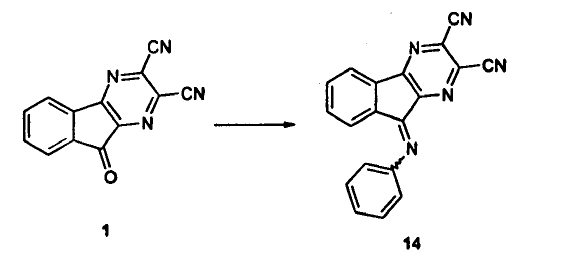

To a suspension of **1** (118 mg, 0.51 mmol) and molecular sieves in toluene (3 ml) aniline (0.037 ml, 0.41 mmol) was added. The mixture was heated by MW (150°C, 10 min), then the solvent was evaporated and the crude purified by flash chromatography on silica (petroleum spirit/EtOAc 9:1), affording **14** (93 mg, 60%) as red solid in diastereoisomeric ratio 7:3.
¹H NMR (300 MHz, CDCl₃): δ 8.14 (d, 1 H), 7.65 (dd, 1 H), 7.50 (dd, 2H), 7.44-7.29 (m, 3H), 7.05 (d, 2H). ESI⁺MS: calcd for C₁₉H₉N₅: 307.32; found: 308.0 (MH⁺).

### General procedure B: synthesis of 1,2-indandiones

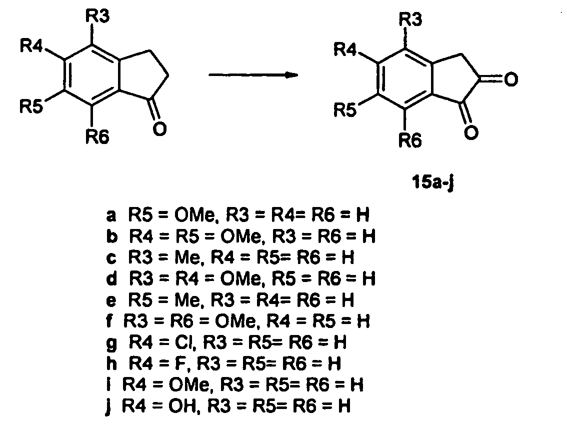

To a suspension of substituted 1-indanone (5 mmol) in MeOH (12 ml) warmed to 40°C isopentyl nitrite (0.73 ml, 5.5 mmol) and HCl 37% (0.5 ml) were added. After 1 hour at 40°C the formed precipitate was collected by filtration, washed with MeOH and dried under vacuum. The solid obtained was suspended in CH₂O (36% aqueous, 1.6 ml) and HCl 37% (3.2 ml) and the mixture was stirred at room temperature for 16 hours. Water (20 ml) was added and the suspension was extracted with CH₂Cl₂ (3x15 ml). Collected organic phases were dried over Na₂SO₄, filtered and evaporated. The crude product was used without further purification.

### 6-Methoxy-indan-1,2-dione (15a).

Prepared according to the general procedure B in 60% yield as yellow solid. ESI⁺MS: calcd for C₁₀H₈O₃: 176.17; found: 177.0 (MH⁺).

### 5,6-Dimethoxy-indan-1,2-dione (15b).

Prepared according to the general procedure B in 95% yield as light brown solid. ESI⁺MS: calcd for C₁₁H₁₀O₄: 206.20; found: 207.0 (MH⁺).

### 4-Methyl-indan-1,2-dione (15c).

Prepared according to the general procedure B in 60% yield as yellow solid. ESI⁺MS: calcd for C₁₀H₈O₂: 160.17; found: 161.0 (MH⁺).

### 4,5-Dimethoxy-indan-1,2-dione (15d).

Prepared according to the general procedure B in 94% yield as yellow solid. ESI⁺MS: calcd for C₁₁H₁₀O₄: 206.20; found: 207.0 (MH⁺).

### 6-Methyl-indan-1,2-dione (15e).

Prepared according to the general procedure B in 61% yield as yellow solid. ESI⁺MS: calcd for C₁₀H₈O₂: 160.17; found: 161.0 (MH⁺).

### 4,7-Dimethoxy-indan-1,2-dione (15f).

Prepared according to the general procedure B in 52% yield as light brown solid. ESI⁺MS: calcd for C₁₁H₁₀O₄: 206.20; found: 207.0 (MH⁺).

### 5-Chloro-indan-1,2-dione (15g).

Prepared according to the general procedure B in 57% yield as yellow solid. ESI⁺MS: calcd for C₉H₅ClO₂: 180.59; found: 181.0 (MH⁺).

### 5-Fluoro-indan-1,2-dione (15h).

Prepared according to the general procedure B in 63% yield as yellow solid. ESI⁺MS: calcd for C₉H₅FO₂: 164.14; found: 165.0 (MH⁺).

### 5-Methoxy-indan-1,2-dione (15i).

Prepared according to the general procedure B in 70% yield as yellow solid. ESI⁺MS: calcd for C₁₀H₈O₃: 176.17; found: 177.1 (MH⁺).

### 5-Hydroxy-indan-1,2-dione (15j).

Prepared according to the general procedure B in 64% yield as yellow solid. ESI⁺MS: calcd for C₉H₆O₃: 162.15; found: 163.0 (MH⁺).

### General procedure C: pyrazine ring formation

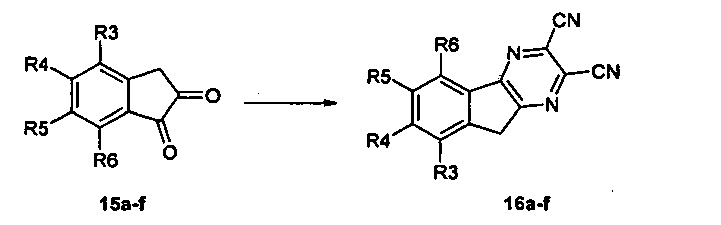

To a suspension of **15** (3 mmol) in iPrOH (15 ml) a suspension of diaminomaleodinitrile (324 mg, 3 mmol) in iPrOH (15 ml) was added. The mixture was stirred at room temperature for **24** hours, then the precipitate was collected by filtration, washed with EtOH and dried under vacuum.

### 6-Methoxy-9H-indeno[1,2-b]pyrazine-2,3-dicarbonitrile (16a).

Prepared according to the general procedure C in 65% yield as brown solid. ESI⁺MS: calcd for C₁₄H₈N₄O: 248.25; found: 249.0 (MH⁺).

### 6,7-Dimethoxy-9H-indeno[1,2-b]pyrazine-2,3-dicarbonitrile (16b).

Prepared according to the general procedure C in 91% yield as light brown solid. ESI⁺MS: calcd for **C₁₅H₁₀N₄O₂:** 278.27; found: 279.0 (MH⁺).

### 8-Methyl-9H-indeno[1,2-b]pyrazine-2,3-dicarbonitrile (16c).

Prepared according to the general procedure C in 60% yield as light brown solid. ¹H NMR (300 MHz, CDCl₃): δ 8.03 (d, 1H), 7.57-7.46 (m, 2H), 4.03 (s, 2H), 2.50 (s, 3H). ESI⁺MS: calcd for C₁₄H₈N₄: 232.25; found: 233.0 (MH⁺).

### 7,8-Dimethoxy-9H-indeno[1,2-b]pyrazine-2,3-dicarbonitrile (16d).

Prepared according to the general procedure C in 72% yield as yellow solid. ¹H NMR (300 MHz, CDCl₃): δ 7.90 (d, 1H), 7.17 (d, 1 H), 4.10 (s, 2H); 4.02 (s, 3H), 4.01 (s, 3H). ESI⁺MS: calcd for C₁₅H₁₀N₄O₂: 278.27; found: 279.2 (MH⁺).

### 6-Methyl-9H-indeno[1,2-b]pyrazine-2,3-dicarbonitrile (16e).

Prepared according to the general procedure C in 48% yield as light brown solid. ESI⁺MS: calcd for C₁₄H₈N₄: 232.25; found: 233.0 (MH⁺).

### 5,8-Dimethoxy-9H-indeno[1,2-b]pyrazine-2,3-dicarbonitrile (16f).

Prepared according to the general procedure C in 45% yield as light brown solid. ESI⁺MS: calcd for C₁₅H₁₀N₄O₂: 278.27; found: 278.9 (MH⁺).

### General procedure D: oxidation of methylenic group

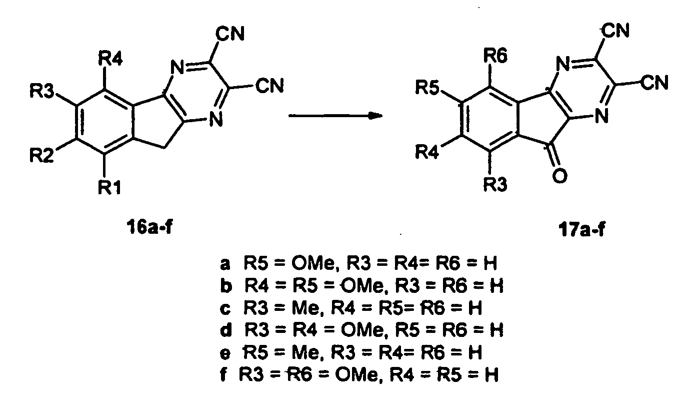

To a suspension of **16** (0.8 mmol) in AcOH (1.6 ml) a suspension of K₂Cr₂O₇ (434 mg, 1.44 mmol) in AcOH (0.8 ml) and water (0.2 ml) was added. The mixture was slowly heated to 100°C and it was vigorously stirred at this temperature for 1 hour. The hot suspension was poured in water (10 ml) and the precipitate collected by filtration, washed with water and dried under vacuum.

### 6-Methoxy-9-oxo-9H-indeno[1,2-b]pyrazine-2,3-dicarbonitrile (17a).

Prepared according to the general procedure D in 70% yield as light brown solid. ¹H NMR (300 MHz, CDCl₃) δ 7.92 (d, 1H), 7.48 (d, 1H), 7.18 (dd, 1H), 4.04 (s, 3H). ESI⁺MS: calcd for C₁₄H₆N₄O₂: 262.23; found: 263.0 (MH⁺).

### 6,7-Dimethoxy-9-oxo-9H-indeno[1,2-b]pyrazine-2,3-dicarbonitrile (17b).

Prepared according to the general procedure D in 37% yield as red solid. ¹H NMR (300 MHz, CDCl₃): δ 7.39 (s, 1 H), 7.37 (s, 1 H), 4.10 (s, 3H), 4.03 (s, 3H). ESI⁺MS: calcd for C₁₅H₈N₄O₃: 292.26; found: 293.0 (MH⁺).

### 8-Methyl-9-oxo-9H-indeno[1,2-b]pyrazine-2,3-dicarbonitrile (17c).

Prepared according to the general procedure D in 91% yield as yellow solid. ¹H NMR (300 MHz, DMSO d₆ 368K): δ 7.90 (d, 1 H), 7.79 (dd, 1 H), 7.61 (d, 1 H); 2.69 (s, 3H). ESI⁺MS: calcd for C₁₄H₆N₄O: 246.23; found: 247.0 (MH⁺).

### 7,8-Dimethoxy-9-oxo-9H-indeno[1,2-b]pyrazine-2,3-dicarbonitrile (17d).

Prepared according to the general procedure D in 71% yield as red solid. ¹H NMR (300 MHz, DMSO d₆ 368K): δ 7.74 (d, 1H), 7.49 (bd, 1H), 4.07 (s, 3H), 3.99 (s, 3H). ESI⁺MS: calcd for C₁₅H₈N₄O₃: 292.26; found: 293.0 (MH⁺).

### 6-Methyl-9-oxo-9H-indeno[1,2-b]pyrazine-2,3-dicarbonitrile (17e).

Prepared according to the general procedure D in 73% yield as yellow solid. ¹H NMR (300 MHz, DMSO d₆): δ 7.95 (d, 1H), 7.86 (d, 1H), 7.63 (dd, 1H), 2.52 (s, 3H). ESI⁺MS: calcd for C₁₄H₆N₄O: 246.23; found: 247.0 (MH⁺).

### 5,8-Dimethoxy-9-oxo-9H-indeno[1,2-b]pyrazine-2,3-dicarbonitrile (17f).

Prepared according to the general procedure D in 68% yield as brown solid. ¹H NMR (300 MHz, CDCl₃): δ 7.35 (d, 1 H), 7.24 (d, 1 H), 4.06 (s, 3H), 4.05 (s, 3H). ESI⁺MS: calcd for C₁₅H₈N₄O₃: 292.26; found: 293.0 (MH⁺).

### General procedure E: one-pot pyrazine ring formation and oxidation

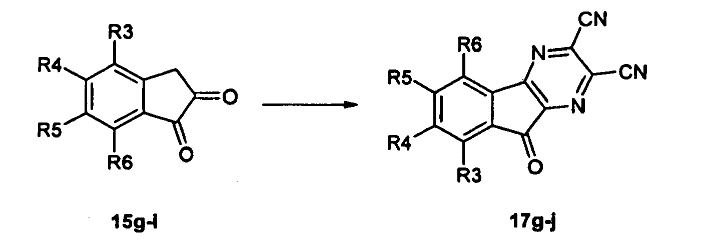

To a suspension of **15** (3 mmol) in iPrOH (15 ml) a suspension of diaminomaleodinitrile (324 mg, 3 mmol) in iPrOH (15 ml) was added. The mixture was stirred at room temperature for 24 hours then for 48 hours at 80°C. The precipitate was collected by filtration, washed with EtOH and dried under vacuum.

### 7-Chloro-9-oxo-9H-indeno[1,2-b]pyrazine-2,3-dicarbonitrile (17g).

Prepared according to the general procedure E in 40% as yellow solid. ¹H NMR (300 MHz, DMSO d₆): δ 8.13 (d, 1H), 8.04 (bs, 1 H), 7.97 (bd, 1 H). ESI⁺MS: calcd for C₁₃H₃ClN₄O: 266.65; found: 266.9 (MH⁺).

### 7-Fluoro-9-oxo-9H-indeno[1,2-b]pyrazine-2,3-dicarbonitrile (17h).

Prepared according to the general procedure E in 55% as pink solid. ¹H NMR (300 MHz, CDCl₃): δ 8.03 (dd, 1H), 7.74 (dd, 1H), 7.42 (ddd, 1H). ESI⁺MS: calcd for C₁₃H₃FN₄O: 250.19; found: 251.0 (MH⁺).

### 7-Methoxy-9-oxo-9H-indeno[1,2-b]pyrazine-2,3-dicarbonitrile (17i).

Prepared according to the general procedure E in 23% as light brown solid. ¹H NMR (300 MHz, CDCl₃): δ 7.92 (d, 1 H), 7.48 (d, 1 H), 7.18 (dd, 1 H), 4.03 (s, 3H). ESI⁺MS: calcd for C₁₄H₆N₄O₂: 262.23; found: 263.0 (MH⁺).

### 7-Hydroxy-9-oxo-9H-indeno[1,2-b]pyrazine-2,3-dicarbonitrile (17j).

Prepared according to the general procedure E in 35% as orange solid. The product was not purified by precipitation but, after evaporation of the solvent, by flash chromatography (CH₂Cl₂/MeOH 9:1). ¹H NMR (300 MHz, DMSO d₆): δ 11.66 (bs, 1 H), 7.84 (d, 1H), 7.30 (d, 1 H), 7.09 (dd, 1 H). ESI⁺MS: calcd for C₁₃H₄N₄O₂: 248.20; found: 249.0 (MH⁺).

### Synthesis of benzo[b]thiophene-2,3-dione (18):

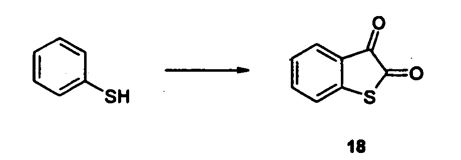

To a solution of benzenthiol (1 ml, 9.7 mmol) in Et₂O (30 ml) at 0°C oxalyl chloride (0.94 ml, 10.7 mmol) was added dropwise. The mixture was stirred at room temperature for 1.5 hour, then the solvent was evaporated under reduced pressure. The crude was dissolved in CH₂Cl₂ (40 ml) and a solution of AlCl₃ (4.75 g, 35 mmol) in CH₂Cl₂ (32 ml) was added dropwise at 0°C. The mixture was stirred for 16 hours at room temperature, then ice and 1 M HCl were added until a clear mixture was obtained. After 1 hour, the phases were separated and the aqueous layer was extracted with CH₂Cl₂ (3x30 ml). The collected organic phases were dried over Na₂SO₄, filtered and evaporated, affording **18** (1.2 g, 78%) as orange solid that was used without further purification.
ESI⁺MS: calcd for C₈H₄O₂S: 164.18; found: 165.1 (MH⁺).

### Synthesis of benzo[4,5]thieno[2,3-b]pyrazine-2,3-dicarbonitrile (19):

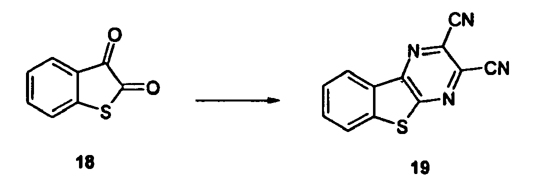

**18** (300 mg, 1.83 mmol) and diaminomaleodinitrile (198 mg, 1.83 mmol) were added to boiling water (10 ml). The mixture was refluxed for 1 h, then the crude precipitate was filtered, suspended in MeOH and refluxed for 10 min. After cooling at room temperature, the solid was filtered and dried under vacuum, yielding **19** (216 mg, 50%) as brown powder.
¹H NMR (300 MHz, DMSO d₆): δ 8.58 (d, 1 H), 8.38 (d, 1 H), 7.94 (dd, 1 H), 7.80 (dd, 1 H). ESI⁺MS: calcd for C₁₂H₄N₄S: 236.26; found: 237.1 (MH⁺).

### Synthesis of 5,10-dioxo-5,10-dihydro-benzo[g]quinoxaline-2,3-dicarbonitrile (20):

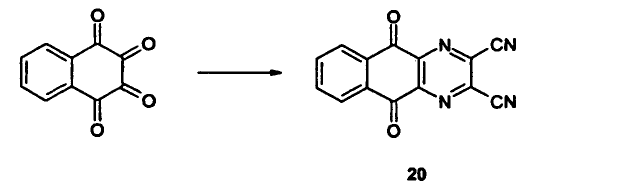

A suspension of 1,2,3,4-tetraoxo-1,2,3,4-tetrahydro-naphtaline dihydrate (214 mg, 0.95 mmol) and diamminomaleodinitrile (102 mg, 0.95 mmol) in EtOH (9.5 ml) and a catalytic amount of AcOH was stirred at room temperature for 24 hours. The precipitate was collected by filtration, washed with EtOH and dried under vacuum, obtaining **20** (65 mg, 35%) as light brown solid.
¹H NMR (300 MHz, DMSO d₆): δ 9.16 (m, 2H), 8.24 (m, 2H).

### Synthesis of 2-cyano-9-oxo-9H-indeno[1,2-b]pyrazin-3-yl-cyanamide (21):

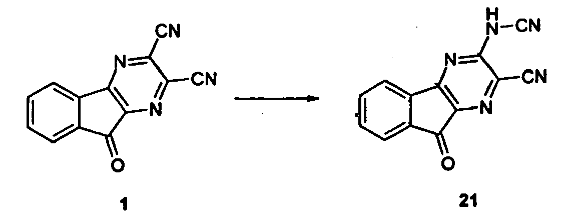

Under inert atmosphere, cyanamid (44 mg, 1.037 mmol) was dissolved in dry DMF (1 ml) and NaH (21 mg, 0.519 mmol) was added in one portion. After 20 min, a solution of **1** (96 mg, 0.415 mmol) in dry DMF (2 ml) was added dropwise. After 1 h the solvent was evaporated and the crude purified by flash chromatography (CH₂Cl₂/MeOH 8:2) affording **21** (84 mg, 82%) as orange solid.
¹H NMR (300 MHz, DMSO d₆): δ 7.85 (ddd, 1 H), 7.77 (ddd, 1 H), 7.76 (m, 1H), 7.67 (ddd, 1H). ESI⁺MS: calcd for C₁₃H₅N₅O: 247.22; found: 248.1 (MH⁺).

### Synthesis of 3-(1-cyano-2-ethoxy-2-hydroxy-vinyl)-9-oxo-9H-indeno[1,2-b]pyrazine-2-carbonitrile (22):

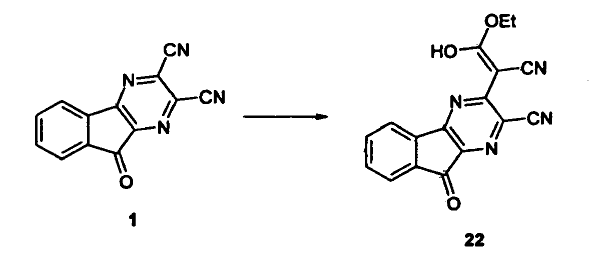

Ethylcyanoacetate (110 mg, 0.970 mmol) was dissolved, under inert atmosphere, in dry DMF (1 ml) and NaH (39 mg, 0.970 mmol) was added in one portion. After 30 min, a solution of **1** (150 mg, 0.646 mmol) in dry DMF (2 ml) was added dropwise. After 15 min MeOH was added and the solution stirred for 10 min. The solvents were evaporated and the crude purified by flash chromatography (EtOAc:MeOH 9:1) affording **22** as a dark red solid (200 mg, 97%).
¹H NMR (300 MHz, DMSO d₆): δ 7.78-7.55 (m, 4H), 4.11 (q, 2H), 1.22 (t, 3H). ESI⁺MS: calcd for C₁₇H₁₀N₄O₃: 318.29; found: 319.2 (MH⁺).

### Synthesis of 3-ethylsulfanyl-9-oxo-9H-indeno[1,2-b]pyrazine-2-carbonitrile (23):

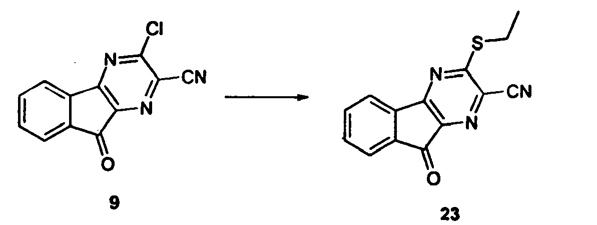

To a mixture of ethanethiol (62 µl, 0.84 mmol) and 1 N NaOH (0.5 ml, 0.5 mmol) in THF (2.1 ml) **9** (101 mg, 0.42 mmol) was added. The mixture was stirred at room temperature for 30 min then the solvent was evaporated under reduced pressure. The residue was dissolved in H₂O (4 ml) and extracted with CH₂Cl₂ (2x4 ml). The collected organic phases were dried over Na₂SO₄, filtered and evaporated. The crude was purified by flash chromatography (CH₂Cl₂) affording **23** (98 mg, 87%) as orange solid.
¹H NMR (300 MHz, CDCl₃): δ 7.92 (d, 1H), 7.86 (d, 1 H), 7.73 (ddd, 1 H), 7.63 (ddd, 1 H), 3.44 (q, 2H), 1.51 (t, 3H). ESI⁺MS: calcd for C₁₄H₉N₃OS: 267.31; found: 268.1 (MH⁺).

### General procedure F: synthesis of alkyloxyimines

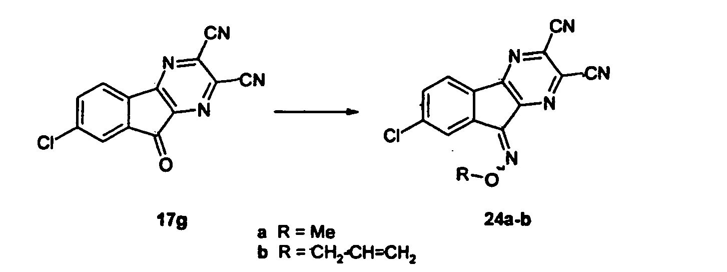

To a suspension of **17g** (151 mg, 0.56 mmol) in pyridine (5.6 ml) O-alkyl-hydroxylamine hydrochloride (1.68 mmol) and molecular sieves were added and the mixture was stirred at 60°C for 1.5h. The insoluble residue was filtered, the solvent evaporated and the crude purified by flash chromatography on silica (petroleum spirit/CH₂Cl₂ 1:1).

### 7-Chloro-9-methoxyimino-9H-indenof[1,2-b]pyrazine-2,3-dicarbonitrile (24a)

Prepared according to the general procedure F in 65% yield as light brown solid in diastereoisomeric ratio 1:1. ¹H NMR (300 MHz, CDCl₃) (mixture of syn-anti diastereoisomers): δ 8.36 (d, 1H), 8.04 (d, 1H), 7.64 (dd, 1 H), 4.43 (s, 3H) and 7.94 (d, 1 H), 7.89 (d, 1 H), 7.54 (dd, 1 H), 4.36(s, 3H). ESI⁺MS: calcd for C₁₄H₆ClN₅O: 295.69; found: 296.0 (MH⁺).

### 9-Allyloxyimino-7-chloro-9H-indeno[1,2-b]pyrazine-2,3-dicarbonitrile (24b)

Prepared according to the general procedure F in 56% yield as light brown solid in diastereoisomeric ratio 1:1. ¹H NMR (300 MHz, CDCl₃) (mixture of syn-anti diastereoisomers): δ 8.41 (d, 1 H), 8.07 (d, 1 H), 7.67 (dd, 1 H), 6.22-6.03 (m, 1 H), 5.47 (m, 1 H), 5.36 (m, 1 H), 5.15 (m, 2H) and 7.97 (d, 1 H), 7.94 (d, 1 H), 7.57 (dd, 1 H), 6.22-6.03 (m, 1 H), 5.47 (m, 1H), 5.40 (m, 1 H), 5.05 (m, 2H). ESI⁺MS: calcd for C₁₆H₈ClN₅O: 321.73; found: 322.1 (MH⁺).

### 6-Chloro-9-oxo-9H-indeno[1,2-b]pyrazine-2,3-dicarbonitrile (25):

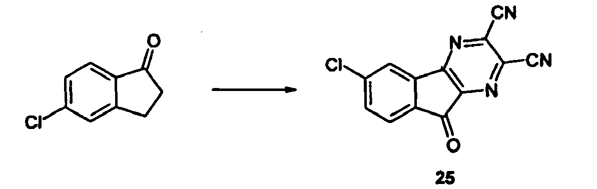

A mixture of 5-chloro-1-indanone (1.05 g, 6.28 mmol) and N-bromosuccinimide (2.23 g, 12.56 mmol) in DMSO (25 ml) was stirred overnight at 40°C and 5h at 80°C under vacuum. Water (125 ml) was added and the mixture was extracted with CH₂Cl₂ (25 ml). The aqueous phase was saturated with brine and solid NaCl and extracted with CH₂Cl₂ (4x80 ml). The collected organic phases were dried over Na₂SO₄ and the solvent evaporated. The crude was dissolved in EtOH (63 ml), diaminomaleonitrile (678 mg, 6.28 mmol) and a catalytic amount of AcOH were added and the mixture stirred at 80°C for 45min. The precipitate was collected by filtration and washed with EtOH (464 mg). The filtered solution was evaporated and the crude purified by flash

### Representative cysteine proteases

### USP5 activity assay

USP5 was diluted in USP buffer (50 mM Tris HCl; 0.5 mM EDTA; 5 mM DTT; 0.01% Triton X-100; Bovine Serum Albumin 0.05 mg.ml⁻¹ pH7.6). Compounds stocks (100 mM) were stored at -20°C in DMSO. Compounds were tested at the following final concentrations: 100 µM; 33.3 µM; 11.1 µM; 3.7 µM; 1.23 µM; 412 nM; 137 nM; 45.7 nM; 15.2 nM; 5 nM.

Reactions were performed as duplicates in Black LJL 96 well plates (HE microplates; Molecular Devices; 20 µl final reaction volume).The substrate concentration for USP5 was 400 nM Ub-AMC (Boston Biochem). The concentrations of the enzyme (USP5) in specificity assays was 300 pM. The concentrations were determined in order to perform specificity assays under initial velocities at fixed substrate concentration. Compounds were pre-incubated with enzymes for 30 minutes at 25°C. Reactions were initiated by addition of substrate to the plates containing the enzymes (+/- compounds) diluted in assay buffer. Reactions were incubated for 60 minutes at 37°C. Reactions were stopped by adding acetic acid (100 mM final). Readings were performed on a Pherastar Fluorescent Reader (BMG). λ Emission 380 nm; λ Excitation = 460 nm. Data (mean values +/- standard deviation) were analyzed as % of control (no compound) and plotted as percentage versus the Log of the compound concentration using GraphPad (Prism). Data were fitted to a sigmoidal model (variable slope).

### Cloning & purification of USP7

The cDNA encoding USP7 was obtained by PCR amplification from placenta mRNA. USP7 cDNA was subcloned by PCR into a baculovirus expression vector (pFastBac-HT; Invitrogen). A cDNA encoding a mutated USP7 was generated by mutagenic PCR. The corresponding protein encodes a cysteine to alanine substitution at residue 223. The sequences were ascertained by sequencing of the entire open reading frame. Bacmids encoding USP7 were generated following DH10bac transposition. The corresponding bacmids were transfected into insect cells (Sf9). Viruses were recovered from culture supernatant and amplified twice. Insect cells (Sf9 or High Five; Invitrogen) were infected for 72 hours. Total cell lysates were harvested and lyzed in lysis buffer (Tris HCl 50 mM pH7.6; 0.75 % NP40; 500 mM NaCl; 10 % glycerol; 1 mM DTT; 10 mM imidazole; Protease Inhibitor Cocktail; AEBSF 20 µg.ml⁻¹; Aprotinin 10 µg.ml⁻¹). Proteins were affinity purified on metal affinity resins (Talon Metal affinity resin; BD Biosciences). Bound materials were extensively washed in wash buffer (50mM Sodium Phosphate pH7.0; 300 mM NaCl; 10 mM imidazole; 0.5% Triton X-100; 10% glycerol) and eluted from the resin in 250 mM imidazole-containing wash buffer. Proteins were dialyzed in dialysis buffer (Tris HCl pH 7.6 20 mM; NaCl 200 mM; DTT 1 mM; EDTA 1 mM; 10% Glycerol). Proteins purifications were analyzed on 4-12% NuPAGE (Invitrogen).

### USP7 activity assay

USP7 was diluted in USP buffer (50 mM Tris HCl; 0.5 mM EDTA; 5 mM DTT; 0.01 % Triton X-100; Bovine Serum Albumin 0.05 mg.ml⁻¹ pH7.6). Compounds stocks (100 mM) were stored at -20°C in DMSO. Compounds were tested at the following final concentrations: 100 µM; 33.3 µM; 11.1 µM; 3.7 µM; 1.23 µM; 412 nM; 137 nM; 45.7 nM; 15.2 nM; 5 nM.

Reactions were performed as duplicates in Black LJL 96 well plates (HE microplates; Molecular Devices; 20 µl final reaction volume).The substrate concentration for USP7 was 400 nM Ub-AMC (Chem. Biol., 2003, 10, p. 837-846) (Boston Biochem). The concentrations of the enzyme (USP7) in specificity assays was 152 pM. The concentrations were determined in order to perform specificity assays under initial velocities at fixed substrate concentration. Compounds were pre-incubated with enzymes for 30 minutes at 25°C. Reactions were initiated by addition of substrate to the plates containing the enzymes (+/- compounds) diluted in assay buffer. Reactions were incubated for 60 minutes at 37°C. Reactions were stopped by adding acetic acid (100 mM final). Readings were performed on a Pherastar Fluorescent Reader (BMG). λ Emission 380 nm; λ Excitation = 460 nm. Data (mean values +/- standard deviation) were analyzed as % of control (no compound) and plotted as percentage versus the Log of the compound concentration using GraphPad (Prism). Data were fitted to a sigmoidal model (variable slope).

### Cloning & purification of USP8

The cDNA encoding USP8 was obtained by PCR amplification from placenta mRNA. USP8 cDNA was subcloned by PCR into a baculovirus expression vector (pFastBac-HT; Invitrogen). A cDNA encoding a mutated USP8 was generated by mutagenic PCR. The corresponding protein encodes a cysteine to alanine substitution at residue 786. The sequences were ascertained by sequencing of the entire open reading frame. Bacmids encoding USP7 were generated following DH10bac transposition. The corresponding bacmids were transfected into insect cells (Sf9). Viruses were recovered from culture supernatant and amplified twice. Insect cells (Sf9 or High Five; Invitrogen) were infected for 72 hours. Total cell lysates were harvested and lyzed in lysis buffer (Tris HCl 50 mM pH7.6; 0.75 % NP40; 500 mM NaCl; 10 % glycerol; 1 mM DTT; 10 mM imidazole; Protease Inhibitor Cocktail; AEBSF 20 µg.ml⁻¹; Aprotinin 10 µg.ml⁻¹). Proteins were affinity purified on metal affinity resins (Talon Metal affinity resin; BD Biosciences). Bound materials were extensively washed in wash buffer (50 mM Sodium Phosphate pH 7.0; 300 mM NaCl; 10 mM imidazole; 0.5% Triton X-100; 10% glycerol) and eluted from the resin in 250 mM imidazole-containing wash buffer. Proteins were dialyzed in dialysis buffer (Tris HCl pH 7.6 20 mM; NaCl 200 mM; DTT 1 mM; EDTA 1 mM; 10% Glycerol). Proteins purifications were analyzed on 4-12% NuPAGE (Invitrogen).

### USP8 activity assay

USP8 was diluted in USP buffer (50 mM Tris HCl; 0.5 mM EDTA; 5 mM DTT; 0.01% Triton X-100; Bovine Serum Albumin 0.05 mg.ml⁻¹ pH8.8). Compounds stocks (100 mM) were stored at -20°C in DMSO. Compounds were tested at the following final concentrations: 100 µM; 33.3 µM; 11.1 µM; 3.7 µM; 1.23 µM; 412 nM; 137 nM; 45.7 nM; 15.2 nM; 5 nM.

Reactions were performed as duplicates in Black LJL 96 well plates (HE microplates; Molecular Devices; 20 µl final reaction volume).The substrate concentration for USP8 was 400 nM Ub-AMC (Boston Biochem). The concentrations of the enzyme (USP8) in specificity assays was 630 pM. The concentrations were determined in order to perform specificity assays under initial velocities at fixed substrate concentration. Compounds were pre-incubated with enzymes for 30 minutes at 25°C. Reactions were initiated by addition of substrate to the plates containing the enzymes (+/- compounds) diluted in assay buffer. Reactions were incubated for 60 minutes at 37°C. Reactions were stopped by adding acetic acid (100 mM final). Readings were performed on a Pherastar Fluorescent Reader (BMG). λ Emission 380 nm; λ Excitation = 460 nm. Data (mean values +/- standard deviation) were analyzed as % of control (no compound) and plotted as percentage versus the Log of the compound concentration using GraphPad (Prism). Data were fitted to a sigmoidal model (variable slope).

### UCH-L3 activity assay

Uch-L3 was diluted in USP buffer (50 mM Tris HCl; 0.5 mM EDTA; 5 mM DTT; 0.01% Triton X-100; Bovine Serum Albumin 0.05 mg.ml⁻¹ pH7.6). Compounds stocks (100 mM) were stored at -20°C in DMSO. Compounds were tested at the following final concentrations: 100 µM; 33.3 µM; 11.1 µM; 3.7 µM; 1.23 µM; 412 nM; 137 nM; 45.7 nM; 15.2 nM; 5 nM.

Reactions were performed as duplicates in Black LJL 96 well plates (HE microplates; Molecular Devices; 20 µl final reaction volume).The substrate concentration for Uch-L3 was 400 nM Ub-AMC (Boston Biochem). The concentration of the enzyme (Uch-L3) in specificity assays was 13 pM. The concentrations were determined in order to perform specificity assays under initial velocities at fixed substrate concentration. Compounds were pre-incubated with enzymes for 30 minutes at 25°C. Reactions were initiated by addition of substrate to the plates containing the enzymes (+/- compounds) diluted in assay buffer. Reactions were incubated for 60 minutes at 37°C. Reactions were stopped by adding acetic acid (100 mM final). Readings were performed on a Pherastar Fluorescent Reader (BMG). δ Emission 380 nm; δ Excitation = 460 nm. Data (mean values +/- standard deviation) were analyzed as % of control (no compound) and plotted as percentage versus the Log of the compound concentration using GraphPad (Prism). Data were fitted to a sigmoidal model (variable slope).

### Caspase 3 activity assay

Caspase 3 was diluted in Caspase 3 buffer (100 mM Hepes pH 7.5; 10% sucrose; 0.1% CHAPS). Compounds stocks (100 mM) were stored at -20°C in DMSO. Compounds were tested at the following final concentrations: 100 µM; 33.3 µM; 11.1 µM; 3.7 µM; 1.23 µM; 412 nM; 137 nM; 45.7 nM; 15.2 nM; 5 nM. Reactions were performed as duplicates in Black LJL 96 well plates (HE microplates; Molecular Devices; 20 µl final reaction volume). The substrate concentration for caspase 3 specificity assay was 500 nM (Ac-DEVD-AMC; Promega). The concentration of the enzyme (Caspase 3) in specificity assays was 3.2 nM. The concentrations were determined in order to perform specificity assays under initial velocities at fixed substrate concentration. Compounds were pre-incubated with enzymes for 30 minutes at 25°C. Reactions were initiated by addition of substrate to the plates containing the enzymes (+/compounds) diluted in assay buffer. Reactions were incubated for 60 minutes at 37°C. Reactions were stopped by adding acetic acid (100 mM final). Readings were performed on a Pherastar Fluorescent Reader (BMG). δ Emission 380 nm; δ Excitation = 460 nm. Data (mean values +/- standard deviation) were analyzed as % of control (no compound) and plotted as percentage versus the Log of the compound concentration using GraphPad (Prism). Data were fitted to a sigmoidal model (variable slope).

### Cathepsin B activity assay

Cathepsin B was diluted in Cathepsin B buffer (20 mM Tris HCl pH 6.8; 1 mM EDTA; 1 mM DTT). Compounds stocks (100 mM) were stored at -20°C in DMSO. Compounds were tested at the following final concentrations: 100 µM; 33.3 µM; 11.1 µM; 3.7 µM; 1.23 µM; 412 nM; 137 nM; 45.7 nM; 15.2 nM; 5 nM. Reactions were performed as duplicates in Black LJL 96 well plates (HE microplates; Molecular Devices; 20 µl final reaction volume). The substrate concentration for cathepsin B specificity assay was 36 µM (z-RR-AMC; Calbiochem).The concentration of the enzyme (Cathepsin B) in specificity assays was 3.6 nM. The concentrations were determined in order to perform specificity assays under initial velocities at fixed substrate concentration. Compounds were pre-incubated with enzymes for 30 minutes at 25°C. Reactions were initiated by addition of substrate to the plates containing the enzymes (+/- compounds) diluted in assay buffer. Reactions were incubated for 60 minutes at 37°C. Reactions were stopped by adding acetic acid (100 mM final). Readings were performed on a Pherastar Fluorescent Reader (BMG). δ Emission 380 nm; δ Excitation = 460 nm. Data (mean values +/- standard deviation) were analyzed as % of control (no compound) and plotted as percentage versus the Log of the compound concentration using GraphPad (Prism). Data were fitted to a sigmoidal model (variable slope).

### Cell viability and proliferation methods

### HCT116 cell viability and proliferation assay

HCT116 colon cancer cells were obtained from ATCC (American Type Culture Collection), and maintained in Mc Coy's 5A medium containing 10% FBS, 3 mM glutamine and 1% penicillin/streptomycin. Cells were incubated at 37°C in a humidified atmosphere containing 5% CO₂.

Cell viability was assayed using the MTS technique in 96-well culture plates (CellTiter 96^{®} Aqueous Non-Radioactive Cell Proliferation Assay, Promega) according to the manufacturer's instructions. MTS (3-(4,5-dimethylthiazol-2-yl)-5-(3-carboxymethoxyphenyl)-2-(4-sulfophenyl)-2H-tetrazolium) is a MTT-derived tetrazolium that is reduced in metabolically active cells into a soluble, cell-permeant formazan. The amount of formazan, detected by its absorbance at 492 nm is proportional to the number of living, metabolically active cells.

10³ HCT116 cells were seeded per well. 24 hours later, the medium was changed and the cells treated in triplicate with the following concentrations of each compound: 10µM - 3.33µM - 1.11µM - 370nM - 123nM - 41nM - 14 nM and 5 nM. The compounds were diluted in 100% DMSO, whose final concentration on cells was kept at 0.5%.

Cells were incubated with the compounds for 72 hours, and their viability then assayed by the addition of MTS for 2 hours. Absorbance at 492 nm was measured directly from the 96-well culture plates. GI50 (Growth Inhibition 50) concentrations for each compound were calculated using a sigmoidal variable slope fit (Prism 4.0, Graphpad Softwares). Values represent mean of 3 independent experiments.

### PC3 cell viability and proliferation assay

PC-3 prostate cancer cells were obtained from ATCC, and maintained in F-12K medium containing 7% FBS and 1% penicillin/streptomycin. Cells were incubated at 37°C in a humidified atmosphere containing 5% CO₂.

Cell viability was assayed using the MTS technique in 96-well culture plates (CellTiter 96^{®} Aqueous Non-Radioactive Cell Proliferation Assay, Promega) according to the manufacturer's instructions. MTS (3-(4,5-dimethylthiazol-2-yl)-5-(3-carboxymethoxyphenyl) -2-(4-sulfophenyl)-2H-tetrazolium) is a MTT-derived tetrazolium that is reduced in metabolically active cells into a soluble, cell-permeant formazan. The amount of formazan, detected by its absorbance at 492 nm is proportional to the number of living, metabolically active cells.

2 x 10³ PC3 cells were seeded per well. 24 hours later, the medium was changed and the cells treated in triplicate with the following concentrations of each compound: 10µM - 3.33µM - 1.11 µM - 370nM - 123nM - 41 nM - 14 nM and 5 nM. The compounds were diluted in 100% DMSO, whose final concentration on cells was kept at 0.5%.

Cells were incubated with the compounds for 72 hours, and their viability then assayed by the addition of MTS for 2 hours. Absorbance at 492 nm was measured directly from the 96-well culture plates. GI50 (Growth Inhibition 50) concentrations for each compound were calculated using a sigmoidal variable slope fit (Prism 4.0, Graphpad Softwares). Values represent mean of 3 independent experiments.

### Results

### 1. Inhibition of cysteine protease activities

### *USPs

| **Experimental N°** | **USP 5** | | **Experimental N°** | **USP 5** |
|---|---|---|---|---|
| Example 1 | 1.3 µM | | Example 16b | > 100 µM |
| Example 2 | AF | | Example 16c | > 100 µM |
| Example 3 | > 100 µM | | Example 17a | 29 µM |
| Example 4 | > 100 µM | | Example 17b | > 100 µM |
| Example 5 | > 100 µM | | Example 17c | 60 µM |
| Example 6 | 9.1 µM | | Example 17d | 2.5 µM |
| Example 7 | > 100 µM | | Example 17e | 7.8 µM |
| Example 8 | > 100 µM | | Example 17f | 3.3 µM |
| Example 9 | > 100 µM | | Example 17g | 0.16 µM |
| Example 10 | > 100 µM | | Example 17h | 1.0 µM |
| Example 11 | 2.1 µM | | Example 17i | 23 µM |
| Example 12 | 45 µM | | Example 17j | > 100 µM |
| Example 13a | 19.5 µM | | Example 19 | > 100 µM |
| Example 13b | > 100 µM | | Example 20 | > 100 µM |
| Example 13C | > 100 µM | | Example 21 | > 100 µM |
| Example 13d | > 100 µM | | Example 22 | > 100 µM |
| Example 13e | 32 µM | | Example 23 | > 100 µM |
| Example 14 | 1.9 µM | | | |
| **Experimental N°** | **USP 7** | | **Experimental N°** | **USP 7** |
| Example 1 | 3.5 µM | | Example 16b | > 100 µM |
| Example 2 | AF | | Example 16c | > 100 µM |
| Example 3 | > 100 µM | | Example 17a | > 100 µM |
| Example 4 | > 100 µM | | Example 17b | 10.2 µM |
| Example 5 | > 100 µM | | Example 17c | > 100 µM |
| Example 6 | 21.8 µM | | Example 17d | 18 µM |
| Example 7 | > 100 µM | | Example 17e | 7.2 µM |
| Example 8 | > 100 µM | | Example 17f | 12.7 µM |
| Example 9 | > 100 µM | | Example 17g | 0.53 µM |
| Example 10 | > 100 µM | | Example 17h | 4.3 µM |
| Example 11 | 13 µM | | Example 17i | >100 µM |
| Example 12 | > 100 µM | | Example 17j | 66 µM |
| Example 13a | > 100 µM | | Example 19 | > 100 µM |
| Example 13b | > 100 µM | | Example 20 | > 100 µM |
| Example 13C | > 100 µM | | Example 21 | > 100 µM |
| Example 13d | > 100 µM | | Example 22 | > 100 µM |
| Example 13e | > 100 µM | | Example 23 | > 100 µM |
| Example 14 | 4.1 µM | | | |
| | | | | |
| **Experimental N°** | **USP 8** | | **Experimental N°** | **USP 8** |
| Example 1 | 0.29 µM | | Example 16b | 72 µM |
| Example 2 | AF | | Example 16c | > 100 µM |
| Example 3 | 31 µM | | Example 17a | 4.0 µM |
| Example 4 | > 100 µM | | Example 17b | 2.5 µM |
| Example 5 | 53 µM | | Example 17c | 3.1 µM |
| Example 6 | 8.4 µM | | Example 17d | 0.71 µM |
| Example 7 | 48 µM | | Example 17e | 0.93 µM |
| Example 8 | > 100 µM | | Example 17f | 0.81 µM |
| Example 9 | 16.2 µM | | Example 17g | 0.096 µM |
| Example 10 | 13 µM | | Example 17h | 0.25 µM |
| Example 11 | 0.73 µM | | Example 17i | 2.1 µM |
| Example 12 | 7.0 µM | | Example 17j | > 100 µM |
| Example 13a | 0.98 µM | | Example 19 | > 100 µM |
| Example 13b | 0.56 µM | | Example 20 | > 100 µM |
| Example 13C | 0.85 µM | | Example 21 | > 100 µM |
| Example 13d | 0.28 µM | | Example 22 | > 100 µM |
| Example 13e | 0.24 µM | | Example 23 | 46 µM |
| Example 14 | 0.35 µM | | | |

| | | | | |
|---|---|---|---|---|
| AF: Autofluorescent | | | | |

### UCH-L3

| | | | | |
|---|---|---|---|---|
| **Experimental N°** | **Uch-L3** | | **Experimental N°** | **Uch-L3** |
| Example 1 | 0.76 µM | | Example 16b | > 49 µM |
| Example 2 | AF | | Example 16c | > 100 µM |
| Example 3 | 13 µM | | Example 17a | 7.0 µM |
| Example 4 | > 100 µM | | Example 17b | 5.4 µM |
| Example 5 | 24 µM | | Example 17c | 2.0 µM |
| Example 6 | 1.1 µM | | Example 17d | 0.77 µM |
| Example 7 | 52 µM | | Example 17e | 2.1 µM |
| Example 8 | > 100 µM | | Example 17f | 0.60 µM |
| Example 9 | 8.8 µM | | Example 17g | 0.070 µM |
| Example 10 | 20 µM | | Example 17h | 0.33 µM |
| Example 11 | 0.60 µM | | Example 17i | 3.1 µM |
| Example 12 | 1.3 µM | | Example 17j | 8 µM |
| Example 13a | 2.2 µM | | Example 19 | > 100 µM |
| Example 13b | 10 µM | | Example 20 | 39.6 µM |
| Example 13C | > 100 µM | | Example 21 | > 100 µM |
| Example 13d | 1.3 µM | | Example 22 | 22 µM |
| Example 13e | 0.54 µM | | Example 23 | 86 µM |
| Example 14 | 0.39 µM | | | |

| | | | | |
|---|---|---|---|---|
| AF: Autofluorescent | | | | |

### Caspase 3

| **Experimental N°** | **Casp3** | | **Experimental N°** | **Casp3** |
|---|---|---|---|---|
| Example 1 | 0.69 µM | | Example 16b | > 100 µM |
| Example 2 | AF | | Example 16c | > 100 µM |
| Example 3 | > 100 µM | | Example 17a | 10 µM |
| Example 4 | > 100 µM | | Example 17b | 51 µM |
| Example 5 | > 100 µM | | Example 17c | 13 µM |
| Example 6 | 9.3 µM | | Example 17d | 1.8 µM |
| Example 7 | > 100 µM | | Example 17e | 3.3 µM |
| Example 8 | > 100 µM | | Example 17f | 2.0 µM |
| Example 9 | > 100 µM | | Example 17g | 0.29 µM |
| Example 10 | > 100 µM | | Example 17h | 1.0 µM |
| Example 11 | 2.3 µM | | Example 17i | 2.0 µM |
| Example 12 | 5.6 µM | | Example 17j | 47 µM |
| Example 13a | 52 µM | | Example 19 | > 100 µM |
| Example 13b | > 100 µM | | Example 20 | > 100 µM |
| Example 13C | > 100 µM | | Example 21 | > 100 µM |
| Example 13d | > 100 µM | | Example 22 | > 100 µM |
| Example 13e | > 100 µM | | Example 23 | > 100 µM |
| Example 14 | 1.3 µM | | | |

| | | | | |
|---|---|---|---|---|
| AF: Autofluorescent | | | | |

### Cathepsine B

| **Experimental N°** | **cathepB** | | **Experimental N°** | **cathepB** |
|---|---|---|---|---|
| Example 1 | 12 µM | | Example 14 | 74 µM |
| Example 2 | AF | | Example 16b | > 100 µM |
| Example 3 | > 100 µM | | Example 16c | > 100 µM |
| Example 4 | > 100 µM | | Example 17b | > 100 µM |
| Example 5 | > 100 µM | | Example 17c | > 100 µM |
| Example 6 | > 100 µM | | Example 17d | 87 µM |
| Example 7 | > 100 µM | | Example 17e | 20 µM |
| Example 8 | > 100 µM | | Example 17f | 52.6 µM |
| Example 9 | > 100 µM | | Example 17g | 1.5 µM |
| Example 10 | > 100 µM | | Example 17h | 6.0 µM |
| Example 11 | 11.1 µM | | Example 17i | 32 µM |
| Example 12 | 21 µM | | Example 17j | 40 µM |
| Example 13a | > 100 µM | | Example 19 | > 100 µM |
| Example 13b | > 100 µM | | Example 20 | > 100 µM |
| Example 13C | > 100 µM | | Example 21 | > 100 µM |
| Example 13d | > 100 µM | | Example 22 | > 100 µM |
| Example 13e | > 100 µM | | Example 23 | > 100 µM |

| | | | | |
|---|---|---|---|---|
| AF: Autofluorescent | | | | |

### 2. Inhibition of cell viability and proliferation

| **Experimental N°** | **HCT116 D3 GI50** | | **Experimental N°** | **HCT116 D3 GI50** |
|---|---|---|---|---|
| Example 1 | 0.15 µM | | Example 16c | 1.47 µM |
| Example 2 | >10 µM | | Example 17a | 0.61 µM |
| Example 3 | >10 µM | | Example 17b | 0.53 µM |
| Example 4 | >10 µM | | Example 17c | 0.39 µM |
| Example 5 | >10 µM | | Example 17d | 0.68 µM |
| Example 6 | >10 µM | | Example 17e | 0.30 µM |
| Example 7 | >10 µM | | Example 17f | 0.93 µM |
| Example 8 | >10 µM | | Example 17g | 0.32 µM |
| Example 9 | > 10 µM | | Example 17h | 0.16 µM |
| Example 10 | 3.4 µM | | Example 17i | 0.56 µM |
| Example 11 | 1.60 µM | | Example 17j | >10 µM |
| Example 12 | 6.5 µM | | Example 19 | 10 µM |
| Example 13a | 0.39 µM | | Example 20 | 0.78 µM |
| Example 13b | 0.58 µM | | Example 21 | > 10 µM |
| Example 13c | 0.75 µM | | Example 22 | > 10 µM |
| Example 13d | 1.18 µM | | Example 23 | >10 µM |
| Example 13e | 0.54 µM | | Example 24a | 0.50 µM |
| Example 14 | 0.43 µM | | Example 24b | 0.94 µM |
| Example 16b | 2.85 µM | | Example 25 | 0.19 µM |
| | | | | |

| **Experimental N°** | **PC3 D3 GI50** | | **Experimental N°** | **PC3 D3 GI50** |
|---|---|---|---|---|
| Example 1 | 0.61 µM | | Example 16c | 3.37 µM |
| Example 2 | >10 µM | | Example 17a | 1.40 µM |
| Example 3 | >10 µM | | Example 17b | 0.92 µM |
| Example 4 | >10 µM | | Example 17c | 0.93 µM |
| Example 5 | > 10 µM | | Example 17d | 0.91 µM |
| Example 6 | >10 µM | | Example 17e | 1.32 µM |
| Example 7 | >10 µM | | Example 17f | 0.80 µM |
| Example 8 | >10 µM | | Example 17g | 1.11 µM |
| Example 9 | > 10 µM | | Example 17h | 0.57 µM |
| Example 10 | 4.4 µM | | Example 17i | 1.27 µM |
| Example 11 | 1.24 µM | | Example 17j | >10 µM |
| Example 12 | 4.4 µM | | Example 19 | 4.2 µM |
| Example 13a | 0.43 µM | | Example 20 | 1.26 µM |
| Example 13b | 0.54 µM | | Example 21 | > 10 µM |
| Example 13C | 0.70 µM | | Example 22 | > 10 µM |
| Example 13d | 1.54 µM | | Example 23 | > 10 µM |
| Example 13e | 0.53 µM | | Example 24a | 0.39 µM |
| Example 14 | 0.51 µM | | Example 24b | 0.62 µM |
| Example 16b | 2.78 µM | | Example 25 | 1.4 µM |

## Claims

1. A compound of formula (I): 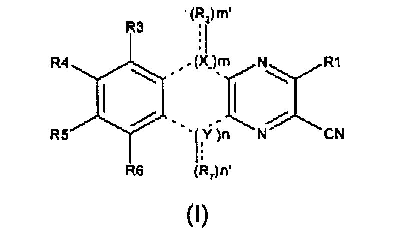 wherein :
m is 0 ; 1 or 2 , wherein when m=0, ----- (X(R2)_{m'})ₘ ----- is none so as to form an open ring or a single bond;
n is 0, 1 or 2, , wherein when n=0, ----- (Y(R7)_{n'})ₙ ----- is none so as to form an open ring or a single bond;
m' and n' are independently 0, 1 or 2;
X is a carbon atom or S or N;
Y is a carbon atom, or S or N;
Provided m and n are not simultaneously 0;
- - - - is either a single or double bond, as appropriate ;
- - - - is either none or a single bond, as appropriate;
R1 is chosen from the group consisting in H, CN, Hal, OAlk, OH, NRCN, C(CN)=C(OH)(OAlk), SR, NRR', C(O)NRR', Heterocycle, Aryle, Heteroaryle, where Alk, Aryle, Heteroaryle, heterocycle are optionally substituted by Hal, NRR', CN, OH, CF₃, Aryle, Heteroaryle , OAlk;
R3, R4, R5, R6 are each identical or different and are independently chosen from the group consisting in H, OAlk, Alk, Hal, NRR', CN, OH, CF₃, Aryle, Heteroaryle;
R2 is chosen from the group consisting in H, O, OH, N-OH, N-Aryle, N-OAlk, NO-Aryle, N-O-Alk-Aryle or 2 R2 bound at the same X form together with that X an heterocycle; wherein said Alk, Aryle or heterocycle are optionally substituted by OAlk, Alk, Hal, NRR', CN, OH, CF₃;
R7 is chosen from the group consisting in H, O, OH, N-OH, N-Aryle, N-OAlk, NO-Aryle, N-O-Alk-Aryle or 2 R7 bound at the same Y form together with that Y an heterocycle; wherein said Alk, Aryle or heterocycle are optionally substituted by OAlk, Alk, Hal, NRR', CN, OH, CF₃;
R and R' are each identical or different and are independently chosen from the group consisting in H, Alk, wherein Alk is optionally substituted by Hal, NRR', CN, OH, CF₃, Aryle, Heteroaryle;
or their pharmaceutically acceptable salts, hydrates, or hydrated salts, or the polymorphic crystalline structures of these compounds or their optical isomers, racemates, diastereomers or enantiomers,
with the exception of compounds where:
R3, R4, R5, R6=H, R1=CN, ---(X(R2)_{m'})ₘ--- represents a single bond, and
---(Y(R7)_{n'})ₙ--- represents -C(=N-(2-,4-,6-trimethylphenyl))-, -C(=N-(2-,6-dimethylphenyl))-, -C(=N-(2-,6-diethylphenyl))-, -C(=N-(2-methylphenyl))-, -C(=N-(2-ethylphenyl))-, -C(=N-(2-trifluoromethylphenyl))-, -C(=N-(2-isopropylphenyl))-, -C(=N-phenyl)-, -C(=N-(naphtyl)- or-C(=O)-, -CH2-, or
R3, R5, R6=H, R4=OMe, R1=CN, ---(X(R2)_{m'})ₘ--- represents a single bond, and
---(Y(R7)_{n'})ₙ--- represents -C(=O)-, or
R3, R4, R5, R6=H, R1=NH2, ---(X(R2)_{m'})ₘ--- represents a single bond, and
---(Y(R7)_{n'})ₙ--- represents -CH2- or -CH2-CH2-; or
R3, R4, R5, R6=H, R1=NH2, ---(X(R2)_{m'})ₘ--- represents -CH2- or -CH2-CH2- and ---(Y(R7)_{n'})ₙ--- represents a single bond.

2. Compound according to claim 1 with the further exception of the following compound: R3, R4, R5, R6=H, R1=CN, ---(X(R2)_{m'})ₘ--- represents a single bond, and -(Y(R7)_{n'})ₙ- represents -C(=N-OH)-.

3. Compound according to claim 1 or 2, wherein:
R1 is chosen from the group consisting in H, CN, Hal, OAlk, OH, NRCN, C(CN)=C(OH)(OAlk), NRR', C(O)NRR', Heterocycle, where Alk is optionally substituted by OAlk and where Heterocycle is optionally substituted by Hal;
R3, R4, R5, R6 are each identical or different and are independently chosen from the group consisting in H, OAlk, Alk, Hal;
---(Y(R7)_{n'})ₙ--- is a single bond or Y represents a carbon atom or a S atom;
R2 is chosen from the group consisting in H, O;
R7 is chosen from the group consisting in H, O, OH, N-OH, N-OAlk, N-Aryle, NO-Aryle, N-O-Alk-Aryle or 2 R7 bound at the same Y form together with that Y an heterocycle;
R and R' are each identical or different and are independently chosen from the group consisting in H, Alk;

4. Compound according to anyone of the preceding claims, wherein ---(X(R2)_{m'})ₘ--- represents a single bond, n is 1, n' is 1, Y is a carbon atom.

5. Compound according to anyone of the preceding claims, wherein R1 is chosen from the group consisting in H, CN, Hal, OAlk, OH, NRCN, C(CN)=C(OH)(OAlk), SR, NRR', C(O)NRR', Heterocycle, where Alk is optionally substituted by OAlk and where Heterocycle is optionally substituted by Hal;

6. Compound according to anyone of the preceding claims, wherein R3, R4, R5, R6 are each identical or different and are independently chosen from the group consisting in H, OAlk, Alk, Hal.

7. Compound according to anyone of the preceding claims, wherein R7 is chosen from the group consisting in O, N-OH, N-OAlk, N-Aryle, N-O-Aryle, N-O-Alk-Aryle.

8. Compound according to anyone of the preceding claims, wherein R and R' are each identical or different and are independently chosen from the group consisting in H, Alk.

9. Compound according to anyone of the preceding claims chosen from the group consisting in:
9-hydroxy-3-methoxy-9H-indeno[1,2-b]pyrazine-2-carbonitrile
3-methoxy-9-oxo-9H-indeno[1,2-b]pyrazine-2-carbonitrile
3-dimethylamino-9-oxo-9H-indeno[1,2-b]pyrazine-2-carbonitrile
3-(2-methoxy-ethoxy)-9-oxo-9H-indeno[1,2-b]pyrazine-2-carbonitrile
3-hydroxy-9-oxo-9H-indeno[1,2-b]pyrazine-2-carbonitrile
3-amino-9-oxo-9H-indeno[1,2-b]pyrazine-2-carbonitrile
3-(4,4-difluoro-piperidin-1-yl)-9-oxo-9H-indeno[1,2-b]pyrazine-2-carbonitrile
3-chloro-9-oxo-9H-indeno[1,2-b]pyrazine-2-carbonitrile
9-(1',3'-dioxolan-2'-yl)-9H-indeno[1,2-b]pyrazine-2,3-dicarbonitrile
2-cyano-9-[hydroxyimino]-9H-indeno[1,2-b]pyrazine-3-carboxylic acid amide
9-(methoxyimino)-9H-indeno[1,2-b]pyrazine-2,3-dicarbonitrile
9-(Allyloxyimino)-9H-indeno[1,2-b]pyrazine-2,3-dicarbonitrile
9-Benzyloxyimino-9H-indeno[1,2-b]pyrazine-2,3-dicarbonitrile
9-Ethoxyimino-9H-indeno[1,2-b]pyrazine-2,3-dicarbonitrile
9-Phenoxyimino-9H-indeno[1,2-b]pyrazine-2,3-dicarbonitrile
6-Methoxy-9H-indeno[1,2-b]pyrazine-2,3-dicarbonitrile
6,7-Dimethoxy-9H-indeno[1,2-b]pyrazine-2,3-dicarbonitrile
8-Methyl-9H-indeno[1,2-b]pyrazine-2,3-dicarbonitrile
7,8-Dimethoxy-9H-indeno[1,2-b]pyrazine-2,3-dicarbonitrile
6-Methyl-9H-indeno[1,2-b]pyrazine-2,3-dicarbonitrile
5,8-Dimethoxy-9H-indeno[1,2-b]pyrazine-2,3-dicarbonitrile
6-Methoxy-9-oxo-9H-indeno[1,2-b]pyrazine-2,3-dicarbonitrile
6,7-Dimethoxy-9-oxo-9H-indeno[1,2-b]pyrazine-2,3-dicarbonitrile
8-Methyl-9-oxo-9H-indeno[1,2-b]pyrazine-2,3-dicarbonitrile
7,8-Dimethoxy-9-oxo-9H-indeno[1,2-b]pyrazine-2,3-dicarbonitrile
6-Methyl-9-oxo-9H-indeno[1,2-b]pyrazine-2,3-dicarbonitrile
5,8-Dimethoxy-9-oxo-9H-indeno[1,2-b]pyrazine-2,3-dicarbonitrile
7-Chloro-9-oxo-9H-indeno[1,2-b]pyrazine-2,3-dicarbonitrile
7-Fluoro-9-oxo-9H-indeno[1,2-b]pyrazine-2,3-dicarbonitrile
7-Hydroxy-9-oxo-9H-indeno[1,2-b]pyrazine-2,3-dicarbonitrile
benzo[4,5]thieno[2,3-b]pyrazine-2,3-dicarbonitrile
5,10-dioxo-5,10-dihydro-benzo[g]quinoxaline-2,3-dicarbonitrile
9-[hydroxyimino]-9H-indeno[1,2-b]pyrazine-2,3-dicarbonitrile
2-cyano-9-oxo-9H-indeno[1,2-b]pyrazin-3-yl-cyanamide
3-(1-cyano-2-ethoxy-2-hydroxy-vinyl)-9-oxo-9H-indeno[1,2-b]pyrazine-2-carbonitrile
3-ethylsulfanyl-9-oxo-9H-indeno[1,2-b]pyrazine-2-carbonitrile
7-Chloro-9-methoxyimino-9H-indeno[1,2-b]pyrazine-2,3-dicarbonitrile
9-Allyloxyimino-7-chloro-9H-indeno[1,2-b]pyrazine-2,3-dicarbonitrile
6-Chloro-9-oxo-9H-indeno[1,2-b]pyrazine-2,3-dicarbonitrile
or their pharmaceutically acceptable salts, hydrates, or hydrated salts, or
the polymorphic crystalline structures of these compounds or their optical isomers, racemates, diastereomers or enantiomers.

10. Compound according to anyone of the preceding claims chosen from the group consisting in:
9-hydroxy-3-methoxy-9H-indeno[1,2-b]pyrazine-2-carbonitrile
3-methoxy-9-oxo-9H-indeno[1,2-b]pyrazine-2-carbonitrile
3-dimethylamino-9-oxo-9H-indeno[1,2-b]pyrazine-2-carbonitrile
3-(2-methoxy-ethoxy)-9-oxo-9H-indeno[1,2-b]pyrazine-2-carbonitrile
3-hydroxy-9-oxo-9H-indeno[1,2-b]pyrazine-2-carbonitrile
3-amino-9-oxo-9H-indeno[1,2-b]pyrazine-2-carbonitrile
3-(4,4-difluoro-piperidin-1-yl)-9-oxo-9H-indeno[1,2-b]pyrazine-2-carbonitrile
3-chloro-9-oxo-9H-indeno[1,2-b]pyrazine-2-carbonitrile
9-(1',3'-dioxolan-2'-yl)-9H-indeno[1,2-b]pyrazine-2,3-dicarbonitrile
2-cyano-9-[hydroxyimino]-9H-indeno[1,2-b]pyrazine-3-carboxylic acid amide
9-(methoxyimino)-9H-indeno[1,2-b]pyrazine-2,3-dicarbonitrile
9-(Allyloxyimino)-9H-indeno[1,2-b]pyrazine-2,3-dicarbonitrile
9-Benzyloxyimino-9H-indeno[1,2-b]pyrazine-2,3-dicarbonitrile
9-Ethoxyimino-9H-indeno[1,2-b]pyrazine-2,3-dicarbonitrile
9-Phenoxyimino-9H-indeno[1,2-b]pyrazine-2,3-dicarbonitrile
6-Methoxy-9H-indeno[1,2-b]pyrazine-2,3-dicarbonitrile
6,7-Dimethoxy-9H-indeno[1,2-b]pyrazine-2,3-dicarbonitrile
8-Methyl-9H-indeno[1,2-b]pyrazine-2,3-dicarbonitrile
7,8-Dimethoxy-9H-indeno[1,2-b]pyrazine-2,3-dicarbonitrile
6-Methyl-9H-indeno[1,2-b]pyrazine-2,3-dicarbonitrile
5,8-Dimethoxy-9H-indeno[1,2-b]pyrazine-2,3-dicarbonitrile
6-Methoxy-9-oxo-9H-indeno[1,2-b]pyrazine-2,3-dicarbonitrile
6,7-Dimethoxy-9-oxo-9H-indeno[1,2-b]pyrazine-2,3-dicarbonitrile
8-Methyl-9-oxo-9H-indeno[1,2-b]pyrazine-2,3-dicarbonitrile
7,8-Dimethoxy-9-oxo-9H-indeno[1,2-b]pyrazine-2,3-dicarbonitrile
6-Methyl-9-oxo-9H-indeno[1,2-b]pyrazine-2,3-dicarbonitrile
5,8-Dimethoxy-9-oxo-9H-indeno[1,2-b]pyrazine-2,3-dicarbonitrile
7-Chloro-9-oxo-9H-indeno[1,2-b]pyrazine-2,3-dicarbonitrile
7-Fluoro-9-oxo-9H-indeno[1,2-b]pyrazine-2,3-dicarbonitrile
7-Hydroxy-9-oxo-9H-indeno[1,2-b]pyrazine-2,3-dicarbonitrile
benzo[4,5]thieno[2,3-b]pyrazine-2,3-dicarbonitrile
5,10-dioxo-5,10-dihydro-benzo[g]quinoxaline-2,3-dicarbonitrile
2-cyano-9-oxo-9H-indeno[1,2-b]pyrazin-3-yl-cyanamide
3-(1-cyano-2-ethoxy-2-hydroxy-vinyl)-9-oxo-9H-indeno[1,2-b]pyrazine-2-carbonitrile
3-ethylsulfanyl-9-oxo-9H-indeno[1,2-b]pyrazine-2-carbonitrile
7-Chloro-9-methoxyimino-9H-indeno[1,2-b]pyrazine-2,3-dicarbonitrile
9-Allyloxyimino-7-chloro-9H-indeno[1,2-b]pyrazine-2,3-dicarbonitrile
6-Chloro-9-oxo-9H-indeno[1,2-b]pyrazine-2,3-dicarbonitrile
or their pharmaceutically acceptable salts, hydrates, or hydrated salts, or the polymorphic crystalline structures of these compounds or their optical isomers, racemates, diastereomers or enantiomers.

11. Compound according to anyone of the preceding claims chosen from the group consisting in:
2-cyano-9-[hydroxyimino]-9H-indeno[1,2-b]pyrazine-3-carboxylic acid amide
9-(methoxyimino)-9H-indeno[1,2-b]pyrazine-2,3-dicarbonitrile
9-Benzyloxyimino-9H-indeno[1,2-b]pyrazine-2,3-dicarbonitrile (13c).
9-Ethoxyimino-9H-indeno[1,2-b]pyrazine-2,3-dicarbonitrile (13d).
9-Phenoxyimino-9H-indeno[1,2-b]pyrazine-2,3-dicarbonitrile (13e).
8-Methyl-9-oxo-9H-indeno[1,2-b]pyrazine-2,3-dicarbonitrile
6-Methyl-9-oxo-9H-indeno[1,2-b]pyrazine-2,3-dicarbonitrile
5,8-Dimethoxy-9-oxo-9H-indeno[1,2-b]pyrazine-2,3-dicarbonitrile
7-Chloro-9-oxo-9H-indeno[1,2-b]pyrazine-2,3-dicarbonitrile
7-Fluoro-9-oxo-9H-indeno[1,2-b]pyrazine-2,3-dicarbonitrile
or their pharmaceutically acceptable salts, hydrates, or hydrated salts, or the polymorphic crystalline structures of these compounds or their optical isomers, racemates, diastereomers or enantiomers.

12. Process of preparation of a compound according to anyone of the preceding claims comprising the step of reacting a corresponding compound of formula (II) 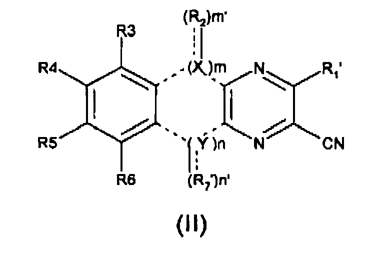 wherein R3, R4, R5, R6, X, Y, m, m', n, n' are defined as in formula (I) and R7' is R7 as defined in formula (I) or a precursor thereof and R1' is R1 as defined in formula (I) or a precursor thereof.

13. Process according to claim 12, wherein R1' is CN.

14. Process according to claim 12 or 13, wherein ---(Y(R7)_{n'})ₙ--- is -C(=O)-.

15. Process according to claim 12 to 14, wherein said compound of formula (II) is obtained from a corresponding compound of formula (III) or (III') 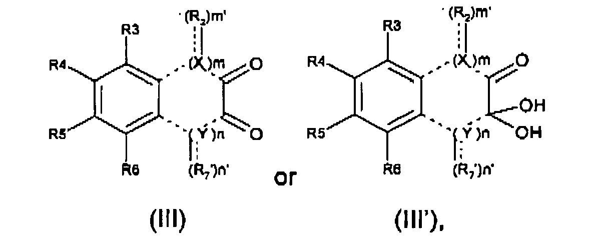 wherein R3, R4, R5, R6, X, Y, m, m', n, n' are defined as in formula (I) and R7' is defined as in formula (II).

16. Process according to claim 15, wherein when R1'=CN, this step is carried out in the presence of diaminomaleodinitrile.

17. Process according to claim 12 to 14, wherein said compound of formula (II) is obtained from a corresponding compound of formula (IV) 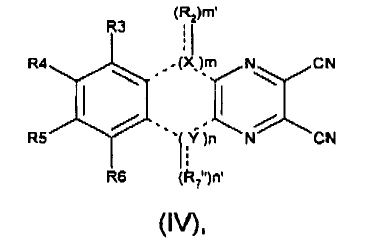 wherein R3, R4, R5, R6, X, Y, m, m', n, n' are defined as in formula (I) and R7" represents R7' or a precursor thereof, if appropriate.

18. A pharmaceutical composition comprising a compound of formula 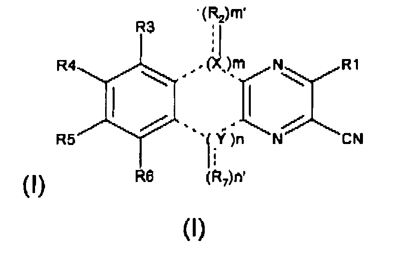 wherein :
m is 0 ; 1 or 2 , wherein when m=0, ----(X(R2)_{m'})ₘ----- is none so as to form an open ring or a single bond;
n is 0, 1 or 2, , wherein when n=0, ----- (Y(R7)_{n'})ₙ ----- is none so as to form an open ring or a single bond;
m' and n' are independently 0, 1 or 2;
X is a carbon atom or S or N;
Y is a carbon atom, or S or N;
Provided m and n are not simultaneously 0;
- - - - is either a single or double bond, as appropriate ;
- - - - - - is either none or a single bond, as appropriate;
R1 is chosen from the group consisting in H, CN, Hal, OAlk, OH, NRCN, C(CN)=C(OH)(OAlk), NRR', C(O)NRR', Heterocycle, Aryle, Heteroaryle, where Alk, Aryle, Heteroaryle, Heterocycle are optionally substituted by Hal, NRR', CN, OH, CF₃, Aryle, Heteroaryle, OAlk;
R3, R4, R5, R6 are each identical or different and are independently chosen from the group consisting in H, OAlk, Alk, Hal, NRR', CN, OH, CF₃, Aryle, Heteroaryle;
R2 is chosen from the group consisting in H, O, OH, N-OH, N-Aryle, N-OAlk, NO-Aryle, N-O-Alk-Aryle or 2 R2 bound at the same X form together with that X an heterocycle; wherein said Alk, Aryle or heterocycle are optionally substituted by OAlk, Alk, Hal, NRR', CN, OH, CF₃;
R7 is chosen from the group consisting in H, O, OH, N-OH, N-Aryle, N-OAlk, NO-Aryle, N-O-Alk-Aryle or 2 R7 bound at the same Y form together with that Y an heterocycle; wherein said Alk, Aryle or heterocycle are optionally substituted by OAlk, Alk, Hal, NRR', CN, OH, CF₃;
R and R' are each identical or different and are independently chosen from the group consisting in H, Alk, wherein Alk is optionally substituted by Hal, NRR', CN, OH, CF₃, Aryle, Heteroaryle;
or their pharmaceutically acceptable salts, hydrates, or hydrated salts, or the polymorphic crystalline structures of these compounds or their optical isomers, racemates, diastereomers or enantiomers.

19. Pharmaceutical composition according to claim 18, wherein the compound of formula (I) is defined as in anyone of claims 1 to 9.

20. Pharmaceutical composition according to claim 18 or 19, wherein said compound of formula (I) is chosen from the group consisting in:
9-oxo-9H-indeno[1,2-b]pyrazine-2,3-dicarbonitrile
9-hydroxy-3-methoxy-9H-indeno[1,2-b]pyrazine-2-carbonitrile
3-methoxy-9-oxo-9H-indeno[1,2-b]pyrazine-2-carbonitrile
3-dimethylamino-9-oxo-9H-indeno[1,2-b]pyrazine-2-carbonitrile
3-(2-methoxy-ethoxy)-9-oxo-9H-indeno[1,2-b]pyrazine-2-carbonitrile
3-hydroxy-9-oxo-9H-indeno[1,2-b]pyrazine-2-carbonitrile
3-amino-9-oxo-9H-indeno[1,2-b]pyrazine-2-carbonitrile
3-(4,4-difluoro-piperidin-1-yl)-9-oxo-9H-indeno[1,2-b]pyrazine-2-carbonitrile
3-chloro-9-oxo-9H-indeno[1,2-b]pyrazine-2-carbonitrile
9-(1',3'-dioxolan-2'-yl)-9H-indeno[1,2-b]pyrazine-2,3-dicarbonitrile
2-cyano-9-[hydroxyimino]-9H-indeno[1,2-b]pyrazine-3-carboxylic acid amide
9-[hydroxyimino]-9H-indeno[1,2-b]pyrazine-2,3-dicarbonitrile
9-(methoxyimino)-9H-indeno[1,2-b]pyrazine-2,3-dicarbonitrile
9-(Allyloxyimino)-9H-indeno[1,2-b]pyrazine-2,3-dicarbonitrile
9-Benzyloxyimino-9H-indeno[1,2-b]pyrazine-2,3-dicarbonitrile
9-Ethoxyimino-9H-indeno[1,2-b]pyrazine-2,3-dicarbonitrile
9-Phenoxyimino-9H-indeno[1,2-b]pyrazine-2,3-dicarbonitrile
9-[phenylimino]-9H-indeno[1,2-b]pyrazine-2,3-dicarbonitrile
6-Methoxy-9H-indeno[1,2-b]pyrazine-2,3-dicarbonitrile
6,7-Dimethoxy-9H-indeno[1,2-b]pyrazine-2,3-dicarbonitrile
8-Methyl-9H-indeno[1,2-b]pyrazine-2,3-dicarbonitrile
7,8-Dimethoxy-9H-indeno[1,2-b]pyrazine-2,3-dicarbonitrile
6-Methyl-9H-indeno[1,2-b]pyrazine-2,3-dicarbonitrile
5,8-Dimethoxy-9H-indeno[1,2-b]pyrazine-2,3-dicarbonitrile
6-Methoxy-9-oxo-9H-indeno[1,2-b]pyrazine-2,3-dicarbonitrile
6,7-Dimethoxy-9-oxo-9H-indeno[1,2-b]pyrazine-2,3-dicarbonitrile
8-Methyl-9-oxo-9H-indeno[1,2-b]pyrazine-2,3-dicarbonitrile
7,8-Dimethoxy-9-oxo-9H-indeno[1,2-b]pyrazine-2,3-dicarbonitrile
6-Methyl-9-oxo-9H-indeno[1,2-b]pyrazine-2,3-dicarbonitrile
5,8-Dimethoxy-9-oxo-9H-indeno[1,2-b]pyrazine-2,3-dicarbonitrile
7-Chloro-9-oxo-9H-indeno[1,2-b]pyrazine-2,3-dicarbonitrile
7-Fluoro-9-oxo-9H-indeno[1,2-b]pyrazine-2,3-dicarbonitrile
7-Methoxy-9-oxo-9H-indeno[1,2-b]pyrazine-2,3-dicarbonitrile
7-Hydroxy-9-oxo-9H-indeno[1,2-b]pyrazine-2,3-dicarbonitrile
benzo[4,5]thieno[2,3-b]pyrazine-2,3-dicarbonitrile
5,10-dioxo-5,10-dihydro-benzo[g]quinoxaline-2,3-dicarbonitrile
2-cyano-9-oxo-9H-indeno[1,2-b]pyrazin-3-yl-cyanamide
3-(1-cyano-2-ethoxy-2-hydroxy-vinyl)-9-oxo-9H-indeno[1,2-b]pyrazine-2-carbonitrile
3-ethylsulfanyl-9-oxo-9H-indeno[1,2-b]pyrazine-2-carbonitrile
7-Chloro-9-methoxyimino-9H-indeno[1,2-b]pyrazine-2,3-dicarbonitrile
9-Allyloxyimino-7-chloro-9H-indeno[1,2-b]pyrazine-2,3-dicarbonitrile
6-Chloro-9-oxo-9H-indeno[1,2-b]pyrazine-2,3-dicarbonitrile
or their pharmaceutically acceptable salts, hydrates, or hydrated salts, or the polymorphic crystalline structures of these compounds or their optical isomers, racemates, diastereomers or enantiomers.

21. Use of a compound of formula (I) as defined in anyone of claims 18 to 20 for the preparation of a medicament for inhibiting one or more cysteine proteases.

22. Use according to claim 21, wherein said cysteine proteases belong to one or more groups of de-ubiquitination enzymes, caspases, cathepsins, calpains as well as viral, bacterial, fungal or parasitic cysteine proteases.

23. Use of a compound of formula (I) as defined in anyone of claims 18 to 20 for the preparation of a medicament for treating and/or preventing cancer and metastasis, neurodegenerative diseases, such as Alzheimer's disease and Parkinson's disease, inflammatory disorders, cardiovascular diseases and/or viral infectivity and/or latency in particular for Herpes simplex virus-1, Epstein-Barr virus or SARS coronavirus.

24. Use according to claim 23, wherein said compound inhibits one or more de-ubiquitination enzymes.

25. Use of a compound of formula (I) as defined in anyone of claims 18 to 20 for the preparation of a medicament for treating and/or preventing inflammatory disorders, neurodegenerative disorders, preferably nervous cell damage caused by stroke, liver damage and liver failure resulting from acute or chronic infectious, ischemic or chemical liver injury, renal damage and renal failure resulting from acute or chronic infectious, ischemic or chemical kidney injury, heart damage and heart failure resulting from acute or chronic infectious, ischemic or chemical cardiac injury, diabetes resulting from acute or chronic autoimmune, chemical, oxidative or metabolic injury to the insulin beta-cells of the pancreatic islets.

26. Use according to claim 25, wherein said compound inhibits one or more caspases.

27. Use of a compound of formula (I) as defined in anyone of claims 18 to 20 for the preparation of a medicament for treating and/or preventing cancer and metastasis, cardiovascular diseases, immunological disorders, bone and joint diseases, osteoporosis and arthritis.

28. Use according to claim 27, wherein said compound inhibits one or more cathepsins.

29. Use of a compound of formula (I) as defined in anyone of claims 18 to 20 for the preparation of a medicament for treating and/or preventing ageing disorders, late onset diabetes and cataract.

30. Use according to claim 29, wherein said compound inhibits one or more calpains.

31. Use of a compound of formula (I) as defined in anyone of claims 18 to 20 for the preparation of a medicament for treating and/or preventing viral infections and diseases.

32. Use according to claim 31, wherein said viral infections and diseases are chosen from hepatitis A, hepatitis C, SARS coronavirus infection and disease, rhinoviral infections and diseases, adenoviral infections and diseases, poliomyelitis.

33. Use according to claim 31 or 32, wherein said compound inhibits one or more viral cysteine proteases.

34. Use of a compound of formula (I) as defined in anyone of claims 18 to 20 for the preparation of a medicament for treating and/or preventing bacterial infections and diseases.

35. Use according to claim 34, wherein said bacterial infections or diseases are chosen from streptococcal infections and diseases, infections and diseases caused by bacteria of the *Clostridium sp.* Genus, staphylococcal infections and diseases, gingivitis and periodontal diseases.

36. Use according to claim 34 or 35, wherein said compound inhibits one or more bacterial cysteine proteases.

37. Use according to anyone of claims 34 to 36, wherein said compound inhibits one or more bacterial cysteine proteases chosen from streptopain, clostripain, staphylococcal cysteine protease, gingipain.

38. Use of a compound of formula (I) as defined in anyone of claims 18 to 20 for the preparation of a medicament for treating and/or preventing fungal infections and diseases.

39. Use according to claim 38, wherein said compound inhibits one or more fungal cysteine protease.

40. Use of a compound of formula (I) as defined in anyone of claims 18 to 20 for the preparation of a medicament for treating and/or preventing protozoal parasitic infections and diseases.

41. Use according to claim 40, wherein said compound inhibits one or more cysteine proteases from protozoal parasites.

42. Use of a compound of formula (I) as defined in anyone of claims 18 to 20 for the preparation of a medicament for treating and/or preventing flat worm parasitic infections and diseases.

43. Use according to claim 42, wherein said compound inhibits one or more cysteine proteases from flat worm parasites.

44. Use of a compound of formula (I) as defined in anyone of claims 18 to 20 for the preparation of a medicament for treating and/or preventing round worm parasitic infections and diseases.

45. Use according to claim 44, wherein said compound inhibits one or more cysteine proteases from round worm parasites.

46. Use according to anyone of claims 23 to 45, wherein said medicament is used in combination with one or more therapies chosen from anti-cancer therapies, neurological therapies, thrombolytic therapies, antioxidant therapies anti-infective, anti-hypertensive therapies, diuretic therapies, thrombolytic therapies, immunosuppressive therapies, cardiovascular therapies, immunomodulatory therapies, anti-inflammatory therapies, antiviral therapies, anti-bacterial therapies, anti-fungal therapies, anti-protozoal therapies, antiparasitic therapies.

## Patentansprüche

1. Eine Verbindung nach Formel (I): 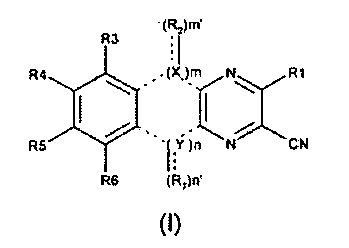 wobei:
m 0, 1 oder 2 ist, wobei wenn m = 0, ist ---(X(R2)_{m'})ₘ--- nichts, um einen offenen Ring oder eine Einfachbindung zu bilden;
n 0, 1 oder 2 ist, wobei wenn n = 0, ist ---(Y(R7)_{n'})ₙ--- nichts, um einen offenen Ring oder eine Einfachbindung zu bilden;
m' und n' unabhängig 0, 1 oder 2 sind;
X ein Kohlenstoffatom oder S oder N ist;
Y ein Kohlenstoffatom oder S oder N ist;
vorausgesetzt, dass m und n nicht gleichzeitig 0 sind;
- - - - entweder eine Einfach- oder eine Doppelbindung ist, soweit erforderlich;
- - - entweder keine oder eine Einfachbindung ist, soweit erforderlich;
R₁ aus der Gruppe, die aus H, CN, Hal, OAlk, OH, NRCN, C(CN)=C(OH)(OAlk), SR, NRR', C(O)NRR', Heterocyclus, Aryl, Heteroaryl besteht, ausgewählt wird, wobei Alk, Aryl, Heteroaryl, Heterocyclus optional substituiert sind durch Hal, NRR', CN, OH, CF₃, Aryl, Heteroaryl, OAlk;
R3, R4, R5, R6 jeweils gleich oder verschieden sind und unabhängig aus der Gruppe, die aus H, OAlk, Alk, Hal, NRR', CN, OH, CF₃, Aryl, Heteroaryl besteht, ausgewählt werden;
R2 aus der Gruppe, die aus H, O, OH, N-OH, N-Aryl, N-OAlk N-O-Aryl, N-O-Alk-Aryl besteht, ausgewählt wird, oder 2 R2, die an das gleiche X gebunden sind, zusammen mit dem X einen Heterocyclus bilden, wobei Alk, Aryl oder der Heterocyclus optional mit OAlk, Alk, Hal, NRR', CN, OH, CF₃ substituiert sind;
R7 aus der Gruppe, die aus H, O, OH, N-OH, N-Aryl, N-OAlk, N-O-Aryl, N-O-Alk-Aryl besteht, ausgewählt wird, oder 2 R7, die an das gleiche Y gebunden sind, zusammen mit dem Y einen Heterocyclus bilden, wobei Alk, Aryl oder der Heterocyclus optional mit OAlk, Alk, Hal, NRR', CN, OH, CF₃ substituiert sind;
R und R' jeweils gleich oder verschieden sind und unabhängig aus der Gruppe, die aus H, Alk besteht, ausgewählt werden, wobei Alk optional mit Hal, NRR', CN, OH, CF₃, Aryl, Heteroaryl substituiert ist;
oder ihre pharmazeutisch geeigneten Salze, Hydrate oder hydrierten Salze oder die polymorphen kristallinen Strukturen dieser Verbindungen oder ihre optischen Isomere, Racemate, Diastereomere oder Enantiomere;
mit der Ausnahme der Verbindungen, bei denen:
R3, R4, R5, R6 = H, R1 = CN, ---(X(R2)_{m'})ₘ--- eine Einfachbindung darstellt, und ---(Y(R7)_{n'})ₙ--- -C(=N-(2-,4-,6-Trimethylphenyl))-, -C(=N-(2-,6-Dimethylphenyl))-, -C(=N-(2-,6-Diethylphenyl))-, -C(=N-(2-Methylphenyl))-, -C(=N-(2-Ethylphenyl))-, -C(=N-(2-Trifluormethylphenyl))-, -C(=N-(2-Isopropylphenyl))-,
-C(=N-Phenyl)-, -C(=N-(Naphtyl) oder -C(=O)-, -CH₂- darstellt, oder
R3, R5, R6 = H, R4 = OMe, R1 = CN, ---(X(R2)_{m'})ₘ--- eine Einfachbindung darstellt und ---(Y(R7)_{n'})ₙ--- -C(=O)-darstellt, oder
R3, R4, R5, R6 = H, R1 - = NH₂, ---(X(R2)_{m'})ₘ--- eine Einfachbindung darstellt und ---(Y(R7)_{n'})ₙ--- -CH₂- oder -CH₂-CH₂- darstellt; oder
R3, R4, R5, R6 = H, R1 = NH₂, ---(X(R2)_{m'})ₘ--- -CH₂- oder -CH₂-CH₂- darstellt und ---(Y(R7)_{n'})ₙ--- eine Einfachbindung darstellt.

2. Verbindung gemäß Anspruch 1 mit der weiteren Ausnahme der folgenden Verbindung: R3, R4, R5, R6 = H, R1 = CN, ---(X(R2)_{m'})ₘ--- stellt eine Einfachbindung dar und ---(Y(R7)_{n'})ₙ--- stellt -C(=N-OH)- dar.

3. Verbindung nach Anspruch 1 oder 2, wobei:
R1 aus der Gruppe, die aus H, CN, Hal, OAlk, OH, NRCN, C(CN)=C(OH)(OAlk), NRR', C(O)NRR', Heterocyclus besteht, ausgewählt wird, wobei Alk optional durch OAlk substituiert ist und wobei Heterocyclus optional durch Hal substituiert ist;
R3, R4, R5, R6 jeweils gleich oder verschieden sind und unabhängig aus der Gruppe, die aus H, OAlk, Alk, Hal besteht, ausgewählt werden;
---(Y(R7)_{n'})ₙ--- eine Einfachbindung ist oder Y ein Kohlenstoffatom oder ein S-Atom darstellt;
R2 aus der Gruppe, die aus H, O besteht, ausgewählt wird; R7 aus der Gruppe, die aus H, O, OH, N-OH, N-OAlk, N-Aryl, N-O-Aryl, N-O-Alk-Aryl besteht, ausgewählt wird oder 2 R7, die an dasselbe Y gebunden sind, zusammen mit dem Y einen Heterocyclus bilden;
R und R' gleich oder verschieden sind und unabhängig aus der Gruppe, die aus H, Alk besteht, ausgewählt werden.

4. Verbindung gemäß einem der vorangegangenen Ansprüche, wobei --- (X (R₂) _{m'})ₘ--- eine Einfachbindung darstellt, n 1 ist, n' 1 ist und Y ein Kohlenstoffatom ist.

5. Verbindung gemäß einem der vorangegangenen Ansprüche, wobei R1 aus der Gruppe, die aus H, CN, Hal, OAlk, OH, NRCN, C(CN)=C(OH)(OAlk), SR, NRR', C(O)NRR', Heterocyclus besteht, ausgewählt wird, wobei Alk optional durch OAlk substituiert ist und wobei Heterocyclus optional durch Hal substituiert ist.

6. Verbindung gemäß einem der vorangegangenen Ansprüche, wobei R3, R4, R5, R6 jeweils gleich oder verschieden sind und unabhängig aus der Gruppe, die aus H, OAlk, Alk, Hal besteht, ausgewählt werden.

7. Verbindung gemäß einem der vorangegangenen Ansprüche, wobei R7 aus der Gruppe, die aus O, N-OH, N-OAlk, N-Aryl, N-O-Aryl, N-O-Alk-Aryl besteht, ausgewählt wird.

8. Verbindung gemäß einem der vorangegangenen Ansprüche, wobei R und R' gleich oder verschieden sind und unabhängig aus der Gruppe, die aus H, Alk besteht, ausgewählt werden.

9. Verbindung gemäß einem der vorangegangenen Ansprüche, die aus der Gruppe, die aus
9-Hydroxy-3-methoxy-9H-indeno[1,2-b]pyrazin-2-carbonitril;
3-Methoxy-9-oxo-9H-indeno[1,2-b]pyrazin-2-carbonitril;
3-Dimethylamino-9-oxo-9H-indeno[1,2-b]pyrazin-2-carbonitril;
3-(2-Methoxy-ethoxy)-9-oxo-9H-indeno[1,2-b]pyrazin-2-carbonitril;
3-Hydroxy-9-oxo-9H-indeno[1,2-b]pyrazin-2-carbonitril;
3-Amino-9-oxo-9H-indeno[1,2-b]pyrazin-2-carbonitril;
3-(4,4-Difluorpiperidin-1-yl)-9-oxo-9H-indeno[1,2-b]pyrazin-2-carbonitril;
3-Chlor-9-oxo-9H-indeno[1,2-b]pyrazin-2-carbonitril;
9-(1',3'-Dioxolan-2'-yl)-9H-indeno[1,2-b]pyrazin-2,3-dicarbonitril;
2-Cyano-9-[hydroxyimino]-9H-indeno[1,2-b]pyrazin-3-carbonsäureamid;
9-(Methoxyimino)-9H-indeno[1,2-b]pyrazin-2,3-dicarbonitril;
9-(Allyloxyimino)-9H-indeno[1,2-b]pyrazin-2,3-dicarbonitril;
9-Benzyloxyimino-9H-indeno[1,2-b]pyrazin-2,3-dicarbonitril;
9-Ethoxyimino-9H-indeno[1,2-b]pyrazin-2,3-dicarbonitril;
9-Phenoxyimino-9H-indeno[1,2-b]pyrazin-2,3-dicarbonitril;
6-Methoxy-9H-indeno[1,2-b]pyrazin-2,3-dicarbonitril;
6,7-Dimethoxy-9H-indeno[1,2-b]pyrazin-2,3-dicarbonitril;
8-Methyl-9H-indeno[1,2-b]pyrazin-2,3-dicarbonitril;
7,8-Dimethoxy-9H-indeno[1,2-b]pyrazin-2,3-dicarbonitril;
6-Methyl-9H-indeno[1,2-b]pyrazin-2,3-dicarbonitril;
5,8-Dimethoxy-9H-indeno[1,2-b]pyrazin-2,3-dicarbonitril;
6-Methoxy-9-oxo-9H-indeno[1,2-b]pyrazin-2,3-dicarbonitril;
6,7-Dimethoxy-9-oxo-9H-indeno[1,2-b]pyrazin-2,3-dicarbonitril;
8-Methyl-9-oxo-9H-indeno[1,2-b]pyrazin-2,3-dicarbonitril;
7,8-Dimethoxy-9-oxo-9H-indeno[1,2-b]pyrazin-2,3-dicarbonitril;
6-Methyl-9-oxo-9H-indeno[1,2-b]pyrazin-2,3-dicarbonitril;
5,8-Dimethoxy-9-oxo-9H-indeno[1,2-b]pyrazin-2,3-dicarbonitril;
7-Chlor-9-oxo-9H-indeno[1,2-b]pyrazin-2,3-dicarbonitril;
7-Fluor-9-oxo-9H-indeno[1,2-b]pyrazin-2,3-dicarbonitril;
7-Hydroxy-9-oxo-9H-indeno[1,2-b]pyrazin-2,3-dicarbonitril;
Benzo[4,5]thieno[2,3-b]pyrazin-2,3-dicarbonitril;
5,10-Dioxo-5,10-dihydro-benzo[g]chinoxalin-2,3-dicarbonitril;
9-[Hydroxyimino]-9H-indeno[1,2-b]pyrazin-2,3-dicarbonitril;
2-Cyano-9-oxo-9H-indeno[1,2-b]pyrazin-3-yl-cyanamid;
3-(1-Cyano-2-ethoxy-2-hydroxyvinyl)-9-oxo-9H-indeno[1,2-b]pyrazin-2-carbonitril;
3-Ethylsulfanyl-9-oxo-9H-indeno[1,2-b]pyrazin-2-carbonitril;
7-Chlor-9-methoxyimino-9H-indeno[1,2-b]pyrazin-2,3-dicarbonitril;
9-Allyloxyimino-7-chlor-9H-indeno[1,2-b]pyrazin-2,3-dicarbonitril;
6-Chlor-9-oxo-9H-indeno[1,2-b]pyrazin-2,3-dicarbonitril; besteht, ausgewählt wird,
oder ihre pharmazeutisch geeigneten Salze, Hydrate oder hydrierten Salze oder die polymorphen kristallinen Strukturen dieser Verbindungen oder ihre optischen Isomere, Racemate, Diastereomere oder Enantiomere.

10. Verbindung gemäß einem der vorangegangenen Ansprüche, die aus der Gruppe, die aus
9-Hydroxy-3-methoxy-9H-indeno[1,2-b]pyrazin-2-carbonitril;
3-Methoxy-9-oxo-9H-indeno[1,2-b]pyrazin-2-carbonitril;
3-Dimethylamino-9-oxo-9H-indeno[1,2-b]pyrazin-2-carbonitril;
3-(2-Methoxy-ethoxy)-9-oxo-9H-indeno[1,2-b]pyrazin-2-carbonitril;
3-Hydroxy-9-oxo-9H-indeno[1,2-b]pyrazin-2-carbonitril;
3-Amino-9-oxo-9H-indeno[1,2-b]pyrazin-2-carbonitril;
3-(4,4-Difluorpiperidin-1-yl)-9-oxo-9H-indeno[1,2-b]pyrazin-2-carbonitril;
3-Chlor-9-oxo-9H-indeno[1,2-b]pyrazin-2-carbonitril;
9-(1',3'-Dioxolan-2'-yl)-9H-indeno[1,2-b]pyrazin-2,3-dicarbonitril;
2-Cyano-9-[hydroxyimino]-9H-indeno[1,2-b]pyrazin-3-carbonsäureamid;
9-(Methoxyimino)-9H-indeno[1,2-b]pyrazin-2,3-dicarbonitril;
9-(Allyloxyimino)-9H-indeno[1,2-b]pyrazin-2,3-dicarbonitril;
9-Benzyloxyimino-9H-indeno[1,2-b]pyrazin-2,3-dicarbonitril;
9-Ethoxyimino-9H-indeno[1,2-b]pyrazin-2,3-dicarbonitril;
9-Phenoxyimino-9H-indeno[1,2-b]pyrazin-2,3-dicarbonitril;
6-Methoxy-9H-indeno[1,2-b]pyrazin-2,3-dicarbonitril;
6,7-Dimethoxy-9H-indeno[1,2-b]pyrazin-2,3-dicarbonitril;
8-Methyl-9H-indeno[1,2-b]pyrazin-2,3-dicarbonitril;
7,8-Dimethoxy-9H-indeno[1,2-b]pyrazin-2,3-dicarbonitril;
6-Methyl-9H-indeno[1,2-b]pyrazin-2,3-dicarbonitril;
5,8-Dimethoxy-9H-indeno[1,2-b]pyrazin-2,3-dicarbonitril;
6-Methoxy-9-oxo-9H-indeno[1,2-b]pyrazin-2,3-dicarbonitril;
6,7-Dimethoxy-9-oxo-9H-indeno[1,2-b]pyrazin-2,3-dicarbonitril;
8-Methyl-9-oxo-9H-indeno[1,2-b]pyrazin-2,3-dicarbonitril;
7,8-Dimethoxy-9-oxo-9H-indeno[1,2-b]pyrazin-2,3-dicarbonitril;
6-Methyl-9-oxo-9H-indeno[1,2-b]pyrazin-2,3-dicarbonitril;
5,8-Dimethoxy-9-oxo-9H-indeno[1,2-b]pyrazin-2,3-dicarbonitril;
7-Chlor-9-oxo-9H-indeno[1,2-b]pyrazin-2,3-dicarbonitril;
7-Fluor-9-oxo-9H-indeno[1,2-b]pyrazin-2,3-dicarbonitril;
7-Hydroxy-9-oxo-9H-indeno[1,2-b]pyrazin-2,3-dicarbonitril;
Benzo[4,5]thieno[2,3-b]pyrazin-2,3-dicarbonitril;
5,10-Dioxo-5,10-dihydro-benzo[g]chinoxalin-2,3-dicarbonitril;
2-Cyano-9-oxo-9H-indeno[1,2-b]pyrazin-3-yl-cyanamid;
3-(1-Cyano-2-ethoxy-2-hydroxyvinyl)-9-oxo-9H-indeno[1,2-b]pyrazin-2-carbonitril;
3-Ethylsulfanyl-9-oxo-9H-indeno[1,2-b]pyrazin-2-carbonitril;
7-Chlor-9-methoxyimino-9H-indeno[1,2-b]pyrazin-2,3-dicarbonitril;
9-Allyloxyimino-7-chlor-9H-indeno[1,2-b]pyrazin-2,3-dicarbonitril;
6-Chlor-9-oxo-9H-indeno[1,2-b]pyrazin-2,3-dicarbonitril; besteht, ausgewählt wird,
oder ihre pharmazeutisch geeigneten Salze, Hydrate oder hydrierten Salze oder die polymorphen kristallinen Strukturen dieser Verbindungen oder ihre optischen Isomere, Racemate, Diastereomere oder Enantiomere.

11. Verbindung gemäß einem der vorangegangenen Ansprüche, die aus der Gruppe, die aus
2-Cyano-9-[hydroxyimino]-9H-indeno[1,2-b]pyrazin-3-carbonsäureamid;
9-(Methoxyimino)-9H-indeno[1,2-b]pyrazin-2,3-dicarbonitril;
9-Benzyloxyimino-9H-indeno[1,2-b]pyrazin-2,3-dicarbonitril (13c);
9-Ethoxyimino-9H-indeno[1,2-b]pyrazin-2,3-dicarbonitril (13d);
9-Phenoxyimino-9H-indeno[1,2-b]pyrazin-2,3-dicarbonitril (13e);
8-Methyl-9-oxo-9H-indeno[1,2-b]pyrazin-2,3-dicarbonitril;
6-Methyl-9-oxo-9H-indeno[1,2-b]pyrazin-2,3-dicarbonitril;
5,8-Dimethoxy-9-oxo-9H-indeno[1,2-b]pyrazin-2,3-dicarbonitril;
7-Chlor-9-oxo-9H-indeno[1,2-b]pyrazin-2,3-dicarbonitril;
7-Fluor-9-oxo-9H-indeno[1,2-b]pyrazin-2,3-dicarbonitril; besteht, ausgewählt wird,
oder ihre pharmazeutisch geeigneten Salze, Hydrate oder hydrierten Salze oder die polymorphen kristallinen Strukturen dieser Verbindungen oder ihre optischen Isomere, Racemate, Diastereomere oder Enantiomere.

12. Verfahren zur Herstellung einer Verbindung gemäß einem der vorangegangenen Ansprüche, das den Schritt des Umsetzens einer entsprechenden Verbindung nach Formel (II) 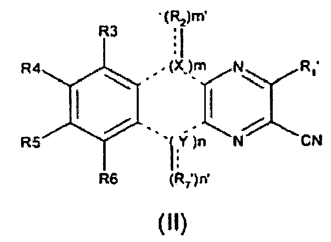 umfaßt, wobei R3, R4, R5, R6, X, Y, m, m', n, n' wie in Formel (I) definiert sind und R7' R7, wie in Formel (I) definiert, oder ein Precursor davon ist und R1' R1, wie in Formel (I) definiert, oder ein Precursor davon ist.

13. Verfahren gemäß Anspruch 12, wobei R1' -CN ist.

14. Verfahren gemäß Anspruch 12 oder 13, wobei ---(Y(R7)_{n'})ₙ--- -(C=O)- ist.

15. Verfahren gemäß Anspruch 12 bis 14, wobei die Verbindung nach Formel (II) aus einer entsprechenden Verbindung nach Formel (III) oder (III') 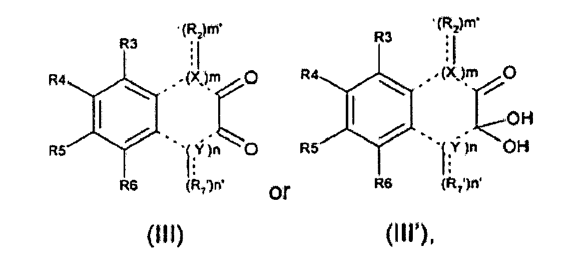 erhalten wird,
wobei R3, R4, R5, R6, X, Y, m, m', n, n' wie in Formel (I) definiert sind und R7' wie in Formel (II) definiert ist.

16. Verfahren gemäß Anspruch 15, wobei wenn R1' = CN, dieser Schritt in Gegenwart von Diaminomaleodinitril ausgeführt wird.

17. Verfahren gemäß Anspruch 12 bis 14, wobei die Verbindung nach Formel (II) aus einer entsprechenden Verbindung nach Formel (IV) 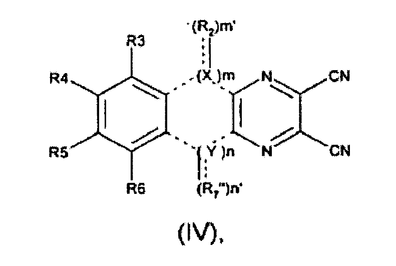 erhalten wird,
wobei R3, R4, R5, R6, X, Y, m, m', n, n' wie in Formel (I) definiert sind und R7" R7' oder einen Precursor davon darstellt, wenn erforderlich.

18. Eine pharmazeutischen Zusammensetzung, die eine Verbindung nach Formel (I) umfasst 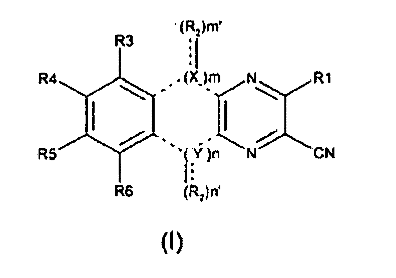 wobei:
m 0, 1 oder 2 ist, wobei wenn m = 0, ist ---(X(R2)_{m'})ₘ--- nichts, um einen offenen Ring oder eine Einfachbindung zu bilden;
n 0, 1 oder 2 ist, wobei wenn n = 0, ist ---(Y(R7)_{n'})ₙ--- nichts, um einen offenen Ring oder eine Einfachbindung zu bilden;
m' und n' unabhängig 0, 1 oder 2 sind;
X ein Kohlenstoffatom oder S oder N ist;
Y ein Kohlenstoffatom oder S oder N ist;
vorausgesetzt, dass m und n nicht gleichzeitig 0 sind;
- - - - entweder eine Einfach- oder eine Doppelbindung ist, soweit erforderlich;
- - - entweder keine oder eine Einfachbindung ist, soweit erforderlich;
R₁ aus der Gruppe, die aus H, CN, Hal, OAlk, OH, NRCN, C(CN)=C(OH)(OAlk), NRR', C(O)NRR', Heterocyclus, Aryl, Heteroaryl besteht, ausgewählt wird, wobei Alk, Aryl, Heteroaryl, Heterocyclus optional substituiert sind durch Hal, NRR', CN, OH, CF₃, Aryl, Heteroaryl, OAlk;
R3, R4, R5, R6 jeweils gleich oder verschieden sind und unabhängig aus der Gruppe, die aus H, OAlk, Alk, Hal, NRR', CN, OH, CF₃, Aryl, Heteroaryl besteht, ausgewählt werden;
R2 aus der Gruppe, die aus H, O, OH, N-OH, N-Aryl, N-OAlk, N-O-Aryl, N-O-Alk-Aryl besteht, ausgewählt wird, oder 2 R2, die an das gleiche X gebunden sind, zusammen mit dem X einen Heterocyclus bilden, wobei das Alk, Aryl oder der Heterocyclus optional mit OAlk, Alk, Hal, NRR', CN, OH, CF₃ substituiert sind;
R7 aus der Gruppe, die aus H, O, OH, N-OH, N-Aryl, N-OAlk, N-O-Aryl, N-O-Alk-Aryl besteht, ausgewählt wird, oder 2 R7, die an das gleiche Y gebunden sind, zusammen mit dem Y einen Heterocyclus bilden, wobei das Alk, Aryl oder der Heterocyclus optional mit OAlk, Alk, Hal, NRR', CN, OH, CF₃ substituiert sind;
R und R' jeweils gleich oder verschieden sind und unabhängig aus der Gruppe, die aus H, Alk besteht, ausgewählt werden, wobei Alk optional durch Hal, NRR', CN, OH, CF₃, Aryl, Heteroaryl substituiert ist;
oder ihre pharmazeutisch geeigneten Salze, Hydrate oder hydrierten Salze oder die polymorphen kristallinen Strukturen dieser Verbindungen oder ihre optischen Isomere, Racemate, Diastereomere oder Enantiomere.

19. Pharmazeutische Zusammensetzung gemäß Anspruch 18, wobei die Verbindung nach Formel (I) wie in jedem der Ansprüche 1 bis 9 definiert ist.

20. Pharmazeutische Zusammensetzung gemäß Anspruch 18 oder 19, wobei die Verbindung nach Formel (I) aus der Gruppe, die aus
9-Oxo-9H-indeno[1,2-b]pyrazin-2,3-dicarbonitril;
9-Hydroxy-3-methoxy-9H-indeno[1,2-b]pyrazin-2-carbonitril;
3-Methoxy-9-oxo-9H-indeno[1,2-b]pyrazin-2-carbonitril;
3-Dimethylamino-9-oxo-9H-indeno[1,2-b]pyrazin-2-carbonitril;
3-(2-Methoxy-ethoxy)-9-oxo-9H-indeno[1,2-b]pyrazin-2-carbonitril;
3-Hydroxy-9-oxo-9H-indeno[1,2-b]pyrazin-2-carbonitril;
3-Amino-9-oxo-9H-indeno[1,2-b]pyrazin-2-carbonitril;
3-(4,4-Difluorpiperidin-1-yl)-9-oxo-9H-indeno[1,2-b]pyrazin-2-carbonitril;
3-Chlor-9-oxo-9H-indeno[1,2-b]pyrazin-2-carbonitril;
9-(1',3'-Dioxolan-2'-yl)-9H-indeno[1,2-b]pyrazin-2,3-dicarbonitril;
2-Cyano-9-[hydroxyimino]-9H-indeno[1,2-b]pyrazin-3-carbonsäureamid;
9-[Hydroxyimino]-9H-indeno[1,2-b]pyrazin-2,3-dicarbonitril;
9-(Methoxyimino)-9H-indeno[1,2-b]pyrazin-2,3-dicarbonitril;
9-(Allyloxyimino)-9H-indeno[1,2-b]pyrazin-2,3-dicarbonitril;
9-Benzyloxyimino-9H-indeno[1,2-b]pyrazin-2,3-dicarbonitril;
9-Ethoxyimino-9H-indeno[1,2-b]pyrazin-2,3-dicarbonitril;
9-Phenoxyimino-9H-indeno[1,2-b]pyrazin-2,3-dicarbonitril;
9-[Phenylimino]-9H-indeno[1,2-b]pyrazin-2,3-dicarbonitril;
6-Methoxy-9H-indeno[1,2-b]pyrazin-2,3-dicarbonitril;
6,7-Dimethoxy-9H-indeno[1,2-b]pyrazin-2,3-dicarbonitril;
8-Methyl-9H-indeno[1,2-b]pyrazin-2,3-dicarbonitril;
7,8-Dimethoxy-9H-indeno[1,2-b]pyrazin-2,3-dicarbonitril;
6-Methyl-9H-indeno[1,2-b]pyrazin-2,3-dicarbonitril;
5,8-Dimethoxy-9H-indeno[1,2-b]pyrazin-2,3-dicarbonitril;
6-Methoxy-9-oxo-9H-indeno[1,2-b]pyrazin-2,3-dicarbonitril;
6,7-Dimethoxy-9-oxo-9H-indeno[1,2-b]pyrazin-2,3-dicarbonitril;
8-Methyl-9-oxo-9H-indeno[1,2-b]pyrazin-2,3-dicarbonitril;
7,8-Dimethoxy-9-oxo-9H-indeno[1,2-b]pyrazin-2,3-dicarbonitril;
6-Methyl-9-oxo-9H-indeno[1,2-b]pyrazin-2,3-dicarbonitril;
5,8-Dimethoxy-9-oxo-9H-indeno[1,2-b]pyrazin-2,3-dicarbonitril;
7-Chlor-9-oxo-9H-indeno[1,2-b]pyrazin-2,3-dicarbonitril;
7-Fluor-9-oxo-9H-indeno[1,2-b]pyrazin-2,3-dicarbonitril;
7-Methoxy-9-oxo-9H-indeno[1,2-b]pyrazin-2,3-dicarbonitril;
7-Hydroxy-9-oxo-9H-indeno[1,2-b]pyrazin-2,3-dicarbonitril;
Benzo[4,5]thieno[2,3-b]pyrazin-2,3-dicarbonitril;
5,10-Dioxo-5,10-dihydro-benzo[g]chinoxalin-2,3-dicarbonitril;
2-Cyano-9-oxo-9H-indeno[1,2-b]pyrazin-3-yl-cyanamid;
3-(1-Cyano-2-ethoxy-2-hydroxyvinyl)-9-oxo-9H-indeno[1,2-b]pyrazin-2-carbonitril;
3-Ethylsulfanyl-9-oxo-9H-indeno[1,2-b]pyrazin-2-carbonitril;
7-Chlor-9-methoxyimino-9H-indeno[1,2-b]pyrazin-2,3-dicarbonitril;
9-Allyloxyimino-7-chlor-9H-indeno[1,2-b]pyrazin-2,3-dicarbonitril;
6-Chlor-9-oxo-9H-indeno[1,2-b]pyrazin-2,3-dicarbonitril besteht, ausgewählt wird,
oder ihre pharmazeutisch geeigneten Salze, Hydrate oder hydrierten Salze oder die polymorphen kristallinen Strukturen dieser Verbindungen oder ihre optischen Isomere, Racemate, Diastereomere oder Enantiomere.

21. Verwendung einer Verbindung nach Formel (I), wie in einem der Ansprüche 18 bis 20 definiert, für die Herstellung eines Medikaments zur Inhibierung einer oder mehrerer Cysteinproteasen.

22. Verwendung gemäß Anspruch 21, wobei die Cysteinproteasen zu einer oder mehreren Gruppen von De-Ubiquitinierungsenzymen, Caspasen, Cathepsine, Calpaine als auch virale, bakterielle, Pilz- oder parasitäre Cysteinproteasen gehören.

23. Verwendung einer Verbindung nach Formel (I), wie in einem der Ansprüche 18 bis 20 definiert, zur Herstellung eines Medikaments zur Behandlung und/oder Prävention von Krebs und Metastase, neurodegenerative Erkrankungen, wie zum Beispiel Alzheimer Erkrankung und Parkinson Erkrankung, Entzündungskrankheiten, cardiovaskuläre Erkrankungen und/oder virale Infektiosität und/oder Latenz, insbesondere für Herpes Simplex Virus 1, Epstein-Barr Virus oder SARS Coronavirus.

24. Verwendung gemäß Anspruch 23, wobei die Verbindung ein oder mehrere De-Ubiquitinierungsenzyme inhibiert.

25. Verwendung einer Verbindung nach Formel (I), wie in einem der Ansprüche 18 bis 20 definiert, zur Herstellung eines Medikaments zur Behandlung oder Prävention von Entzündungskrankheiten, neurodegenerativen Krankheiten, insbesondere Nervenzellenschäden, die durch Schlaganfall verursacht werden, Leberschäden und Leberversagen, das aus akuten oder chronischen Infektionen resultiert, Ischämie oder chemische Leberverletzung, Nierenschaden und Nierenversagen, das aus akuten oder chronischen Infektionen resultiert, Ischämie oder chemische Nierenverletzung, Herzschaden und Herzversagen, das aus akuten oder chronischen Infektionen resultiert, Ischämie oder chemische Herzverletzung, Diabetes, das aus akuter oder chronischer Autoimmun-, chemischer, oxidativer oder metabolischer Verletzung der Insulin Beta-Zellen der Langerhansschen Inseln resultiert.

26. Verwendung gemäß Anspruch 25, wobei die Verbindung eine oder mehrere Caspasen inhibiert.

27. Verwendung einer Verbindung nach Formel (I), wie in einem der Ansprüche 18 bis 20 definiert, zur Herstellung eines Medikaments zur Behandlung und/oder Prävention von Krebs und Metastasen, cardiovaskulären Erkrankungen, Immunkrankheiten, Knochen- und Gelenkerkrankungen, Osteoporose und Arthritis.

28. Verwendung gemäß Anspruch 27, wobei die Verbindung eine oder mehrere Cathepsine inhibiert.

29. Verwendung einer Verbindung nach Formel (I), wie in einem der Ansprüche 18 bis 20 definiert, zur Herstellung eines Medikaments zur Behandlung und/oder Prävention von Alterskrankheiten, Altersdiabetes und Katarakt.

30. Verwendung gemäß Anspruch 29, wobei die Verbindung eine oder mehrere Calpaine inhibiert.

31. Verwendung einer Verbindung nach Formel (I), wie in einem der Ansprüche 18 bis 20 definiert, zur Herstellung eines Medikaments zur Behandlung und/oder Prävention viraler Infektionen und Erkrankungen.

32. Verwendung gemäß Anspruch 31, wobei die viralen Infektionen und Erkrankungen aus Hepatitis A, Hepatitis C, SARS Coronovirus Infektion und Erkrankung, rhinovirale Infektionen und Erkrankungen, adenovirale Infektionen und Erkrankungen, Poliomyelitis ausgewählt werden.

33. Verwendung gemäß Anspruch 31 oder 32, wobei die Verbindung eine oder mehrere virale Cysteinproteasen inhibiert.

34. Verwendung einer Verbindung nach Formel (I), wie in einem der Ansprüche 18 bis 20 definiert, zur Herstellung eines Medikaments zur Behandlung und/oder Prävention bakterieller Infektionen und Erkrankungen.

35. Verwendung gemäß Anspruch 34, wobei die bakteriellen Infektionen und Erkrankungen aus Streptokokken-Infektionen und Erkrankungen, Infektionen und Erkrankungen, die durch Bakterien des *Clostridium sp.* Genus verursacht werden, Staphylokokken-Infektionen und Erkrankungen, Gingivitis und Parodontalerkrankungen ausgewählt werden.

36. Verwendung gemäß Anspruch 34 oder 35, wobei die Verbindungen eine oder mehrere bakterielle Cysteinproteasen inhibieren.

37. Verwendung gemäß einem der Ansprüche 34 bis 36, wobei die Verbindungen eine oder mehrere bakterielle Cysteinproteasen inhibieren, die aus Streptopain, Clostripain, Staphylokokken-Cysteinprotease, Gingipain ausgewählt werden.

38. Verwendung einer Verbindung nach Formel (I), wie in einem der Ansprüche 18 bis 20 definiert, zur Herstellung eines Medikaments zur Behandlung und/oder Prävention von Pilzinfektionen und Erkrankungen.

39. Verwendung gemäß Anspruch 38, wobei die Verbindung eine oder mehrere Pilzcysteinproteasen inhibiert.

40. Verwendung einer Verbindung nach Formel (I), wie in einem der Ansprüche 18 bis 20 definiert, zur Herstellung eines Medikaments zur Behandlung und/oder Prävention von protozoalen Parasiteninfektionen und Erkrankungen.

41. Verwendung gemäß Anspruch 40, wobei die Verbindung eine oder mehrere Cysteinproteasen von protozoalen Parasiten inhibiert.

42. Verwendung einer Verbindung nach Formel (I), wie in einem der Ansprüche 18 bis 20 definiert, zur Herstellung eines Medikaments zur Behandlung und/oder Prävention Plattwurm-Parasiteninfektionen und Erkrankungen.

43. Verwendung gemäß Anspruch 42, wobei die Verbindung eine oder mehrere Cysteinproteasen von Plattwurmparasiten inhibiert.

44. Verwendung einer Verbindung nach Formel (I), wie in einem der Ansprüche 18 bis 20 definiert, zur Herstellung eines Medikaments zur Behandlung und/oder Prävention von Fadenwurm-Parasiteninfektionen und Erkrankungen.

45. Verwendung gemäß Anspruch 44, wobei die Verbindung eine oder mehrere Cysteinproteasen von Fadenwurmparasiten inhibiert.

46. Verwendung gemäß einem der Ansprüche 23 bis 45, wobei das Medikament zusammen mit einer oder mehreren Therapien verwendet wird, die aus Anti-Krebs-Therapien, neurologischen Therapien, thrombolytischen Therapien, Antioxidations-Therapien, anti-infektiösen-, antihypertensiven-Therapien, diuretischen Therapien, thrombolytischen Therapien, immunsuppressiven Therapien, cardiovaskulären Therapien, immunmodulatorischen Therapien, entzündungshemmenden Therapien, antiviralen Therapien, anti-bakteriellen Therapien, anti-Pilztherapien, anti-protozealen Therapien, antiparasitären Therapien ausgewählt werden.

## Revendications

1. Composé de formule (I) : 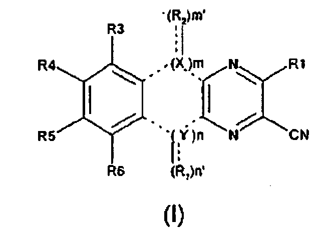 où :
m est 0 ; 1 ou 2, où lorsque m = 0, -----(X(R2)_{m'})ₘ----- est nul afin de former un cycle ouvert ou une liaison simple ;
n est 0, 1 ou 2, , où lorsque n = 0, -----(Y(R7)_{n')n}----- est nul afin de former un cycle ouvert ou une liaison simple ;
m' et n' sont indépendamment 0, 1 ou 2 ;
X est un atome de carbone ou S ou N ;
Y est un atome de carbone ou S ou N ;
à condition que m et n ne soient pas simultanément 0 ;
- - - - est soit une liaison simple ou double, de manière appropriée ;
- - - - - - est soit nul ou une liaison simple, de manière appropriée ;
R1 est choisi parmi le groupe consistant en H, CN, Hal, OAlk, OH, NRCN, C(CN)=C(OH)(OAlk), SR, NRR', C(O)NRR', Hétérocycle, Aryle, Hétéroaryle, où Alk, Aryle, Hétéroaryle, hétérocycle sont éventuellement substitués par Hal, NRR', CN, OH, CF₃, Aryle, Hétéroaryle, OAlk ;
R3, R4, R5, R6 sont chacun identiques ou différents et sont choisis indépendamment parmi le groupe consistant en H, OAlk, Alk, Hal, NRR', CN, OH, CF₃, Aryle, Hétéroaryle ;
R2 est choisi parmi le groupe consistant en H, O, OH, N-OH, N-Aryle, N-OAlk, N-O-Aryle, N-O-Alk-Aryle ou 2 R2 liés au même X forment conjointement avec ce X un hétérocycle ; où lesdits Alk, Aryle ou hétérocycle sont éventuellement substitués par OAlk, Alk, Hal, NRR', CN, OH, CF₃ ;
R7 est choisi parmi le groupe consistant en H, O, OH, N-OH, N-Aryle, N-OAlk, N-O-Aryle, N-O-Alk-Aryle ou 2 R7 liés au même Y forment conjointement avec ce Y un hétérocycle ; où lesdits Alk, Aryle ou hétérocycle sont éventuellement substitués par OAlk, Alk, Hal, NRR', CN, OH, CF₃ ;
R et R' sont chacun identiques ou différents et sont choisis indépendamment parmi le groupe consistant en H, Alk, où Alk est éventuellement substitué par Hal, NRR', CN, OH, CF₃, Aryle, Hétéroaryle ;
ou leurs sels, hydrates, ou sels hydratés pharmaceutiquement acceptables ou les structures cristallines polymorphiques de ces composés ou leurs isomères optiques, racémates, diastéréoisomères ou énantiomères,
à l'exception des composés où :
R3, R4, R5, R6 = H, R1 = CN, ---(X(R2)_{m'})ₘ--- représente une liaison simple, et ---(Y(R7)_{n'})ₙ--- représente -C(=N-(2-,4-,6-triméthylphényl))-, -C(=N-(2-,6-diméthylphényl))-, -C(=N-(2-,6-diéthylphényl))-, -C(=N-(2-méthylphényl))-, - C(=N-(2-éthylphényl))-, -C(=N-(2-trifluorométhylphényl))-, -C(=N-(2-isopropylphényl))-, -C(=N-phényl)-, -C(=N-(naphtyl)- ou -C(=O)-, -CH2-, ou
R3, R5, R6 = H, R4 = OMe, R1 = CN, --- (X(R2)_{m'})ₘ --- représente une liaison simple, et ---(Y(R7)_{n'})ₙ--- représente -C(=O)-, ou
R3, R4, R5, R6 = H, R1 = NH2, ---(X(R2)_{m'})ₘ--- représente une liaison simple, et --- (Y(R7)_{n'})ₙ--- représente -CH2- ou -CH2-CH2- ; ou
R3, R4, R5, R6 = H, R1 = NH2, ---(X(R2)_{m'})ₘ -- représente -CH2- ou -CH2-CH2- et ---(Y(R7)ₙ')ₙ--- représente une liaison simple.

2. Composé selon la revendication 1 à l'exception en outre du composé suivant R3, R4, R5, R6 = H, R1 = CN, ---(X(R2)_{m'})ₘ--- représente une liaison simple, et-(Y(R7)_{n'})ₙ--- représente -C(=N-OH)-.

3. Composé selon la revendication 1 ou 2, où :
R1 est choisi parmi le groupe consistant en H, CN, Hal, OAlk, OH, NRCN, C(CN)=C(OH)(OAIk), NRR', C(O)NRR', Hétérocycle, où Alk est éventuellement substitué par OAlk et où l'Hétérocycle est éventuellement substitué par Hal ;
R3, R4, R5, R6 sont chacun identiques ou différents et sont choisis indépendamment parmi le groupe consistant en H, OAlk, Alk, Hal ;
---(Y(R7)_{n')n}--- est une liaison simple ou Y représente un atome de carbone ou un atome de S ;
R2 est choisi parmi le groupe consistant en H, O ;
R7 est choisi parmi le groupe consistant en H, O, OH, N-OH, N-OAlk, N-Aryle, N-O-Aryle, N-O-Alk-Aryle ou 2 R7 liés au même Y forment conjointement avec ce Y un hétérocycle ;
R et R' sont chacun identiques ou différents et sont choisis indépendamment parmi le groupe consistant en H, Alk.

4. Composé selon l'une quelconque des revendications précédentes, où-(X(R2)_{m'})ₘ--- représente une liaison simple, n est 1, n' est 1, Y est un atome de carbone.

5. Composé selon l'une quelconque des revendications précédentes, où R1 est choisi parmi le groupe consistant en H, CN, Hal, OAlk, OH, NRCN, C(CN)=C(OH)(OAlk), SR, NRR', C(O)NRR', Hétérocycle, où Alk est éventuellement substitué par OAlk et où Hétérocycle est éventuellement substitué par Hal.

6. Composé selon l'une quelconque des revendications précédentes, où R3, R4, R5, R6 sont chacun identiques ou différents et sont choisis indépendamment parmi le groupe consistant en H, OAlk, Alk, Hal.

7. Composé selon l'une quelconque des revendications précédentes, où R7 est choisi parmi le groupe consistant en O, N-OH, N-OAlk, N-Aryle, N-O-Aryle, N-O-Alk-Aryle.

8. Composé selon l'une quelconque des revendications précédentes, où R et R' sont chacun identiques ou différents et sont choisis indépendamment parmi le groupe consistant en H, Alk.

9. Composé selon l'une quelconque des revendications précédentes choisi parmi le groupe consistant en :
9-hydroxy-3-méthoxy-9H-indéno[1,2-b]pyrazine-2-carbonitrile
3-méthoxy-9-oxo-9H-indéno[1,2-b]pyrazine-2-carbonitrile
3-diméthylamino-9-oxo-9H-indéno[1,2-b]pyrazine-2-carbonitrile
3-(2-méthoxy-éthoxy)-9-oxo-9H-indéno[1,2-b]pyrazine-2-carbonitrile
3-hydroxy-9-oxo-9H-indéno[1,2-b]pyrazine-2-carbonitrile
3-amino-9-oxo-9H-indéno[1,2-b]pyrazine-2-carbonitrile
3-(4,4-difluoro-pipéridin-1-yl)-9-oxo-9H-indéno[1,2-b]pyrazine-2-carbonitrile
3-chloro-9-oxo-9H-indéno[1,2-b]pyrazine-2-carbonitrile
9-(1',3'-dioxolan-2'-yl)-9H-indéno[1,2-b]pyrazine-2,3-dicarbonitrile
amide d'acide 2-cyano-9-[hydroxyimino]-9H-indéno[1,2-b]pyrazine-3-carboxylique
9-(méthoxyimino)-9H-indéno[1,2-b]pyrazine-2,3-dicarbonitrile
9-(allyloxyimino)-9H-indéno[1,2-b]pyrazine-2,3-dicarbonitrile
9-benzyloxyimino-9H-indéno[1,2-b]pyrazine-2,3-dicarbonitrile
9-éthoxyimino-9H-indéno[1,2-b]pyrazine-2,3-dicarbonitrile
9-phenoxyimino-9H-indéno[1,2-b]pyrazine-2,3-dicarbonitrile
6-méthoxy-9H-indéno[1,2-b]pyrazine-2,3-dicarbonitrile
6,7-diméthoxy-9H-indéno[1,2-b]pyrazine-2,3-dicarbonitrile
8-méthyl-9H-indéno[1,2-b]pyrazine-2,3-dicarbonitrile
7,8-diméthoxy-9H-indéno[1,2-b]pyrazine-2,3-dicarbonitrile
6-méthyl-9H-indéno[1,2-b]pyrazine-2,3-dicarbonitrile
5,8-diméthoxy-9H-indéno[1,2-b]pyrazine-2,3-dicarbonitrile
6-méthoxy-9-oxo-9H-indéno[1,2-b]pyrazine-2,3-dicarbonitrile
6,7-diméthoxy-9-oxo-9H-indéno[1,2-b]pyrazine-2,3-dicarbonitrile
8-méthyl-9-oxo-9H-indéno[1,2-b]pyrazine-2,3-dicarbonitrile
7,8-diméthoxy-9-oxo-9H-indéno[1,2-b]pyrazine-2,3-dicarbonitrile
6-méthyl-9-oxo-9H-indéno[1,2-b]pyrazine-2,3-dicarbonitrile
5,8-diméthoxy-9-oxo-9H-indéno[1,2-b]pyrazine-2,3-dicarbonitrile
7-chloro-9-oxo-9H-indéno[1,2-b]pyrazine-2,3-dicarbonitrile
7-fluoro-9-oxo-9H-indéno[1,2-b]pyrazine-2,3-dicarbonitrile
7-hydroxy-9-oxo-9H-indéno[1,2-b]pyrazine-2,3-dicarbonitrile
benzo[4,5]thiéno[2,3-b]pyrazine-2,3-dicarbonitrile
5,10-dioxo-5,10-dihydro-benzo[g]quinoxaline-2,3-dicarbonitrile
9-[hydroxyimino]-9H-indéno[1,2-b]pyrazine-2,3-dicarbonitrile
2-cyano-9-oxo-9H-indéno[1,2-b]pyrazin-3-yl-cyanamide
3-(1-cyano-2-éthoxy-2-hydroxy-vinyl)-9-oxo-9H-indéno[1,2-b]pyrazine-2-carbonitrile
3-éthylsulfanyl-9-oxo-9H-indéno[1,2-b]pyrazine-2-carbonitrile
7-chloro-9-méthoxyimino-9H-indéno[1,2-b]pyrazine-2,3-dicarbonitrile
9-allyloxyimino-7-chloro-9H-indéno[1,2-b]pyrazine-2,3-dicarbonitrile
6-chloro-9-oxo-9H-indéno[1,2-b]pyrazine-2,3-dicarbonitrile
ou leurs sels, hydrates, ou sels hydratés pharmaceutiquement acceptables ou les structures cristallines polymorphiques de ces composés ou leurs isomères optiques, racémates, diastéréoisomères ou énantiomères.

10. Composé selon l'une quelconque des revendications précédentes choisi parmi le groupe consistant en :
9-hydroxy-3-méthoxy-9H-indéno[1,2-b]pyrazine-2-carbonitrile
3-méthoxy-9-oxo-9H-indéno[1,2-b]pyrazine-2-carbonitrile
3-diméthylamino-9-oxo-9H-indéno[1,2-b]pyrazine-2-carbonitrile
3-(2-méthoxy-éthoxy)-9-oxo-9H-indéno[1,2-b]pyrazine-2-carbonitrile
3-hydroxy-9-oxo-9H-indéno[1,2-b]pyrazine-2-carbonitrile
3-amino-9-oxo-9H-indéno[1,2-b]pyrazine-2-carbonitrile
3-(4,4-difluoro-pipéridin-1-yl)-9-oxo-9H-indéno[1,2-b]pyrazine-2-carbonitrile
3-chloro-9-oxo-9H-indéno[1,2-b]pyrazine-2-carbonitrile
9-(1',3'-dioxolan-2'-yl)-9H-indéno[1,2-b]pyrazine-2,3-dicarbonitrile
amide d'acide 2-cyano-9-[hydroxyimino]-9H-indéno[1,2-b]pyrazine-3-carboxylique
9-(méthoxyimino)-9H-indéno[1,2-b]pyrazine-2,3-dicarbonitrile
9-(allyloxyimino)-9H-indéno[1,2-b]pyrazine-2,3-dicarbonitrile
9-benzyloxyimino-9H-indéno[1,2-b]pyrazine-2,3-dicarbonitrile
9-éthoxyimino-9H-indéno[1,2-b]pyrazine-2,3-dicarbonitrile
9-phénoxyimino-9H-indéno[1,2-b]pyrazine-2,3-dicarbonitrile
6-méthoxy-9H-indéno[1,2-b]pyrazine-2,3-dicarbonitrile
6,7-diméthoxy-9H-indéno[1,2-b]pyrazine-2,3-dicarbonitrile
8-méthyl-9H-indéno[1,2-b]pyrazine-2,3-dicarbonitrile
7,8-diméthoxy-9H-indéno[1,2-b]pyrazine-2,3-dicarbonitrile
6-méthyl-9H-indéno[1,2-b]pyrazine-2,3-dicarbonitrile
5,8-diméthoxy-9H-indéno[1,2-b]pyrazine-2,3-dicarbonitrile
6-méthoxy-9-oxo-9H-indéno[1,2-b]pyrazine-2,3-dicarbonitrile
6,7-diméthoxy-9-oxo-9H-indéno[1,2-b]pyrazine-2,3-dicarbonitrile
8-méthyl-9-oxo-9H-indéno[1,2-b]pyrazine-2,3-dicarbonitrile
7,8-diméthoxy-9-oxo-9H-indéno[1,2-b]pyrazine-2,3-dicarbonitrile
6-méthyl-9-oxo-9H-indéno[1,2-b]pyrazine-2,3-dicarbonitrile
5,8-diméthoxy-9-oxo-9H-indéno[1,2-b]pyrazine-2,3-dicarbonitrile
7-chloro-9-oxo-9H-indéno[1,2-b]pyrazine-2,3-dicarbonitrile
7-fluoro-9-oxo-9H-indéno[1,2-b]pyrazine-2,3-dicarbonitrile
7-hydroxy-9-oxo-9H-indéno[1,2-b]pyrazine-2,3-dicarbonitrile
benzo[4,5]thiéno[2,3-b]pyrazine-2,3-dicarbonitrile
5,10-dioxo-5,10-dihydro-benzo[g]quinoxaline-2,3-dicarbonitrile
2-cyano-9-oxo-9H-indéno[1,2-b]pyrazin-3-yl-cyanamide
3-(1-cyano-2-éthoxy-2-hydroxy-vinyl)-9-oxo-9H-indéno[1,2-b]pyrazine-2-carbonitrile
3-éthylsulfanyl-9-oxo-9H-indéno[1,2-b]pyrazine-2-carbonitrile
7-chloro-9-méthoxyimino-9H-indéno[1,2-b]pyrazine-2,3-dicarbonitrile
9-allyloxyimino-7-chloro-9H-indéno[1,2-b]pyrazine-2,3-dicarbonitrile
6-chloro-9-oxo-9H-indéno[1,2-b]pyrazine-2,3-dicarbonitrile
ou leurs sels, hydrates, ou sels hydratés pharmaceutiquement acceptables ou les structures cristallines polymorphiques de ces composés ou leurs isomères optiques, racémates, diastéréoisomères ou énantiomères,

11. Composé selon l'une quelconque des revendications précédentes choisi parmi le groupe consistant en :
amide d'acide 2-cyano-9-[hydroxyimino]-9H-indéno[1,2-b]pyrazine-3-carboxylique
9-(méthoxyimino)-9H-indéno [1,2-b]pyrazine-2,3-dicarbonitrile
9-benzyloxyimino-9H-indéno[1,2-b]pyrazine-2,3-dicarbonitrile (13c)
9-éthoxyimino-9H-indéno[1,2-b]pyrazine-2,3-dicarbonitrile (13d)
9-phenoxyimino-9H-indéno[1,2-b]pyrazine-2,3-dicarbonitrile (13e)
8-méthyl-9-oxo-9H-indéno[1,2-b]pyrazine-2,3-dicarbonitrile
6-méthyl-9-oxo-9H-indéno[1,2-b]pyrazine-2,3-dicarbonitrile
5,8-diméthoxy-9-oxo-9H-indéno[1,2-b]pyrazine-2,3-dicarbonitrile
7-chloro-9-oxo-9H-indéno[1,2-b]pyrazine-2,3-dicarbonitrile
7-fluoro-9-oxo-9H-indéno[1,2-b]pyrazine-2,3-dicarbonitrile
ou leurs sels, hydrates, ou sels hydratés pharmaceutiquement acceptables ou les structures cristallines polymorphiques de ces composés ou leurs isomères optiques, racémates, diastéréoisomères ou énantiomères.

12. Procédé de préparation d'un composé selon l'une quelconque des revendications précédentes comprenant l'étape consistant à faire réagir un composé correspondant de formule (II) 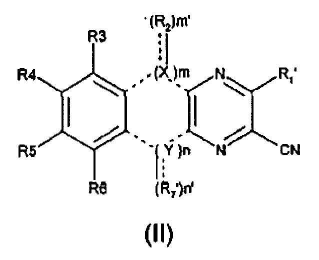 où R3, R4, R5, R6, X, Y, m, m', n, n' sont tels que définis dans la formule (I) et R7' est R7 comme défini dans la formule (I) ou son précurseur et R1' est R1 comme défini dans la formule (I) ou son précurseur.

13. Procédé selon la revendication 12, où R1' est CN.

14. Procédé selon la revendication 12 ou 13, où ---(Y(R7)_{n'})ₙ--- est -C(=O)-.

15. Procédé selon les revendications 12 à 14, où ledit composé de formule (II) est obtenu à partir d'un composé correspondant de formule (III) ou (III') 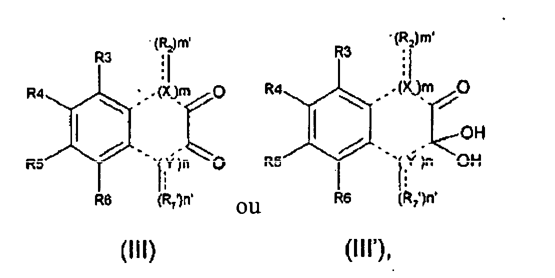 où R3, R4, R5, R6, X, Y, m, m', n, n' sont tels que définis dans la formule (I) et R7' est tel que défini dans la formule (II).

16. Procédé selon la revendication 15, où lorsque R1' = CN, cette étape est réalisée en présence de diaminomaléodinitrile.

17. Procédé selon les revendications 12 à 14, où ledit composé de formule (II) est obtenu à partir d'un composé correspondant de formule (IV) 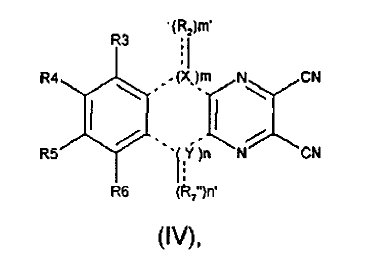 où R3, R4, R5, R6, X, Y, m, m', n, n' sont tels que définis dans la formule (I) et R7" représente R7' ou son précurseur, si approprié.

18. Composition pharmaceutique comprenant un composé de formule 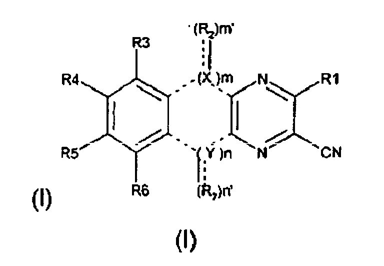 où :
m est 0 ; 1 ou 2, où lorsque m = 0, -----(X(R₂)_{m'})ₘ----- est nul afin de former un cycle ouvert ou une liaison simple ;
n est 0, 1 ou 2, , où lorsque n = 0, -----(Y(R7)_{n'})ₙ----- est nul afin de former un cycle ouvert ou une liaison simple ;
m' et n' sont indépendamment 0, 1 ou 2 ;
X est un atome de carbone ou S ou N ;
Y est un atome de carbone ou S ou N ;
à condition que m et n ne soient pas simultanément 0 ;
- - - - est soit une liaison simple ou double, de manière appropriée;
- - - - - - est soit nul ou une liaison simple, de manière appropriée ;
R1 est choisi parmi le groupe consistant en H, CN, Hal, OAlk, OH, NRCN, C(CN)=C(OH)(OAlk), NRR', C(O)NRR', Hétérocycle, Aryle, Hétéroaryle, où Alk, Aryle, Hétéroaryle, hétérocycle sont éventuellement substitués par Hal, NRR', CN, OH, CF₃, Aryle, Hétéroaryle, OAlk ;
R3, R4, R5, R6 sont chacun identiques ou différents et sont choisis indépendamment parmi le groupe consistant en H, OAlk, Alk, Hal, NRR', CN, OH, CF₃, Aryle, Hétéroaryle ;
R2 est choisi parmi le groupe consistant en H, O, OH, N-OH, N-Aryle, N-OAlk, N-O-Aryle, N-O-Alk-Aryle ou 2 R2 liés au même X forment conjointement avec ce X un hétérocycle ; où ledit Alk, Aryle ou hétérocycle sont éventuellement substitués par OAlk, Alk, Hal, NRR', CN, OH, CF₃ ;
R7 est choisi parmi le groupe consistant en H, O, OH, N-OH, N-Aryle, N-OAlk, N-O-Aryle, N-O-Alk-Aryle ou 2 R7 liés au même Y forment conjointement avec ce Y un hétérocycle ; où ledit Alk, Aryle ou hétérocycle sont éventuellement substitués par OAlk, Alk, Hal, NRR', CN, OH, CF₃ ;
R et R' sont chacun identiques ou différents et sont choisis indépendamment parmi le groupe consistant en H, Alk, où Alk est éventuellement substitué par Hal, NRR', CN, OH, CF₃, Aryle, Hétéroaryle ;
ou leurs sels, hydrates, ou sels hydratés pharmaceutiquement acceptables ou les structures cristallines polymorphiques de ces composés ou leurs isomères optiques, racémates, diastéréoisomères ou énantiomères.

19. Composition pharmaceutique selon la revendication 18, où le composé de formule (I) est tel que défini selon l'une quelconque des revendications 1 à 9.

20. Composition pharmaceutique selon la revendication 18 ou 19, où ledit composé de formule (I) est choisi parmi le groupe consistant en :
9-oxo-9H-indéno[1,2-b]pyrazine-2,3-dicarbonitrile
9-hydroxy-3-méthoxy-9H-indéno[1,2-b]pyrazine-2-carbonitrile
3-méthoxy-9-oxo-9H-indéno[1,2-b]pyrazine-2-carbonitrile
3-diméthylamino-9-oxo-9H-indéno[1,2-b]pyrazine-2-carbonitrile
3-(2-méthoxy-éthoxy)-9-oxo-9H-indéno[1,2-b]pyrazine-2-carbonitrile
3-hydroxy-9-oxo-9H-indéno[1,2-b]pyrazine-2-carbonitrile
3-amino-9-oxo-9H-indéno[1,2-b]pyrazine-2-carbonitrile
3-(4,4-difluoro-pipéridin-1-yl)-9-oxo-9H-indéno[1,2-b]pyrazine-2-carbonitrile
3-chloro-9-oxo-9H-indéno[1,2-b]pyrazine-2-carbonitrile
9-(1',3'-dioxolan-2'-yl)-9H-indéno[1,2-b]pyrazine-2,3-dicarbonitrile
amide d'acide 2-cyano-9-[hydroxyimino]-9H-indéno[1,2-b]pyrazine-3-carboxylique
9-[hydroxyimino]-9H-indéno[1,2-b]pyrazine-2,3-dicarbonitrile
9-(méthoxyimino)-9H-indéno[1,2-b]pyrazine-2,3-dicarbonitrile
9-(allyloxyimino)-9H-indéno[1,2-b]pyrazine-2,3-dicarbonitrile
9-benzyloxyimino-9H-indéno[1,2-b]pyrazine-2,3-dicarbonitrile
9-éthoxyimino-9H-indéno[1,2-b]pyrazine-2,3-dicarbonitrile
9-phenoxyimino-9H-indéno[1,2-b]pyrazine-2,3-dicarbonitrile
9-[phenylimino]-9H-indéno[1,2-b]pyrazine-2,3-dicarbonitrile
6-méthoxy-9H-indéno[1,2-b]pyrazine-2,3-dicarbonitrile
6,7-diméthoxy-9H-indéno[1,2-b]pyrazine-2,3-dicarbonitrile
8-méthyl-H-indéno[1,2-b]pyrazine-2,3-dicarbonitrile
7,8-diméthoxy-9H-indéno[1,2-b]pyrazine-2,3-dicarbonitrile
6-méthyl-9H-indéno[1,2-b]pyrazine-2,3-dicarbonitrile
5,8-diméthoxy-9H-indéno[1,2-b]pyrazine-2,3-dicarbonitrile
6-méthoxy-9-oxo-9H-indéno[1,2-b]pyrazine-2,3-dicarbonitrile
6,7-diméthoxy-9-oxo-9H-indéno[1,2-b]pyrazine-2,3-dicarbonitrile
8-méthyl-9-oxo-9H-indéno[1,2-b]pyrazine-2,3-dicarbonitrile
7,8-diméthoxy-9-oxo-9H-indéno[1,2-b]pyrazine-2,3-dicarbonitrile
6-méthyl-9-oxo-9H-indéno[1,2-b]pyrazine-2,3-dicarbonitrile
5,8-diméthoxy-9-oxo-9H-indéno[1,2-b]pyrazine-2,3-dicarbonitrile
7-chloro-9-oxo-9H-indéno[1,2-b]pyrazine-2,3-dicarbonitrile
7-fluoro-9-oxo-9H-indéno[1,2-b]pyrazine-2,3-dicarbonitrile
7-méthoxy-9-oxo-9H-indéno[1,2-b]pyrazine-2,3-dicarbonitrile
7-hydroxy-9-oxo-9H-indéno[1,2-b]pyrazine-2,3-dicarbonitrile benzo[4,5]thiéno[2,3-b]pyrazine-2,3-dicarbonitrile
5,10-dioxo-5,10-dihydro-benzo[g]quinoxaline-2,3-dicarbonitrile
2-cyano-9-oxo-9H-indéno[1,2-b]pyrazin-3-yl-cyanamide
3-(1-cyano-2-éthoxy-2-hydroxy-vinyl)-9-oxo-9H-indéno[1,2-b]pyrazine-2-carbonitrile
3-éthylsulfanyl-9-oxo-9H-indéno[1,2-b]pyrazine-2-carbonitrile
7-chloro-9-méthoxyimino-9H-indéno[1,2-b]pyrazine-2,3-dicarbonitrile
9-allyloxyimino-7-chloro-9H-indéno[1,2-b]pyrazine-2,3-dicarbonitrile
6-chloro-9-oxo-9H-indéno[1,2-b]pyrazine-2,3-dicarbonitrile
ou leurs sels, hydrates, ou sels hydratés pharmaceutiquement acceptables ou les structures cristallines polymorphiques de ces composés ou leurs isomères optiques, racémates, diastéréoisomères ou énantiomères.

21. Utilisation d'un composé de formule (I) tel que défini selon l'une quelconque des revendications 18 à 20 pour la préparation d'un médicament pour inhiber une ou plusieurs cystéine protéases.

22. Utilisation selon la revendication 21, où lesdites cystéine protéases appartiennent à un ou plusieurs groupes d'enzymes de désubiquitination, de caspases, de cathepsines, de calpaïnes ainsi que des cystéine protéases virales, bactériennes, fongiques ou parasitaires.

23. Utilisation d'un composé de formule (I) tel que défini selon l'une quelconque des revendications 18 à 20 pour la préparation d'un médicament pour traiter et/ou prévenir le cancer et la métastase, les maladies neurodégénératives, telles que la maladie d'Alzheimer et la maladie de Parkinson, les troubles inflammatoires, les maladies cardio-vasculaires et/ou l'infectivité et/ou la latence virales en particulier pour le virus de l'herpès simplex de type 1, le virus d'Epstein-Barr ou le coronavirus SARS.

24. Utilisation selon la revendication 23, où ledit composé inhibe une ou plusieurs enzymes de désubiquitination.

25. Utilisation d'un composé de formule (I) tel que défini selon l'une quelconque des revendications 18 à 20 pour la préparation d'un médicament pour traiter et/ou prévenir les troubles inflammatoires, les troubles neurodégénératifs, de préférence les lésions de cellules nerveuses causées par un accident cérébrovasculaire, les lésions hépatiques et l'insuffisance hépatique résultant de lésions hépatiques aïgues ou chroniques infectieuses, ischémiques ou chimiques, les lésions rénales et l'insuffisance rénale résultant de lésions rénales aïgues ou chroniques infectieuses, ischémiques ou chimiques, les lésions cardiaques et l'insuffisance cardiaque résultant de lésions cardiaques aïgues ou chroniques infectieuses, ischémiques ou chimiques, les diabètes résultant de lésions aïgues ou chroniques auto-immunes, chimiques, oxydatives ou métaboliques des cellules bêta à insuline des îlots pancréatiques.

26. Utilisation selon la revendication 25, où ledit composant inhibe une ou plusieurs caspases.

27. Utilisation d'un composé de formule (I) tel que défini selon l'une quelconque des revendications 18 à 20 pour la préparation d'un médicament pour traiter et/ou prévenir le cancer et la métastase, les maladies cardio-vasculaires, les troubles immunologiques, les maladies osseuses et articulaires, l'ostéoporose et l'arthrite.

28. Utilisation selon la revendication 27, où ledit composant inhibe une ou plusieurs cathepsines.

29. Utilisation d'un composé de formule (I) tel que défini selon l'une quelconque des revendications 18 à 20 pour la préparation d'un médicament pour traiter et/ou prévenir les troubles liés au vieillissement, les diabètes à début tardif et la cataracte.

30. Utilisation selon la revendication 29, où ledit composant inhibe une ou plusieurs calpaïnes.

31. Utilisation d'un composé de formule (I) tel que défini selon l'une quelconque des revendications 18 à 20 pour la préparation d'un médicament pour traiter et/ou prévenir les infections et les maladies virales.

32. Utilisation selon la revendication 31, où lesdites infections et maladies virales sont choisies parmi l'hépatite A, l'hépatite C, les infections et maladies du coronavirus SARS, les infections et maladies rhinovirales, les infections et maladies adénovirales, la poliomyélite.

33. Utilisation selon la revendication 31 ou 32, où ledit composant inhibe une
ou plusieurs cystéine protéases virales.

34. Utilisation d'un composé de formule (I) tel que défini selon l'une quelconque des revendications 18 à 20 pour la préparation d'un médicament pour traiter et/ou prévenir les infections et les maladies bactériennes.

35. Utilisation selon la revendication 34, où lesdites infections et maladies bactériennes sont choisies parmi les infections et maladies streptococciques, les infections et maladies causées par la bactérie du genre *Clostridium sp.,* les infections et maladies staphylococciques, les gingivites et les maladies parodontales.

36. Utilisation selon l'un quelconque des revendications 34 à 36, où ledit composant inhibe une ou plusieurs cystéine protéases bactériennes.

37. Utilisation selon la revendication 34 ou 35, où ledit composant inhibe une
ou plusieurs cystéine protéases bactériennes choisies parmi la streptopaïne, la clostripaïne, la cystéine protéase staphylococcique, la gingipaïne.

38. Utilisation d'un composé de formule (I) tel que défini selon l'une quelconque des revendications 18 à 20 pour la préparation d'un médicament pour traiter et/ou prévenir les infections et les maladies fongiques.

39. Utilisation selon la revendication 38, où ledit composant inhibe une ou plusieurs cystéine protéases fongiques.

40. Utilisation d'un composé de formule (I) tel que défini selon l'une quelconque des revendications 18 à 20 pour la préparation d'un médicament pour traiter et/ou prévenir les infections et les maladies infectieuses parasitaires protozoaires.

41. Utilisation selon la revendication 40, où ledit composant inhibe une ou plusieurs cystéine protéases provenant de parasites protozoaires.

42. Utilisation d'un composé de formule (I) tel que défini selon l'une quelconque des revendications 18 à 20 pour la préparation d'un médicament pour traiter et/ou prévenir les infections et les maladies à vers plats parasitaires.

43. Utilisation selon la revendication 42, où ledit composant inhibe une ou plusieurs cystéine protéases provenant de vers plats parasitaires.

44. Utilisation d'un composé de formule (I) tel que défini selon l'une quelconque des revendications 18 à 20 pour la préparation d'un médicament pour traiter et/ou prévenir les infections et les maladies à vers ronds parasitaires.

45. Utilisation selon la revendication 44, où ledit composant inhibe une ou plusieurs cystéine protéases provenant de vers ronds parasitaires.

46. Utilisation selon l'une quelconque des revendications 23 à 45, où ledit médicament est utilisé en combinaison avec une ou plusieurs thérapies choisies parmi les thérapies anticancéreuses, les thérapies neurologiques, les thérapies thrombolytiques, les thérapies antioxydantes, les thérapies anti-infectieuses, les thérapies anti-hypertensives, les thérapies diurétiques, les thérapies thrombolytiques, les thérapies immunosuppressives, les thérapies cardio-vasculaires, les thérapies immunomodulatrices, les thérapies anti-inflammatoires, les thérapies antivirales, les thérapies anti-bactériennes, les thérapies anti-fongiques, les thérapies anti-protozoaires, les thérapies antiparasitaires.
